(19) Europäisches Patentamt
European Patent Office
Office européen des brevets

(11) Veröffentlichungsnummer: **0 195 346 B1**

(12) # EUROPÄISCHE PATENTSCHRIFT

(45) Veröffentlichungstag der Patentschrift: **24.06.92**

(21) Anmeldenummer: **86103188.8**

(22) Anmeldetag: **10.03.86**

(51) Int. Cl.[5]: **C07D 239/54**, C07D 239/70, C07D 239/96, C07D 239/56, C07D 491/04, C07D 495/04, A01N 43/54, C07C 273/04, C07C 335/06

(54) **3-Aryluracile und deren Verwendung zur Unkrautbekämpfung.**

(30) Priorität: **20.03.85 CH 1240/85**
**06.02.86 CH 460/86**

(43) Veröffentlichungstag der Anmeldung:
**24.09.86 Patentblatt 86/39**

(45) Bekanntmachung des Hinweises auf die Patenterteilung:
**24.06.92 Patentblatt 92/26**

(84) Benannte Vertragsstaaten:
**AT BE CH DE FR GB IT LI LU NL SE**

(56) Entgegenhaltungen:
**GB-A- 2 043 629**

**CHEMICAL ABSTRACTS, Band 94, 1981, Seite 654, Zusammenfassung Nr. 192252u, Columbus, Ohio, US; Z. ECKSTEIN et al.: "Modification of the method for producing lenacil and synthesis of its analogs", & PRZEM. CHEM. 1980, 59(10), 541-4**

(73) Patentinhaber: **CIBA-GEIGY AG**
**Klybeckstrasse 141**
**CH-4002 Basel(CH)**

(72) Erfinder: **Wenger, Jean, Dr.**
**Brunnenwiesenstrasse 1**
**CH-8610 Uster(CH)**
Erfinder: **Winternitz, Paul, Dr.**
**Am Pfisterhölzli 50**
**CH-8606 Greifensee(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft heterocyclische Verbindungen, und zwar 3-Aryluracile der allgemeinen Formel

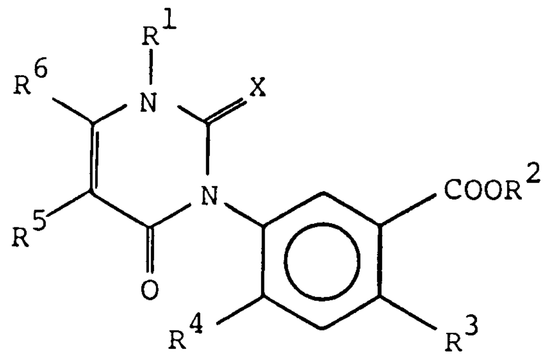

I

worin

| | |
|---|---|
| $R^1$ | Wasserstoff, $C_{1-4}$-Alkyl, $C_{2-4}$-Alkenyl, $C_{2-4}$-Alkinyl, $C_{2-6}$-Alkoxyalkyl, Formyl, $C_{2-6}$-Alkanoyl oder $C_{2-6}$-Alkoxycarbonyl, |
| $R^2$ | Wasserstoff, $C_{1-6}$-Alkyl, $C_{2-4}$-Alkenyl, $C_{2-4}$-Alkinyl oder $C_{2-6}$-Alkoxyalkyl, |
| $R^3$ | Halogen oder Nitro, |
| $R^4$ | Wasserstoff oder Halogen, |
| $R^5$ | Wasserstoff, Halogen, $C_{1-4}$-Alkyl, Chlormethyl, Brommethyl, Hydroxymethyl, ($C_{1-5}$-Alkoxy)-methyl, ($C_{1-5}$-Alkylthio)methyl, Cyano, Nitro oder Thiocyanato, |
| $R^6$ | Wasserstoff, $C_{1-4}$-Alkyl oder $C_{1-4}$-Fluoralkyl, oder |
| $R^5$ und $R^6$ | zusammen Tri- oder Tetramethylen, in welchem ein Methylen durch Sauerstoff oder Schwefel ersetzt sein kann, und welches gegebenenfalls mit $C_{1-3}$-Alkyl substituiert ist, und |
| X | Sauerstoff oder Schwefel bedeuten, mit den Massgaben, dass (i) falls $R^5$ für Fluor steht, $R^6$ ausschliesslich $C_{1-4}$-Alkyl oder $C_{1-4}$-Fluoralkyl bedeutet, und (ii) falls $R^5$ für Cyano steht, $R^6$ ausschliesslich Wasserstoff oder $C_{1-4}$-Alkyl und X ausschliesslich Sauerstoff bedeuten, |

sowie Salze derjenigen Verbindungen der Formel I, in denen $R^1$ und/oder $R^2$ Wasserstoff bedeutet.

Diejenigen Verbindungen der Formel I, in denen $R^1$ und $R^2$ verschieden von Wasserstoff sind, also die Verbindungen der allgemeinen Formel

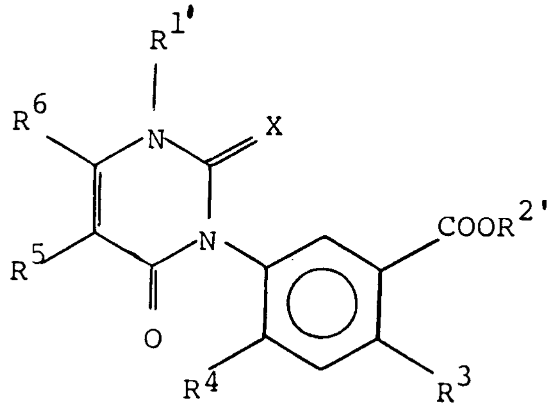

I'

worin

| | |
|---|---|
| $R^{1'}$ | $C_{1-4}$-Alkyl, $C_{2-4}$-Alkenyl, $C_{2-4}$-Alkinyl, $C_{2-6}$-Alkoxyalkyl, Formyl, $C_{2-6}$-Alkanoyl oder $C_{2-6}$-Alkoxycarbonyl bedeutet, |
| $R^{2'}$ | $C_{1-6}$-Alkyl, $C_{2-4}$-Alkenyl, $C_{2-4}$-Alkinyl oder $C_{2-6}$-Alkoxyalkyl bedeutet, und |

$R^3$, $R^4$, $R^5$, $R^6$ und X die oben angegebenen Bedeutungen besitzen, mit den oben angegebenen Massgaben (i) und (ii),

sind herbizid wirksam und eignen sich als Wirkstoffe von Unkrautbekämpfungsmitteln. Die restlichen Verbindungen der Formel I, d.h. diejenigen, in denen $R^1$ und $R^2$ Wasserstoff bedeuten, sowie Salze dieser Verbindungen eignen sich primär als Ausgangsmaterialien zur Herstellung der Verbindungen der Formel I', jedoch besitzen auch einige dieser restlichen Verbindungen I herbizide Eigenschaften.

Die Erfindung umfasst auch Unkrautbekämpfungsmittel, welche Verbindungen der Formel I' als Wirkstoffe enthalten, Verfahren zur Herstellung der erfindungsgemässen Verbindungen sowie die Verwendung der Verbindungen der Formel I' bzw. Mittel zur Bekämpfung von Unkräutern.

Die UK-Patentpublikation Nr. 2.043.629 A beschreibt Uracilderivate, die in der 1-Stellung des Uracilkerns eine gegebenenfalls substituierte Phenylgruppe und in dessen 3-Stellung einen Polyhalogenalkylthio- oder Polyhalogenvinylthiosubstituenten aufweisen. Diese bekannten Verbindungen sollen sich als Pestizide, insbesondere als Fungizide, Bakterizide und Pflanzenwachstumsregulatoren, eignen. Die herbizid wirksamen erfindungsgemässen Verbindungen unterscheiden sich von den bekannten Verbindungen durch den 1-$R^1$-Substituenten (Alkyl, Alkenyl usw. gegenüber gegebenenfalls substituiertem Phenyl) sowie den 3-Arylsubstituenten des Uracilkerns.

US-Patentschriften Nrn. 3.235.360, 3.235.363, 3.291.592, 3.360.520 und 3.436.207, UK-Patentschriften Nrn. 968.661, 968,666, 1.035.096, 1.035.097 und 1.035.098, CH-Patentschrift Nr. 482.402 und DOS 1.924.232 beschreiben herbizid wirksame 3-(subst. Phenyl)-(thio)uracile. Die Substituenten des Phenylrestes sind aus Halogen und Nitro ausgewählt. Die herbizid wirksamen 3-(subst. Phenyl)-(thio)uracile der vorliegenden Erfindung unterscheiden sich im wesentlichen von den bekannten Verbindungen durch den Carbonsäureestersubstituenten des Phenylrestes.

In der obigen Formel I bzw. I' umfasst "Halogen" Fluor, Chlor, Brom und Jod. Die Alkyl-, Alkenyl- und Alkinylreste können geradkettig oder verzweigt sein, wobei dies auch für den bzw. jeden Alkylteil der Alkoxyalkyl-, Alkanoyl-, Alkoxycarbonyl-, Alkoxymethyl-, Alkylthiomethyl- und Fluoralkylgruppen gilt. Die von $R^5$ und $R^6$ gebildeten ankondensierten Ringe sind durch die nachfolgenden Teilstrukturen exemplifiziert:

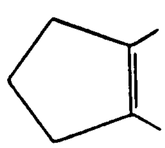
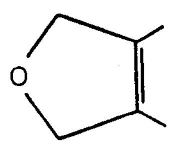
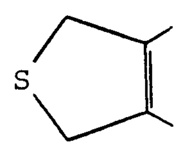

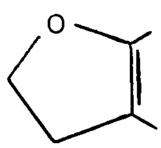
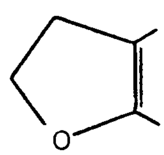
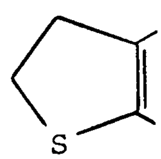

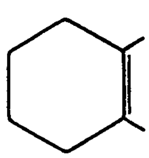
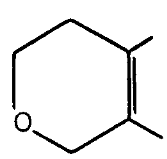
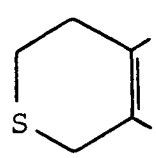

Eine $C_{1-4}$-Fluoralkylgruppe kann ein oder mehrere Fluoratome aufweisen, wobei als Beispiel einer mehrfach fluorierten Alkylgruppe Trifluormethyl genannt sei.

Bei den Salzen der Verbindungen der Formel I handelt es sich insbesondere um Alkalimetallsalze, z.B. Natrium- und Kaliumsalze; Erdalkalimetallsalze, z.B. Calcium- und Magnesiumsalze; Ammoniumsalze, d.h. unsubstituierte Ammoniumsalze und mono- oder mehrfach-substituierte Ammoniumsalze, z.B. Triäthylammonium- und Methylammoniumsalze, sowie um Salze mit anderen organischen Basen, z.B. mit Pyridin.

Das mögliche Vorhandensein mindestens eines asymmetrischen Kohlenstoffatoms in den Verbindungen der Formel I bzw. I' hat zur Folge, dass die Verbindungen in optisch isomeren Formen auftreten können. Durch das Vorliegen einer allfälligen aliphatischen C=C-Doppelbindung kann auch geometrische Isomerie auftreten. Zudem ist bei denjenigen Verbindungen der Formel I, in denen $R^1$ Wasserstoff bedeutet, nicht

ausgeschlossen, dass Keto-Enol-Tautomerie (-NH-CX- = -N=C(XH)-) auftritt. Die Formel I bzw. I' soll all diese möglichen isomeren Formen sowie Gemische davon umfassen.

Bedeutet $R^1$ bzw. $R^{1'}$ oder $R^2$ bzw. $R^{2'}$ $C_{2-4}$-Alkenyl oder $C_{2-4}$-Alkinyl, ist dieser Rest vorzugsweise Allyl bzw. Propargyl. Als $C_{2-6}$-Alkanoylgruppe kommt vorzugsweise $C_{2-4}$-Alkanoyl in Betracht, während die $C_{2-4}$-Alkoxycarbonylgruppen die bevorzugte $C_{2-6}$-Alkoxycarbonylgruppen sind. Im allgemeinen ist ein allfällig vorkommendes Halogenatom vorzugsweise Fluor, Chlor oder Brom.

Unabhängig voneinander bedeuten $R^1$ bzw. $R^{1'}$ vorzugsweise geradkettiges $C_{1-4}$-Alkyl (insbesondere Methyl); $R^2$ bzw. $R^{2'}$ vorzugsweise $C_{1-6}$-Alkyl oder $C_{2-6}$-Alkoxyalkyl; $R^3$ vorzugsweise Chlor oder Brom; $R^4$ vorzugsweise Fluor; $R^5$ vorzugsweise Wasserstoff, Fluor, Chlor, Brom oder geradkettiges $C_{1-4}$-Alkyl (insbesondere Methyl oder Aethyl); und $R^6$ vorzugsweise geradkettiges $C_{1-4}$-Alkyl (insbesondere Methyl oder Aethyl) oder $C_{1-4}$-Fluoralkyl(insbesondere Trifluormethyl). Ebenfalls bevorzugt bedeuten $R^5$ und $R^6$ zusammen Tri- oder Tetramethylen. X ist vorzugsweise Sauerstoff.

Besonders bevorzugte Verbindungen der Formel I bzw. I' sind:

2-Chlor-4-fluor-5-(1,2,4,5,6,7-hexahydro-1-methyl-2,4-dioxo-3H-cyclopenta[d]pyrimidin-3-yl)-benzoesäure-isopropylester,

2-Chlor-4-fluor-5-[1,4,5,6,7,8-hexahydro-1-methyl-2,4-dioxo-3(2H)-chinazolinyl]-benzoesäure-isopropylester,

2-Chlor-4-fluor-5-[3,6-dihydro-3,4-dimethyl-2,6-dioxo-1(2H)-pyrimidinyl]-benzoesäure-isopropylester,

2-Chlor-4-fluor-5-[5-brom-3,6-dihydro-3,4-dimethyl-2,6-dioxo-1(2H)-pyrimidinyl]-benzoesäure-isopropylester,

2-Chlor-4-fluor-5-[3,6-dihydro-3,4-dimethyl-5-fluor-2,6-dioxo-1(2H)-pyrimidinyl]-benzoesäure-isopropylester,

2-Chlor-4-fluor-5-[3,6-dihydro-3,4-dimethyl-5-jod-2,6-dioxo-1(2H)-pyrimidinyl]-benzoesäure-isopropylester,

2-Chlor-4-fluor-5-[3,6-dihydro-3,4-dimethyl-5-hydroxymethyl-2,6-dioxo-1(2H)-pyrimidinyl]-benzoesäure-isopropylester,

2-Chlor-4-fluor-5-[4-äthyl-3,6-dihydro-3-methyl-2,6-dioxo-1(2H)-pyrimidinyl]-benzoesäure-isopropylester,

2-Chlor-4-fluor-5-[3,6-dihydro-3-methyl-4-propyl-2,6-dioxo-1(2H)-pyrimidinyl]-benzoesäure-isopropylester,

2-Chlor-4-fluor-5-[3,6-dihydro-3-methyl-4-trifluormethyl-2,6-dioxo-1(2H)-pyrimidinyl]-benzoesäure-isopropylester,

2-Brom-4-fluor-5-(1,2,4,5,6,7-hexahydro-1-methyl-2,4-dioxo-3H-cyclopenta[d]pyrimidin-3-yl)-benzoesäure-isopropylester,

2-Chlor-4-fluor-5-[3,6-dihydro-5-fluor-3-methyl-4-trifluormethyl-2,6-dioxo-1(2H)-pyrimidinyl]-benzoesäure-isopropylester,

2-Chlor-4-fluor-5-[3,6-dihydro-3,4-dimethyl-5-nitro-2,6-dioxo-1(2H)-pyrimidinyl]-benzoesäure-isopropylester,

2-Chlor-4-fluor-5-(1,2,4,5,6,7-hexahydro-1-methyl-2,4-dioxo-3H-cyclopenta[d]pyrimidin-3-yl)-benzoesäure-(2-methoxy-1-methyläthyl)ester und

2-Chlor-4-fluor-5-(1,2,4,5,6,7-hexahydro-1-methyl-2,4-dioxo-3H-cyclopenta[d]pyrimidin-3-yl)-benzoesäure-tert.butylester.

Weitere Vertreter von Verbindungen der Formel I sind:

2-Chlor-4-fluor-5-(1,2,4,5,6,7-hexahydro-1-methyl-2,4-dioxo-3H-cyclopenta[d]pyrimidin-3-yl)-benzoesäure-(2-methoxyäthyl)ester,

2-Chlor-4-fluor-5-(1,2,4,5,6,7-hexahydro-1-methyl-2,4-dioxo-3H-cyclopenta[d]pyrimidin-3-yl)-benzoesäure-äthylester und

2-Chlor-4-fluor-5-(1,2,4,5,6,7-hexahydro-1-methyl-2,4-dioxo-3H-cyclopenta[d]pyrimidin-3-yl)-benzoesäure-propyl ester.

Das erfindungsgemässe Verfahren zur Herstellung der Verbindungen der Formel I und ihrer Salze ist dadurch gekennzeichnet, dass man

a) zwecks Herstellung derjenigen Verbindungen der Formel I, in denen $R^1$ Wasserstoff und $R^2$ $C_{1-6}$-Alkyl, $C_{2-4}$-Alkenyl, $C_{2-4}$-Alkinyl oder $C_{2-6}$-Alkoxyalkyl bedeuten und $R^5$ verschieden von Chlor, Brom, Jod, Chlormethyl, Brommethyl, Hydroxymethyl, ($C_{1-5}$-Alkoxy)methyl, ($C_{1-5}$-Alkylthio)methyl, Cyano, Nitro oder Thiocyanato ist, sowie von Metallsalzen jener Verbindungen der Formel I, in denen $R^1$ Wasserstoff bedeutet, eine Verbindung der allgemeinen Formel

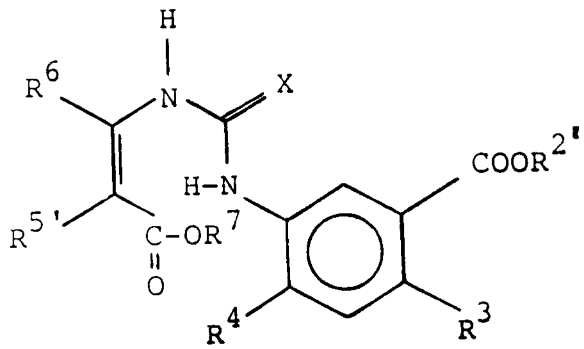

II

worin

$R^{2'}$ $C_{1-6}$-Alkyl, $C_{2-4}$-Alkenyl, $C_{2-4}$-Alkinyl oder $C_{2-6}$-Alkoxyalkyl bedeutet,

$R^3$, $R^4$, $R^6$ und X die oben angegebenen Bedeutungen besitzen,

$R^{5'}$ Wasserstoff, Fluor, $C_{1-4}$-Alkyl oder zusammen mit $R^6$ gegebenenfalls modifiziertes Tri- oder Tetramethylen, wie dies oben näher definiert ist, bedeutet, und

$R^7$ nieder Alkyl, vorzugsweise $C_{1-4}$-Alkyl, bedeutet,

einer basekatalysierten Cyclisierung durch Behandlung mit einer Base unterwirft und gewünschtenfalls eine allfällig erhaltene Metallsalzform des Uracilderivats durch Behandlung mit einer Säure in die entsprechende saure Form ($R^1$ = Wasserstoff) überführt,

b) zwecks Herstellung derjenigen Verbindungen der Formel I, in denen $R^1$ $C_{1-4}$-Alkyl, $C_{2-4}$-Alkenyl, $C_{2-4}$-Alkinyl, $C_{2-6}$-Alkoxyalkyl, Formyl, $C_{2-6}$-Alkanoyl oder $C_{2-6}$-Alkoxycarbonyl bedeutet, ein Uracilderivat der allgemeinen Formel

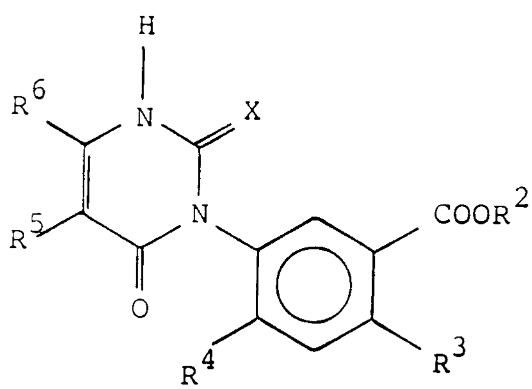

Ia

worin $R^2$, $R^3$, $R^4$, $R^5$, $R^6$ und X die oben angegebenen Bedeutungen besitzen,
zur Alkylierung bzw. Acylierung mit einem entsprechenden eine $C_{1-4}$-Alkyl-, $C_{2-4}$-Alkenyl-, $C_{2-4}$-Alkinyl-, $C_{2-6}$-Alkoxyalkyl-, Formyl-, $C_{2-6}$-Alkanoyl- oder $C_{2-6}$-Alkoxycarbonylgruppe enthaltenden Alkylierungs- bzw. Acylierungsmittel behandelt,

c) zwecks Herstellung derjenigen Verbindungen der Formel I, in denen $R^1$ $C_{1-4}$-Alkyl, $C_{2-4}$-Alkenyl, $C_{2-4}$-Alkinyl oder $C_{2-6}$-Alkoxyalkyl bedeutet, $R^5$ verschieden von Chlor, Brom, Jod, Chlormethyl, Brommethyl, Hydroxymethyl, ($C_{1-5}$-Alkoxy)methyl, ($C_{1-5}$-Alkylthio)methyl, Cyano, Nitro oder Thiocyanato ist, und X Schwefel bedeutet, eine Verbindung der allgemeinen Formel

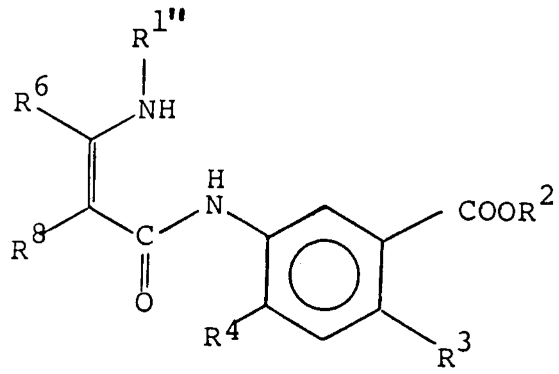

III

worin

$R^{1''}$ $C_{1-4}$-Alkyl, $C_{2-4}$-Alkenyl, $C_{2-4}$-Alkinyl oder $C_{2-6}$-Alkoxyalkyl bedeutet,

$R^2$, $R^3$, $R^4$ und $R^6$ die oben angegebenen Bedeutungen besitzen

und

$R^8$ Fluor, $C_{1-4}$-Alkyl, ($C_{1-4}$-Alkoxy)carbonyl oder zusammen mit $R^6$ wie oben definiertes Tri- oder Tetramethylen bedeutet, wobei, im Falle von $R^8$ = Fluor, $R^6$ $C_{1-4}$-Fluoralkyl ist,

mit N,N'-Thiocarbonyldiimidazolid oder Thiophosgen umsetzt und, falls $R^8$ ($C_{1-4}$-Alkoxy)carbonyl bedeutet, das so hergestellte 5-Alkoxycarbonyl-2-thiouracil der allgemeinen Formel

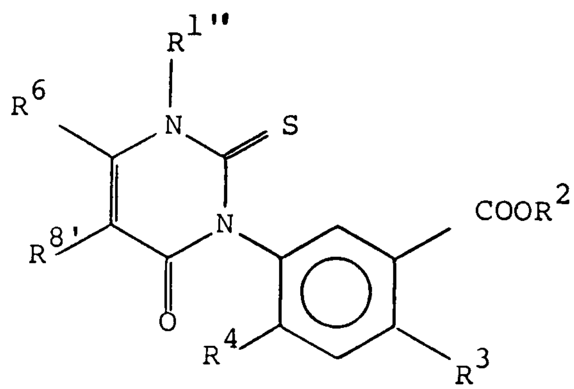

IV

worin

$R^{1''}$, $R^2$, $R^3$, $R^4$ und $R^6$ die oben angegebenen Bedeutungen besitzen

und

$R^{8'}$ ($C_{1-4}$-Alkoxy)carbonyl bedeutet,

zur Hydrolyse und Decarboxylierung in Gegenwart von wässriger Salzsäure oder Trifluoressigsäure aufwärmt,

d) zwecks Herstellung derjenigen Verbindungen der Formel I, in denen $R^5$ Chlor, Brom oder Jod bedeutet, ein Uracilderivat der allgemeinen Formel

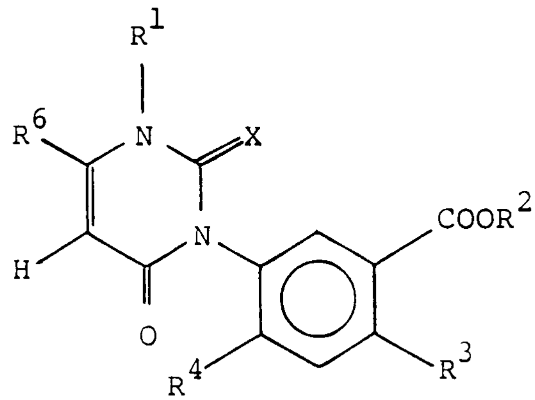

Ib

worin $R^1$, $R^2$, $R^3$, $R^4$, $R^6$ und X die oben angegebenen Bedeutungen besitzen, zur Chlorierung mit elementarem Chlor oder Sulfurylchlorid, zur Bromierung mit elementarem Brom oder Sulfurylbromid bzw. zur Jodierung mit elementarem Jod behandelt,

e) zwecks Herstellung derjenigen Verbindungen der Formel I, in denen $R^5$ Chlor- oder Brommethyl bedeutet, (i) ein Uracilderivat der oben angegebenen Formel Ib mit Chlor- bzw. Brommethoxymethan behandelt oder (ii) ein 5-Hydroxymethyluracil der allgemeinen Formel

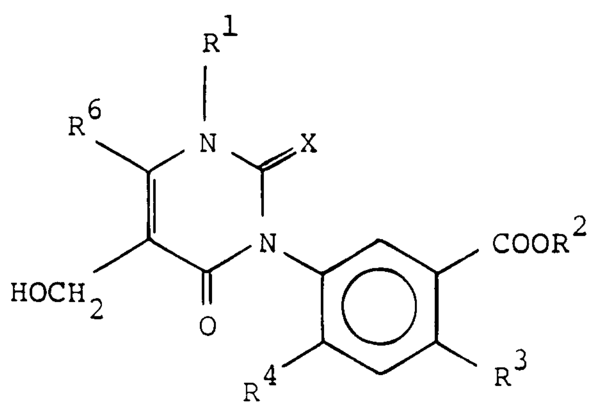

Ic

worin $R^2$, $R^3$, $R^4$ , $R^6$ und X die oben angegebenen Bedeutungen besitzen, mit Thionylchlorid bzw. -bromid behandelt oder (iii) ein 5-Methyluracil der allgemeinen Formel

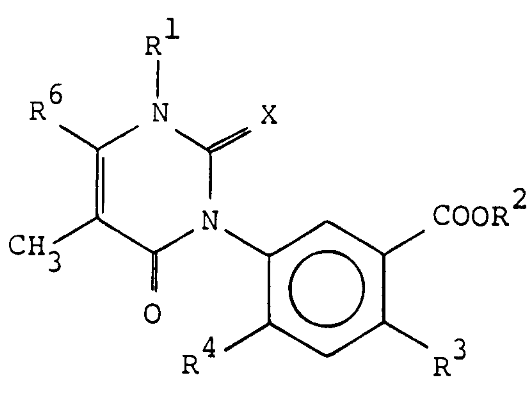

Id

worin $R^1$, $R^2$, $R^3$, $R^4$, $R^6$ und X die oben angegebenen Bedeutungen besitzen, mit N-Chlorsuccinimid bzw. N-Bromsuccinimid behandelt,

f) zwecks Herstellung derjenigen Verbindungen der Formel I, in denen $R^5$ Hydroxymethyl bedeutet, ein 5-Halogenmethyluracil der allgemeinen Formel

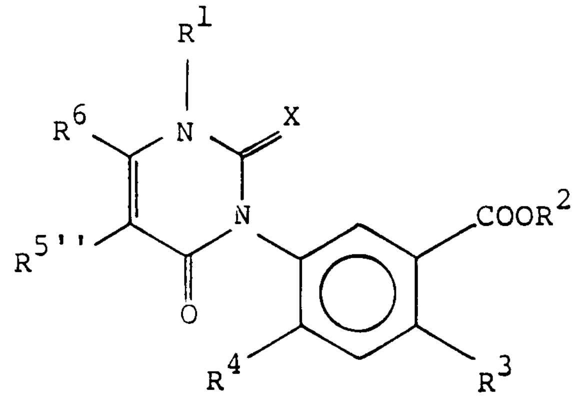

Ie

worin $R^1$, $R^2$, $R^3$, $R^4$, $R^6$ und X die oben angegebenen Bedeutungen besitzen und $R^{5''}$ Chlor-, Brom- oder Jodmethyl bedeutet,
zur Hydrolyse mit einer wässrigen Lösung einer anorganischen Base behandelt,
g) zwecks Herstellung derjenigen Verbindungen der Formel I, in denen $R^5$ ($C_{1-5}$-Alkoxy)methyl oder ($C_{1-5}$-Alkylthio)methyl bedeutet, ein 5-Halogenmethyluracil der oben angegebenen Formel Ie, in der $R^{5''}$ Chlor- oder Brommethyl bedeutet, mit einem Alkalimetallalkoholat oder -thioalkoholat der allgemeinen Formel

$R^9M$ V

worin $R^9$ $C_{1-5}$-Alkoxy oder $C_{1-5}$-Alkylthio und M ein Alkalimetall, vorzugsweise Natrium oder Kalium, bedeuten,
oder mit einem $C_{1-5}$-Alkanol oder $C_{1-5}$-Alkylmercaptan behandelt,
h) zwecks Herstellung derjenigen Verbindungen der Formel I, in denen $R^5$ ($C_{1-5}$-Alkylthio)methyl bedeutet, ein 5-Hydroxymethyluracil der oben angegebenen Formel Ic mit einem $C_{1-5}$-Alkylmercaptan behandelt,
i) zwecks Herstellung derjenigen Verbindungen der Formel I, in denen $R^1$ Wasserstoff, $R^5$ Cyano, $R^6$ Wasserstoff oder $C_{1-4}$-Alkyl und X Sauerstoff bedeuten, eine Verbindung der allgemeinen Formel

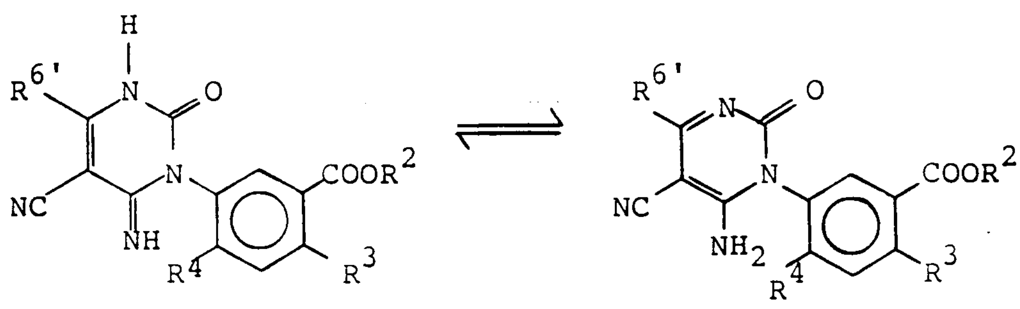

VI

worin $R^2$, $R^3$ und $R^4$ die oben angegebenen Bedeutungen besitzen und $R^{6'}$ Wasserstoff oder $C_{1-4}$-Alkyl bedeutet,
zur säurekatalysierten Hydrolyse mit einer wässrigen Lösung einer Mineralsäure behandelt,
j) zwecks Herstellung derjenigen Verbindungen der Formel I, in denen $R^5$ Nitro bedeutet, ein Uracilderivat der oben angegebenen Formel Ib mit Salpetersäure behandelt,
k) zwecks Herstellung derjenigen Verbindungen der Formel I, in denen $R^5$ Thiocyanato bedeutet, ein Uracilderivat der oben angegebenen Formel Ib mit Thiocyanogen behandelt,
l) zwecks Herstellung derjenigen Verbindungen der Formel I, in denen $R^2$ Wasserstoff bedeutet, einen Benzoesäureester der allgemeinen Formel

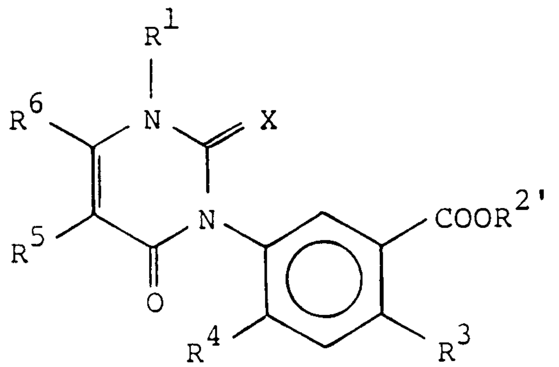

If

worin $R^1$, $R^{2'}$, $R^3$, $R^4$, $R^5$, $R^6$ und X die oben angegebenen Bedeutung besitzen, durch Behandlung mit einer wässrigen Lösung eines organischen Lösungsmittels sowie mit einer anorganischen Base zur entsprechenden Benzoesäure hydrolysiert,

m) zwecks Herstellung derjenigen Verbindungen der Formel I, in denen $R^2$ $C_{1-6}$-Alkyl, $C_{2-4}$-Alkenyl, $C_{2-4}$-Alkinyl oder $C_{2-6}$-Alkoxyalkyl bedeutet, eine Benzoesäure der allgemeinen Formel

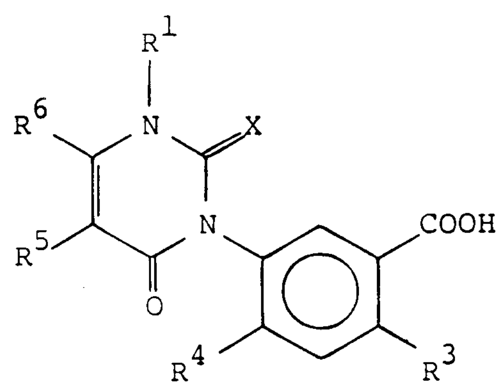

Ig

worin $R^1$, $R^3$, $R^4$, $R^5$, $R^6$ und X die oben angegebenen Bedeutungen besitzen, oder ein reaktionsfähiges Derivat davon entsprechend verestert, wobei, im Falle der Verwendung eines Salzes der Benzoesäure Ig als reaktionsfähiges Derivat, dieses mit einem $C_{1-6}$-Alkyl-, $C_{2-4}$-Alkenyl-, $C_{2-4}$-Alkinyl- oder $C_{2-6}$-Alkoxyalkyl-chlorid, -bromid, -jodid, -sulfat, -mesylat oder -tosylat umgesetzt wird, und im Falle der Verwendung eines Säurehalogenids oder des entsprechenden O-Acyl-1,3-dicyclohexylisoharnstoffes, N-Acylimidazols oder Säureanhydrids der Benzoesäure Ig als reaktionfähiges Derivat, dieses mit einem $C_{1-6}$-Alkanol, $C_{2-4}$-Alkenol, $C_{2-4}$-Alkinol oder $C_{2-6}$-Alkoxyalkanol umgesetzt wird, oder

n) zwecks Herstellung derjenigen Verbindungen der Formel I, in denen $R^2$ $C_{2-6}$-Alkyl, $C_{2-4}$-Alkenyl, $C_{2-4}$-Alkinyl oder $C_{2-6}$-Alkoxyalkyl bedeutet, einen Benzoesäureester der oben angegebenen Formel If einer Umesterungsreaktion mit einem Alkanol, Alkenol bzw. Alkinol der allgemeinen Formel

$R^{2''}OH$ VII

worin $R^{2''}$ $C_{2-6}$-Alkyl, $C_{2-4}$-Alkenyl, $C_{2-4}$-Alkinyl oder $C_{2-6}$-Alkoxyalkyl bedeutet, unterwirft, wobei das Reagenz der Formel VII höher siedend ist als das Alkanol, Alkenol bzw. Alkinol $R^{2'}OH$,

und gewünschtenfalls eine so erhaltene Verbindung der Formel I, in der $R^1$ und/oder $R^2$ Wasserstoff bedeutet, in ein Salz überführt.

Die Cyclisierung nach Verfahrensvariante a) kann zweckmässigerweise durchgeführt werden, indem man die Verbindung der Formel II in einem inerten protischen organischen Lösungsmittel, wie einem Alkohol, z.B. Methanol, Aethanol oder Isopropanol; einem inerten aprotischen organischen Lösungsmittel, wie einem aliphatischen oder cyclischen Aether, z.B. 1,2-Dimethoxyäthan, Tetrahydrofuran oder Dioxan, oder einem Aromaten, z.B. Benzol oder Toluol; einem inerten aprotischen, polaren organischen Lösungsmittel, z.B. Dimethylformamid oder Dimethylsulfoxid, wobei solche Lösungsmittel gegebenenfalls im Zweiphasen-Gemisch mit einem Kohlenwasserstoff, z.B. n-Hexan, verwendet werden können; oder Wasser mit einer Base bei Temperaturen zwischen Raumtemperatur und der Rückflusstemperatur des Reaktionsgemisches behandelt. Als Basen kommen vorzugsweise Natriumalkoholate, Alkalimetallhydroxide, insbesondere Natriumhydroxid und Kaliumhydroxid, Alkalimetallcarbonate, insbesondere Natriumcarbonat und Kaliumcarbonat, und Natriumhydrid in Betracht. Falls als Lösungsmittel ein Alkanol, Alkenol oder Alkinol verwendet wird, dann entspricht dieses Lösungsmittel zweckmässigerweise der jeweiligen Hydroxyverbindung $R^{2'}OH$; dadurch werden unerwünschte konkurrierende Umesterungsreaktionen vermieden. Bei der Verwendung von Natriumhydrid als Base ist das Lösungsmittel vorzugsweise ein aliphatischer oder cyclischer Aether, Dimethylformamid oder Dimethylsulfoxid.

Nach Beendigung der Cyclisierung liegt das Produkt im Falle der Verwendung einer der obenerwähnten Basen oder dergleichen in Form des entsprechenden Alkalimetallsalzes vor. Dieses kann in an sich bekannter Weise isoliert und gereinigt werden, oder man kann das Gemisch ansäuern, um die jeweilige Verbindung der Formel I an sich zu isolieren. Zu diesem Zwecke verwendet man vorzugsweise eine Mineralsäure, wie Salzsäure, oder eine starke organische Säure, wie Essigsäure oder p-Toluolsulfonsäure.

Bei der Verfahrensvariante b) steht der Ausdruck "Alkylierung" für die Einführung einer $C_{1-4}$-Alkyl-, $C_{2-4}$-Alkenyl-, $C_{2-4}$-Alkinyl- oder $C_{2-6}$-Alkoxyalkylgruppe am unsubstituierten Stickstoffatom des Uracilkerns. Zudem gilt in diesem Sinne der Ausdruck "Acylierung" für die entsprechende Einführung einer Formyl-, $C_{2-6}$-Alkanoyl- oder $C_{2-6}$-Alkoxycarbonylgruppe. Als Alkylierungsmittel wird zweckmässigerweise ein $C_{1-4}$-Alkyl-, $C_{2-4}$-Alkenyl-, $C_{2-4}$-Alkinyl- oder $C_{2-6}$-Alkoxyalkylhalogenid, insbesondere das diesbezügliche Chlorid oder Bromid, oder -Sulfat verwendet. Als Acylierungsmittel kommt insbesondere ein Ameisensäurehalogenid, ein $C_{2-6}$-Alkancarbonsäurehalogenid oder -anhydrid bzw. Chlor- oder Bromameisensäure-$C_{1-5}$-alkylester in Frage, wobei das jeweilige Chlorid oder Bromid das bevorzugte Halogenid ist.

Die Alkylierung wird zweckmässigerweise in Gegenwart eines inerten, protischen organischen Lösungsmittels, wie eines niederen Alkanols, z.B. Aethanol, gegebenenfalls im Gemisch mit Wasser; eines inerten, aprotischen organischen Lösungsmittels, wie eines aliphatischen oder cyclischen Aethers, z.B. 1,2-Dimethoxyäthan, Tetrahydrofuran oder Dioxan; oder eines inerten, aprotischen, polaren organischen Lösungsmittels, z.B. Dimethylformamid oder Dimethylsulfoxid, sowie in Gegenwart einer Base, wie Natriumhydrid, eines Alkalimetallalkoholats, insbesondere Natriumalkoholat, oder eines Alkalimetallcarbonats, insbesondere Natriumcarbonat, bei Temperaturen zwischen 0 ° C und ca. 50 ° C, vorzugsweise bei Raumtemperatur, durchgeführt. In einer bevorzugten Ausführungsform wird das Uracilderivat der Formel Ia zunächst mit der Base, wie Natriumhydrid, -äthanolat oder -carbonat, im Lösungsmittel behandelt und nach einer kurzen Reaktionszeit mit dem Halogenid im gleichen Lösungsmittel versetzt. Die Reaktion ist in der Regel je nach verwendetem Lösungsmittel innert relativ kurzer Zeit oder nach wenigen Stunden beendet. Die Acylierung mit einem Halogenid kann auf ähnliche Weise erfolgen, allerdings wird in diesem Fall insbesondere in einem aprotischen Lösungsmittel und in Gegenwart von Natriumhydrid als Base gearbeitet. Bei Verwendung eines Alkancarbonsäureanhydrids als Acylierungsmittel erfolgt die Acylierung geeigneterweise ohne Base.

Im Falle der Alkylierung eines Uracilderivats der Formel Ia, in der X Schwefel bedeutet, werden in der Regel Gemische der entsprechenden N- und S-alkylierten Produkte erzeugt. Das gewünschte N-Alkyl-, N-Alkenyl-, N-Alkinyl- bzw. N-Alkoxyalkyluracil kann auf konventionelle Weise aus einem solchen Gemisch isoliert werden, jedoch empfiehlt sich in diesen Fällen die Verfahrensvariante c).

Die Umsetzung nach Verfahrensvariante c) erfolgt zweckmässigerweise unter Verwendung von N,N'-Thiocarbonyldiimidazolid in der Schmelze bzw. unter Verwendung von Thiophosgen in Gegenwart eines aprotischen organischen Lösungsmittels, wie eines chlorierten aliphatischen Kohlenwasserstoffs, z.B. 1,2-Dichloräthan, oder eines Aromaten, z.B. Toluol, sowie in Gegenwart einer organischen tertiären Base, wie Triäthylamin oder Pyridin. Die Reaktionstemperaturen sind im allgemeinen im Bereich von etwa Raumtemperatur bis 50 ° C, wobei bevorzugt bei Raumtemperatur gearbeitet wird.

Im Falle der Verwendung eines Ausgangsmaterials der Formel III, in der $R^8$ ($C_{1-4}$-Alkoxy)carbonyl bedeutet, wird das so hergestellte 5-Alkoxycarbonyl-2-thiouracil der Formel IV hydrolysiert und anschliessend decarboxyliert, um zur Verbindung der Formel I, in der $R^5$ Wasserstoff bedeutet, zu gelangen. Dies geschieht zweckmässigerweise in einer Stufe durch kurzes Aufwärmen des Produktes IV in Gegenwart von wässriger Salzsäure oder Trifluoressigsäure. In diesem Falle bedeutet $R^{8'}$ vorzugsweise tert.Butoxycarbonyl.

Die Chlorierung bzw. Bromierung nach Verfahrensvariante d) wird zweckmässigerweise mittels elementaren Chlors oder Sulfurylchlorids bzw. elementaren Broms oder Sulfurylbromids durchgeführt, und zwar in Gegenwart eines inerten organischen Lösungsmittels, wie Essigsäure oder eines chlorierten aliphatischen Kohlenwasserstoffes, z.B. Methylenchlorid, Chloroform oder Tetrachlorkohlenstoff, und in einem Temperaturbereich von 0 ° C bis 60 ° C, vorzugsweise bei Raumtemperatur. Zudem kann die Umsetzung unter Zuhilfenahme eines säurebindenden Mittels erfolgen, zu welchem Zweck Natriumacetat und tertiäre Amine,

wie Triäthylamin, Dimethylanilin und Pyridin, besonders bevorzugte säurebindende Mittel sind.

Die Jodierung nach dieser Verfahrensvariante erfolgt zweckmässigerweise unter Verwendung von elementarem Jod als Jodierungsmittel und von einer nieder siedenden aliphatischen Carbonsäure, wie Essigsäure, als Lösungsmittel und bei Temperaturen zwischen ca. 0 °C und ca. 110 °C, vorzugsweise bei Raumtemperatur. Zudem erweist es sich als zweckmässig, die Umsetzung in Gegenwart einer Säure, wie rauchender Salpetersäure, durchzuführen. Zur Beseitigung von überschüssigem Jod kann nach Beendung der Reaktion gesättigte wässrige Natriumhydrogensulfitlösung zugefügt werden.

Bei der Verfahrensvariante e) (i) handelt es sich um die direkte Einführung einer Chlormethyl- oder Brommethylgruppe in unsubstituierter 5-Stellung des Uracilkerns, wobei das Uracilderivat der Formel Ib mit Chlor- bzw. Brommethoxymethan zweckmässigerweise in Abwesenheit eines Verdünnungsmittels und bei erhöhter Temperatur, vorzugsweise im Temperaturbereich von ca. 80 °C bis ca. 140 °C, insbesondere bei ca. 100 °C, umgesetzt wird. Die Umsetzung kann beispielsweise in einem erhitzten geschlossenen Reaktionsgefäss unter Selbstdruck durchgeführt werden.

Auch die Verfahrensvariante e) (ii) kann ohne Verdünnungsmittel durchgeführt werden. Im Falle der Verwendung eines Verdünnungsmittels ist dies zweckmässigerweise ein chlorierter aliphatischer Kohlenwasserstoff, wie Methylenchlorid, Chloroform oder Tetrachlorkohlenstoff. Zudem erfogt die Umsetzung zweckmässigerweise bei Temperaturen zwischen 0 °C und 40 °C, vorzugsweise bei Raumtemperatur.

Als weiteres Verfahren zur Herstellung der erfindungsgemässen 5-Chlormethyl- und 5-Brommethyluracile kommt die Verfahrensvariante e) (iii) in Frage. Diese wird zweckmässigerweise durchgeführt, indem man das 5-Methyluracil der Formel Id in Gegenwart eines Verdünnungsmittels, vorzugsweise eines chlorierten Kohlenwasserstoffes, wie Tetrachlorkohlenstoff, bei erhöhter Temperatur, vorzugsweise bei Temperaturen zwischen 70 °C und 100 °C, mit N-Chlor- oder N-Bromsuccinimid behandelt. Es erweist sich als vorteilhaft, die Umsetzung unter Zuhilfenahme eines radikalbildenden Katalysators, wie Dibenzoylperoxid, und/oder unter UV-Bestrahlung durchzuführen.

Die Umsetzung nach Verfahrensvariante f) kann zweckmässigerweise durchgeführt werden, indem man das 5-Halogenmethyluracil der Formel Ie mit einer wässrigen Lösung einer anorganischen Base, wie eines Alkalimetall-carbonats oder -hydrogencarbonats, insbesondere Natriumcarbonat oder -hydrogencarbonat, behandelt, und zwar bei Temperaturen zwischen 0 °C und 70 °C, vorzugsweise bei Raumtemperatur.

Bei der Verfahrensvariante g) wird das Alkalimetallalkoholat oder -thioalkoholat zweckmässigerweise in situ erzeugt, und zwar durch Umsetzung des Alkalimetalls mit dem Alkohol bzw. Mercaptan $R^9H$. Die Behandlung des 5-Chlormethyl- oder 5-Brommethyluracils der Formel Ie mit dem Alkoholat oder Thioalkoholat erfolgt alsdann in überschüssigem Alkohol bzw. Mercaptan $R^9H$ als Verdünnungsmittel. Gewünschtenfalls kann das Alkoholat oder Thioalkoholat jedoch zunächst isoliert und gegebenenfalls gereinigt werden. In jedem Fall kann ein Hilfslösungsmittel verwendet werden, wie ein aliphatischer oder cyclischer Aether, insbesondere 1,2-Dimethoxyäthan, Tetrahydrofuran oder Dioxan. Im Falle der Behandlung mit dem $C_{1-5}$-Alkanol oder $C_{1-5}$-Alkylmercaptan $R^9H$ erübrigt sich die Umsetzung mit einem Alkalimetall. In beiden Fällen wird die Umsetzung zweckmässigerweise bei Temperaturen zwischen 0 °C und dem Siedepunkt des Reaktionsgemisches, vorzugsweise zwischen Raumtemperatur und 70 °C, durchgeführt.

Als Alternative können die 5-[($C_{1-5}$-Alkylthio)methyl]-uracile nach der Verfahrensvariante h) hergestellt werden, wobei zweckmässigerweise das entsprechende 5-Hydroxymethyluracil der Formel Ic mit dem $C_{1-5}$-Alkylmercaptan in Gegenwart eines Lösungsmittels und bei erhöhter Temperatur behandelt wird. Die bevorzugten Lösungsmittel sind niedere Alkanole, und Reaktionstemperaturen von 100 °C bis 150 °C. Die Wahl des alkoholischen Lösungsmittels kann von der Natur der jeweiligen Gruppe $R^2$ im 5-Hydroxymethyluracil Ic abhängen: Falls das 5-Hydroxymethyluracil der Formel Ic ein Benzoesäureester ist ($R^2$ bedeutet $C_{1-6}$-Alkyl, $C_{2-4}$-Alkenyl, $C_{2-4}$-Alkinyl oder $C_{2-6}$-Alkoxyalkyl) und als Lösungsmittel ein Alkanol, Alkenol oder Alkinol verwendet wird, dann entspricht dieses Lösungsmittel zweckmässigerweise der jeweiligen Hydroxyverbindung $R^2OH$; dadurch werden unerwünschte konkurrierende Umesterungsreaktionen vermieden.

Die Hydrolyse nach Verfahrensvariante i) wird zweckmässigerweise mittels einer Mineralsäure, wie Salzsäure, in wässriger Lösung bei Temperaturen zwischen 20 °C und 100 °C, vorzugsweise bei Raumtemperatur, durchgeführt. Es können auch mit Wasser mischbare Lösungsmittel, wie niedere Alkohole und aliphatische oder cyclische Aether, z.B. 1,2-Dimethoxyäthan, Tetrahydrofuran und Dioxan, verwendet werden, wobei die Wahl eines allfällig verwendeten alkoholischen Lösungsmittels aus dem bereits oben angegebenen Grund von der Natur der jeweiligen Gruppe $R^2$ in der Verbindung VI abhängen kann.

Die Nitrierung nach Verfahrensvariante j) erfolgt zweckmässigerweise mittels Salpetersäure oder Salpetersäure enthaltender Gemische oder Lösungen, wie insbesondere Gemische von Salpetersäure, Schwefelsäure und gegebenenfalls auch Schwefeltrioxid, Lösungen von Salpetersäure in Eisessig und Lösungen von konzentrierter Salpetersäure in chlorierten Kohlenwasserstoffen, z.B. Methylenchlorid, 1,2-Dichloräthan und Tetrachlorkohlenstoff. In der Regel wird die Verbindung der Formel Ib in das Nitrierungsmittel portionenweise eingeführt und das Gemisch bei Raumtemperatur oder leicht erhöhter Temperatur, d.h. bis zu ca. 50 ° C, gerührt.

Das in der Verfahrensvariante k) benötigte Thiocyanogen wird zweckmässigerweise in situ erzeugt, und zwar beispielsweise durch Umsetzung von Blei- oder Ammoniumthiocyanat mit Brom in Gegenwart eines Verdünnungsmittels bei relativ niedrigen Temperaturen, wie 0 ° C bis 30 ° C, vorzugsweise 0 ° C bis 10 ° C. Geeignete Verdünnungsmittel sind halogenierte aliphatische Kohlenwasserstoffe, wie Methylenchlorid und Tetrachlorkohlenstoff, und aliphatische oder cyclische Aether, wie 1,2-Dimethoxyäthan, Tetrahydrofuran und Dioxan, und im Falle von Ammoniumthiocyanat auch niedere Alkancarbonsäuren, wie Essigsäure. Das Uracilderivat der Formel Ib kann von Anfang an im Thiocyanogen erzeugenden Reaktionsmedium vorhanden sein oder kann nachträglich in dieses Medium eingeführt werden, eventuell nach Entfernung, z.B. durch Filtration, der noch verbleibenden festen Anteile des Mediums. In jedem Fall wird die Temperatur des Reaktionsgemisches zweckmässigerweise relativ niedrig gehalten, und zwar innerhalb des oben angegebenen Temperaturbereiches, bis die Reaktion beendet ist.

Die Hydrolyse des Benzoesäureesters If nach Verfahrensvariante 1) kann nach an sich bekannten Methoden durchgeführt werden, insbesondere unter Verwendung eines organischen Lösungsmittels in wässriger Lösung, wie wässriges Alkanol, z.B. Aethanol, oder ein aliphatischer oder cyclischer Aether, z.B. 1,2-Dimethoxyäthan, Tetrahydrofuran oder Dioxan, in wässriger Lösung, sowie einer anorganischen Base, wie Natrium- oder Kaliumhydroxid, bei Temperaturen zwischen 0 ° C und 70 ° C, vorzugsweise bei Raumtemperatur.

Bei der Verfahrensvariante m) handelt es sich um eine Veresterung einer substituierten Benzoesäure bzw. eines reaktionsfähigen Derivats davon, die ebenfalls nach an sich bekannten Methoden durchgeführt werden kann. So wird beispielsweise ein Salz einer Säure der Formel Ig mit einem $C_{1-6}$-Alkyl-, $C_{2-4}$-Alkenyl-, $C_{2-4}$-Alkinyl- oder $C_{2-6}$-Alkoxyalkyl-chlorid, -bromid, -jodid, -sulfat, -mesylat oder -tosylat in einem inerten Verdünnungsmittel bei Temperaturen von etwa Raumtemperatur bis 100 ° C, z.B. bei der Rückflusstemperatur des Reaktionsgemisches, vorzugsweise im Temperaturbereich von 40 ° C bis 70 ° C, umgesetzt. Es kommen als Salze der Benzoesäure der Formel Ig insbesondere Alkalimetallsalze, z.B. das Natrium-, Kalium- oder Lithiumsalz, Erdalkalimetallsalze, z.B. das Magnesium-, Calcium- oder Bariumsalz, und Salze mit organischen Basen, wie tertiäre Amine, z.B. Triäthylamin, 1,5-Diaza-bicyclo[4,3,0]non-5-en, 1,8-Diaza-bicyclo[5,4,0]undec-7-en und 1,4-Diaza-bicyclo[2,2,2]octan, in Frage, wobei die Alkalimetallsalze, insbesondere das Natriumsalz, bevorzugt sind. Die verwendbaren Verdünnungsmittel sind vorzugsweise inerte organische Lösungsmittel, wie niedere Alkanole, z.B. Aethanol, aliphatische und cyclische Aether, z.B. Diäthyläther, Tetrahydrofuran und Dioxan, Ketone, z.B. Aceton und 2-Butanon, Dimethylformamid, Dimethylsulfoxid und Hexamethylphosphorsäuretriamid. Das Salz kann in situ hergestellt werden, indem man die Säure mit einer geeigneten anorganischen Base, z.B. einem Alkalimetall- oder Erdalkalimetallcarbonat oder -hydrogencarbonat, bzw. organischen Base, zum Salz umsetzt und dies anschliessend im gleichen Reaktionsmedium mit dem zweiten Reaktionspartner reagieren lässt.

Im Falle der Verwendung eines Säurehalogenids der Benzoesäure der Formel Ig als reaktionsfähiges Derivat wird dies zweckmässigerweise mit einem $C_{1-6}$-Alkanol, $C_{2-4}$-Alkenol, $C_{2-4}$-Alkinol oder $C_{2-6}$-Alkoxyalkanol in einem inerten organischen Lösungsmittel, wie einem aliphatischen oder cyclischen Aether, z.B. Diäthyläther, Tetrahydrofuran oder Dioxan, einem aliphatischen oder aromatischen Kohlenwasserstoff, z.B. n-Hexan, Benzol oder Toluol, oder einem halogenierten, insbesondere chlorierten, Kohlenwasserstoff, z.B. Methylenchlorid, Chloroform oder Tetrachlorkohlenstoff, bei Temperaturen von etwa -20 ° C bis 100 ° C, vorzugsweise von 0 ° C bis 50 ° C, umgesetzt. Zudem wird zweckmässigerweise in Gegenwart eines säurebindenden Mittels gearbeitet, wie einer organischen Base, z.B. Triäthylamin, Pyridin, 1,5-Diaza-bicyclo[4,3,0]non-5-en, 1,8-Diaza-bicyclo[5,4,0]undec-7-en oder 1,4-Diaza-bicyclo[2,2,2]octan. Das Säurehalogenid ist vorzugsweise das Säurechlorid.

Als weitere in Frage kommende reaktionsfähige Derivate der Benzoesäure der Formel Ig seien der entsprechende O-Acyl-1,3-dicyclohexylisoharnstoff und das entsprechende N-Acylimidazol oder Säureanhydrid genannt. Solche Derivate können wie das Säurehalogenid mit einem $C_{1-6}$-Alkanol, $C_{2-4}$-Alkenol, $C_{2-4}$-Alkinol oder $C_{2-6}$-Alkoxyalkanol umgesetzt werden, um zu den gewünschten Benzoesäureestern zu gelangen. In diesen Fällen erübrigt sich jedoch die Verwendung eines säurebindenden Mittels.

Die Umsetzung nach Verfahrensvariante n) kann zweckmässigerweise durchgeführt werden, indem man den Benzoesäureester der Formel If in überschüssigem Alkanol, Alkenol bzw. Alkinol der Formel VII in Gegenwart eines basischen Katalysators, wie Natriumcyanid, erhitzt, und zwar vorzugsweise bei der Rückflusstemperatur des Reaktionsgemisches. Im Laufe der Reaktion wird der Rest $R^{2'}$ des Benzoesäureesters der Formel If durch den Rest $R^{2''}$ aus der Verbindung der Formel VII ersetzt, wobei das gegenüber der Verbindung VII niedriger siedende Alkanol, Alkenol bzw. Alkinol der Formel $R^{2'}OH$ freigesetzt wird.

Die Salze der so erhaltenen Verbindungen der Formel I, in denen $R^1$ und/oder $R^2$ Wasserstoff bedeutet,

können in an sich bekannter Weise hergestellt werden, wie beispielsweise durch Auflösen der Verbindung der Formel I in einer Lösung einer diesbezüglichen anorganischen oder organischen Base. Die Salzbildung erfolgt in der Regel innert kurzer Zeit bei Raumtemperatur. In einer Ausführungsform wird das Natriumsalz durch Auflösen des Uracilderivats I in wässriger Natriumhydroxidlösung bei Raumtemperatur hergestellt, wobei äquivalente Mengen des Uracilderivats und des Natriumhydroxids verwendet werden. Das feste Salz kann dann durch Fällen mit einem geeigneten inerten Lösungsmittel oder durch Abdampfen des Lösungsmittels isoliert werden. Eine weitere Ausführungsform besteht darin, eine wässrige Lösung eines Alkalimetallsalzes des Uracilderivats I in eine wässrige Lösung eines Salzes, das ein anderes Metallkation als ein Alkalimetallkation aufweist, einzuführen, wobei das zweite Metallsalz des Uracilderivats hergestellt wird. Diese Ausführungsform dient im allgemeinen zur Herstellung von Uracil-Metallsalzen, die in Wasser unlöslich sind.

Die erhaltenen Verbindungen der Formel I sowie deren Salze können nach an sich bekannten Methoden isoliert und gereinigt werden. Ferner ist dem Fachmann geläufig, in welcher Reihenfolge gewisse Umsetzungen unter den Verfahrensvarianten b) und d) bis n) zweckmässig durchzuführen sind, um mögliche, unerwünschte konkurrierende Reaktionen zu vermeiden.

Sofern keine gezielte Synthese zur Isolierung reiner Isomerer durchgeführt wird, kann das Produkt als Gemisch zweier oder mehrerer Isomerer anfallen. Die Isomeren können nach an sich bekannten Methoden aufgetrennt werden. Gewünschtenfalls können beispielsweise reine optisch aktive Isomere auch durch Synthese aus entsprechenden optisch aktiven Ausgangsmaterialien hergestellt werden.

Die Ausgangsmaterialien der Formel II, die neu sind, können in an sich bekannter Weise hergestellt werden, z.B. gemäss den nachfolgenden Reaktionsschemata 1 [Methoden aa), bb) und cc)]:

## Reaktionsschemata 1

aa)

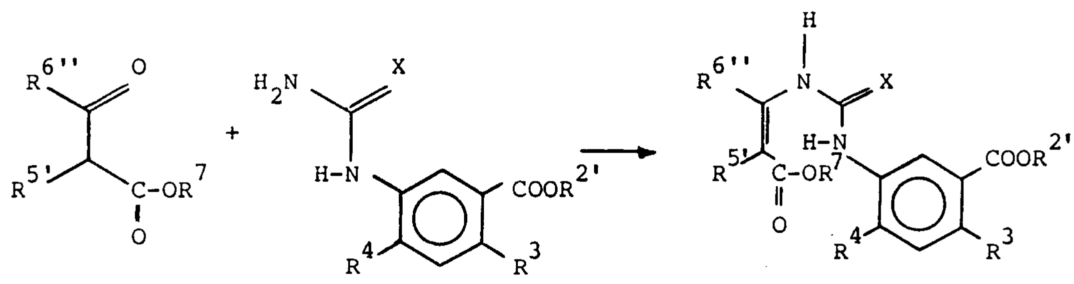

VIII IX IIa

bb)

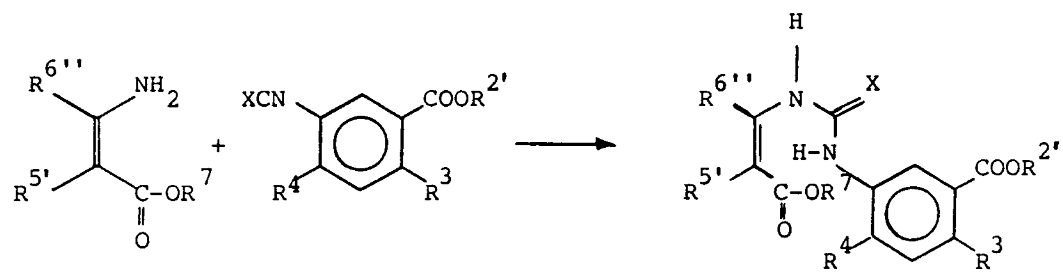

X XI IIb

cc)

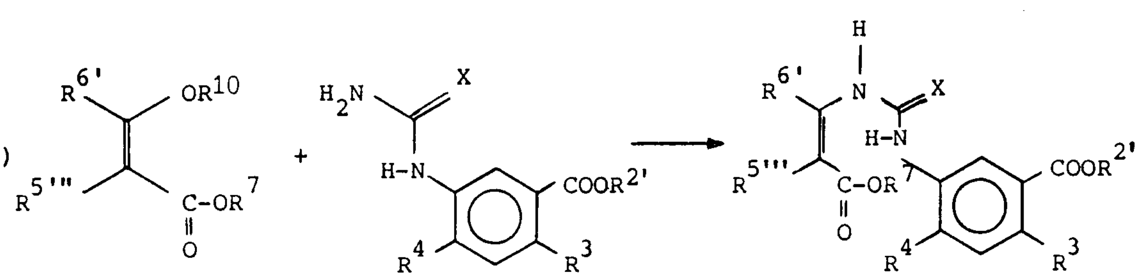

XII IX IIc

In den obigen Reaktionsschemata besitzen $R^{2'}$, $R^3$, $R^4$, $R^{5'}$, $R^{6'}$, $R^7$ und X die oben angegebenen Bedeutungen; $R^{5'''}$ bedeutet Wasserstoff oder $C_{1-4}$-Alkyl; $R^{6''}$ bedeutet $C_{1-4}$-Alkyl, $C_{1-4}$-Fluoralkyl oder zusammen mit $R^{5'}$ gegebenenfalls modifiziertes Tri- oder Tetramethylen, wie dies oben näher definiert ist; und $R^{10}$ bedeutet nieder Alkyl, vorzugsweise $C_{1-4}$-Alkyl.

Die Methode aa) wird zweckmässigerweise dadurch durchgeführt, dass man die Verbindungen der Formeln VIII und IX in einem im wesentlichen wasserfreien Verdünnungsmittel und in Gegenwart eines sauren Katalysators bei erhöhter Temperatur miteinander reagieren lässt. Als Verdünnungsmittel kommen insbesondere mit Wasser azeotrope organische Lösungsmittel, wie Aromate, z.B. Benzol, Toluol und Xylole; halogenierte Kohlenwasserstoffe, wie Methylenchlorid, Chloroform, Tetrachlorkohlenstoff und Chlorbenzol;

und aliphatische und cyclische Aether, wie 1,2-Dimethoxyäthan, Tetrahydrofuran und Dioxan, und als saure Katalysatoren insbesondere starke Mineralsäuren, wie Schwefelsäure und Salzsäure; organische Säuren, wie p-Toluolsulfonsäure; Phosphor enthaltende Säuren, wie Orthophosphorsäure und Polyphosphorsäure; und saure Kationenaustauscher, wie "Amberlyst 15" (Fluka), in Frage. Man arbeitet im allgemeinen in einem Temperaturbereich von etwa 70 ° C bis 120 ° C, vorzugsweise bei der Rückflusstemperatur des Reaktionsgemisches. Unter diesen Reaktionsbedingungen wird die erwünschte rasche Entfernung des in der Reaktion gebildeten Wassers erzielt.

Die Umsetzung nach der Methode bb) erfolgt zweckmässigerweise in Gegenwart eines im wesentlichen wasserfreien aprotischen organischen Lösungsmittels, wie eines aliphatischen oder cyclischen Aethers, z.B. Diäthyläther, 1,2-Dimethoxyäthan, Tetrahydrofuran oder Dioxan, eines aliphatischen oder aromatischen Kohlenwasserstoffs, z.B. n-Hexan, Benzol, Toluol oder eines Xylols; oder eines halogenierten, aliphatischen Kohlenwasserstoffs, z.B. Methylenchlorid, Chloroform, Tetrachlorkohlenstoff oder 1,2-Dichloräthan, sowie gegebenenfalls in Gegenwart einer organischen tertiären Base, wie Triäthylamin oder Pyridin, wobei letzteres sowohl als Lösungsmittel wie auch als Base dienen kann, oder einer Metallhydridbase, wie Natrium- oder Kaliumhydrid. Die Reaktionstemperaturen sind vorzugsweise im Bereich von etwa Raumtemperatur bis 50 ° C, wobei besonders bevorzugt bei Raumtemperatur gearbeitet wird.

Die Umsetzung nach der Methode cc) wird zweckmässigerweise in einem inerten, mit Wasser mischbaren, organischen Lösungsmittel, wie einem aliphatischen oder cyclischen Aether, z.B. 1,2-Dimethoxyäthan, Tetrahydrofuran oder Dioxan, oder einem niederen Alkanol, wie Aethanol, bei Temperaturen zwischen 50 ° C und 100 ° C, vorzugsweise bei der Rückflusstemperatur des Reaktionsgemisches, oder in einem aromatischen Lösungsmittel, wie Benzol, Toluol oder einem Xylol in Gegenwart eines sauren Katalysators, wie Salzsäure oder p-Toluolsulfonsäure, bei Temperaturen zwischen 50 ° C und 100 ° C, vorzugsweise 60 ° C bis 80 ° C, durchgeführt.

Bei den als Ausgangsmaterialien in den Verfahrensvarianten b), d) - h) und j) - n) dienenden Verbindungen der Formeln Ia-Ig handelt es sich um Untergruppen von Verbindungen der Formel I.

Die in der Verfahrensvariante c) benötigten Ausgangsmaterialien der Formel III können in an sich bekannter Weise hergestellt werden, und zwar gemäss dem nachstehenden Reaktionsschema 2:

## Reaktionsschema 2

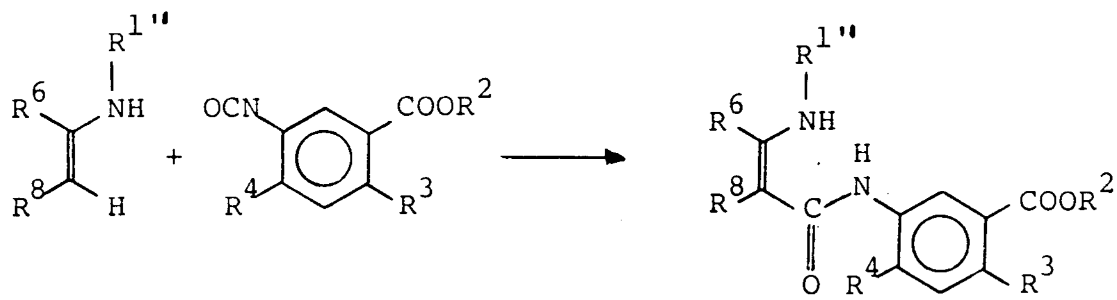

XIII XIV III

Im obigen Reaktionsschema besitzen $R^{1''}$, $R^2$, $R^3$, $R^4$, $R^6$ und $R^8$ die oben angegebenen Bedeutungen, wobei $R^8$ als ($C_{1-4}$-Alkoxy)carbonyl vorzugsweise tert.-Butoxycarbonyl bedeutet. Die Umsetzung der Verbindungen der Formeln XIII und XIV erfolgt zweckmässigerweise in einem aprotischen organischen Lösungsmittel, wie einem aliphatischen oder cyclischen Aether, z.B. Diäthyläther oder Tetrahydrofuran, bei Temperaturen zwischen 0 ° C und 50 ° C, vorzugsweise bei Raumtemperatur. In der Regel reagieren die Reaktionspartner spontan und exotherm.

Auch die in der Verfahrensvariante i) benötigten Ausgangsmaterialien der Formel VI können in an sich bekannter Weise hergestellt werden, und zwar gemäss dem nachstehenden Reaktionsschema 3:

Reaktionsschema 3

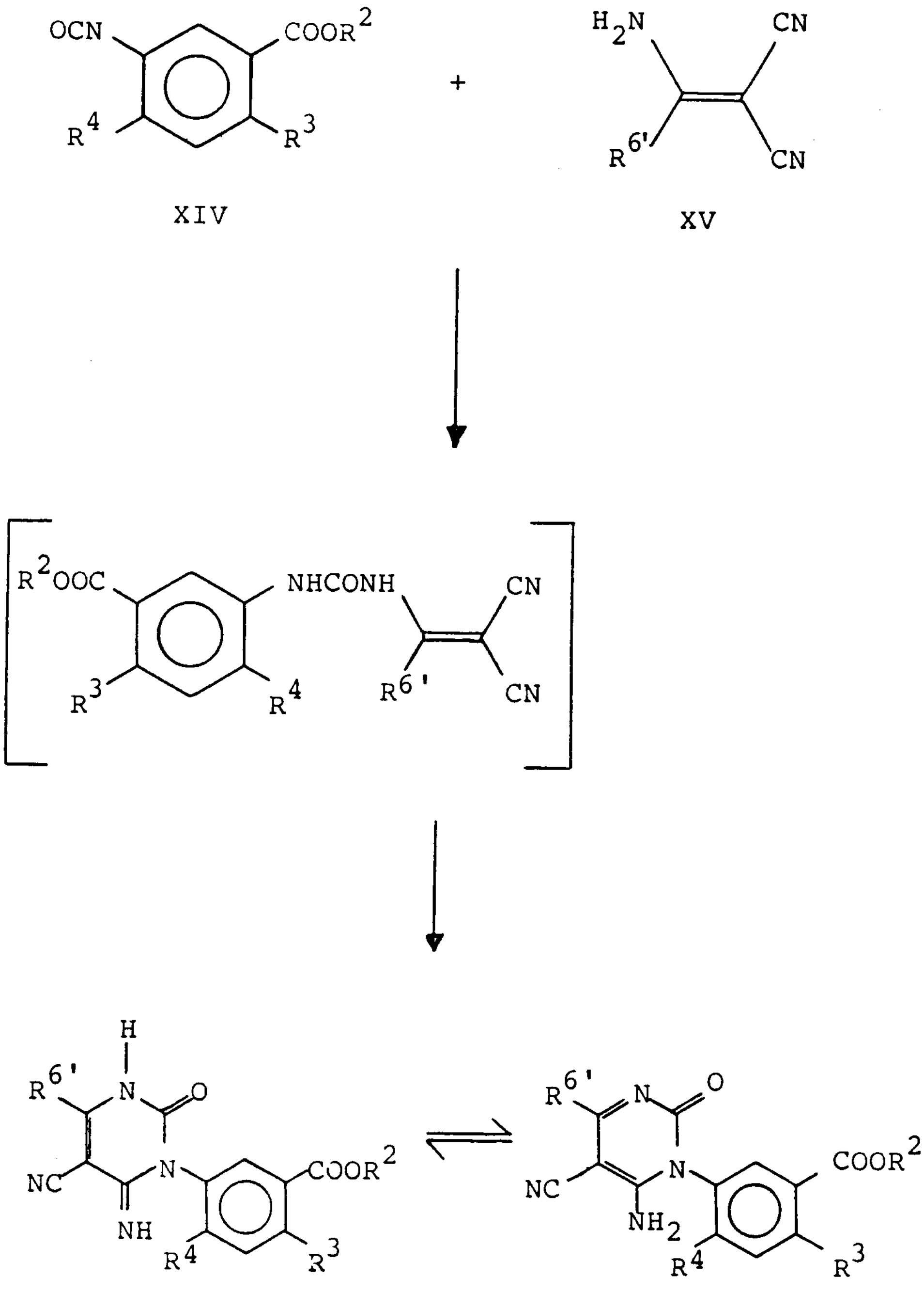

XIV XV

VI

In dem obigen Reaktionsschema besitzen $R^2$, $R^3$, $R^4$ und $R^{6'}$ die oben angegebenen Bedeutungen.

Die Umsetzung der Verbindungen der Formeln XIV und XV miteinander kann zweckmässigerweise in Gegenwart eines Verdünnungsmittels, insbesondere eines aprotischen, polaren organischen Verdünnungsmittels, wie Dimethylformamid oder Dimethylsulfoxid, sowie in Gegenwart einer Base, wie Natriumhydrid, erfolgen. Es wird vorzugsweise bei Temperaturen zwischen 20 ° C und 50 ° C gearbeitet. Zur Isolierung des

Produkts der Formel VI wird angesäuert, wobei das freie 5-Cyanocytosin der Formel VI beispielsweise aus dem entsprechenden Natriumsalz freigesetzt wird.

Die restlichen in den Verfahrensvarianten a) - n) und Methoden aa) - cc) sowie die in den Reaktionsschemata 2 und 3 involvierten Ausgangsmaterialien bzw. Reagenzien sind entweder bekannt oder können nach an sich bekannten Methoden hergestellt werden.

Die erfindungsgemässen Verbindungen der Formel I' besitzen herbizide Eigenschaften und eignen sich zur Bekämpfung von Unkräutern, einschliesslich Ungräsern, insbesondere Setaria faberii, Digitaria sanguinalis, Poa annua, Chenopodium album, Amaranthus retroflexus, Abutilon theopharasti, Sinapsis alba und Datura stramonium, in diversen Nutzpflanzenkulturen, insbesondere in Baumwolle- und Soyakulturen. Zudem sind die Verbindungen sowohl Vorauflauf- als auch Nachauflauf-Herbizide.

Auch gewisse Verbindungen der Formel II besitzen herbizide Eigenschaften und können auf ähnliche Weise wie die Verbindungen I' zur Bekämpfung von Ungräsern und Unkräutern, insbesondere den obenerwähnten, eingesetzt werden. Die neuen Verbindungen II bilden einen weiteren Gegenstand der vorliegenden Erfindung. Aufgrund ihrer besonders ausgeprägten herbiziden Aktivität stellen der 2-Chlor-4-fluor-5-{3-[2-(äthoxycarbonyl)-1-cyclohexen -1-yl]ureido}-benzoesäure-isopropylester und der 2-Chlor-4-fluor-5-{3-[2-(äthoxycarbonyl) -1-methylpropenyl]ureido}-benzoesäure-isopropylester bevorzugte Verbindungen der Formel II dar

In der Praxis genügt üblicherweise eine Konzentration von 0,01-6,0 kg Wirkstoff der Formel I' bzw. II/ha, vorzugsweise 0,05-2,0 kg Wirkstoff der Formel I' bzw. II/ha, um den gewünschten herbiziden Effekt zu erzielen, wobei im allgemeinen die Verbindungen der Formel I' bedeutend wirksamer sind als die herbizid aktiven Verbindungen der Formel II. Besonders bevorzugt ist die Konzentrationsreihe 0,05-1,5 kg Wirkstoff der Formel I' bzw. II/ha.

Das erfindungsgemässe Unkrautbekämpfungsmittel ist dadurch gekennzeichnet, dass es eine wirksame Menge mindestens einer Verbindung der Formel I' oder II, wie oben definiert, sowie Formulierungshilfsstoffe enthält. Das Mittel enthält zweckmässigerweise zumindest einen der folgenden Formulierungshilfsstoffe: feste Trägerstoffe; Lösungs- bzw. Dispersionsmittel; Tenside (Netz- und Emulgiermittel); Dispergatoren (ohne Tensidwirkung); und Stabilisatoren. Unter Verwendung solcher und anderer Hilfsstoffe können diese Verbindungen, also die herbiziden Wirkstoffe, in die üblichen Formulierungen übergeführt werden, wie Stäube, Pulver, Granulate, Lösungen, Emulsionen, Suspensionen, emulgierbare Konzentrate, Pasten und dergleichen.

Die Verbindungen der Formel I' und II sind im allgemeinen wasserunlöslich und können nach den für wasserunlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netz-oder Emulgiermitteln und/oder von Dispergatoren, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorrillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungsmittel vorzugsweise Flammpunkte von mindestens 30 °C und Siedepunkte von mindestens 50 °C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netz- und Emulgiermittel) können nicht-ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen

Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen sein, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäuren, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylonitrilsäureester und Zimtsäureester; und Deaktivatoren, z.B. Salze der Aethylendiaminotetraessigsäure und Polyglykole.

Die erfindungsgemässen Unkrautbekämpfungsmittel können zusätzlich zu den erfindungsgemässen Wirkstoffen Synergisten und andere Wirkstoffe, z.B. Insektizide, Akarizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,01 und 95 Gewichtsprozent, vorzugsweise zwischen 0,5 und 75 Gewichtsprozent einer bzw. mehrerer Verbindungen der Formel I' oder II als Wirkstoff(e). Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 1 und 50 Gewichtsprozent, insbesondere zwischen 10 und 20 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,01 bis 10 Gewichtsprozent, insbesondere ca. 0,5 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine Verbindung der Formel I' oder II, mit festem Trägerstoff vermischt werden, z.B. durch Zusammenmahlen; oder man kann den festen Trägerstoff mit einer Lösung oder Suspension des Wirkstoffes imprägnieren und dann das Lösungs- bzw. Dispersionsmittel durch Abdunsten, Erhitzen oder Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindung der Formel I' oder II kann auch mit einem Tensid und einem festen Trägerstoff zur Bildung eines netzbaren Pulvers vermischt werden, welches in Wasser dispergierbar ist, oder sie kann mit einem festen vorgranulierten Trägerstoff zur Bildung eines granulatförmigen Produktes vermischt werden.

Wenn gewünscht, kann die Verbindung der Formel I' oder II in einem mit Wasser nicht mischbaren Lösungsmittel, wie beispielsweise einem hochsiedenden Kohlenwasserstoff, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel, die einen weiteren Gegenstand der vorliegenden Erfindung bildet, kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter

mit einer erfindungsgemässen Verbindung der Formel I' oder II bzw. mit einem erfindungsgemässen Unkrautbekämpfungsmittel behandelt.

Die nachfolgenden Beispiele dienen zur näheren Erläuterung der Erfindung.

I. Herstellung der Verbindungen der Formel I:

Beispiel 1

Eine Lösung von 118,0 g 2-Chlor-5-{3-[2-(äthoxycarbonyl)-1-cyclopenten -1-yl]ureido}-benzoesäure-äthylester in 800 ml absolutem 1,2-Dimethoxyäthan wird unter Rühren bei 20 ° C während 10 Minuten zu einer Suspension von 7,7 g Natriumhydrid in 800 ml absolutem 1,2-Dimethoxyäthan zugetropft. Das Reaktionsgemisch wird anschliessend 1 Stunde nachgerührt, mit 20 ml Essigsäure versetzt und unter vermindertem Druck zur Trockene eingedampft. Der Rückstand wird in 2 l Methylenchlorid gelöst und zweimal mit 1 l Wasser gewaschen. Die organische Phase wird über wasserfreiem Natriumsulfat getrocknet und bis zur Kristallisation eingedampft. Der Rückstand wird mit 1 l n-Hexan versetzt, und die Kristalle werden abgenutscht und mit n-Hexan nachgewaschen. Man erhält den 2-Chlor-5-(1,2,4,5,6,7-hexahydro-2,4-dioxo-3H-cyclopenta[d]pyrimidin-3-yl)-benzoesäure-äthylester, Smp. 178-180 ° C.

In analoger Weise erhält man beim Einsatz von:

2-Chlor-5-{3-[2-(äthoxycarbonyl)-1-cyclohexen-1-yl]-ureido}-benzoesäure-äthylester den 2-Chlor-5-[1,4,5,6,7,8-hexahydro-2,4-dioxo-3(2H) - chinazolinyl]-benzoesäure-äthylester, Smp. 196-198 ° C.

2-Chlor-4-fluor-5-{3-[2-(äthoxycarbonyl)-1-cylcopenten-1-yl]ureido}-benzoesäure-isopropylester den 2-Chlor-4-fluor-5-(1,2,4,5,6,7-hexahydro-2,4-dioxo-3H -cyclopenta[d]-pyrimidin-3-yl)-benzoesäure-isopropylester, Smp. 204-207 ° C.

2-Chlor-4-fluor-5-{3-[2-(äthoxycarbonyl)-1-cyclohexen-1-yl]ureido}-benzoesäure-isopropylester den 2-Chlor-4-fluor-5-[1,4,5,6,7,8-hexahydro-2,4-dioxo-3(2H) -chinazolinyl]-benzoesäure-isopropylester, Smp. 203-205 ° C.

2-Chlor-5-{3-[2-(äthoxycarbonyl)vinyl]ureido}-benzoesäure-äthylester mit Natriumäthyat in Aethanol den 2-Chlor-5-[3,6-dihydro-2,6-dioxo-1(2H)-pyrimidinyl]-benzoesäure-äthylester, Smp. 170-172 ° C.

2-Chlor-5-{3-[2-(äthoxycarbonyl)-1-methyl-vinyl]-ureido}-benzoesäure-äthylester den 2-Chlor-5-[3,6-dihydro-4-methyl-2,6-dioxo-1(2H)-pyrimidinyl]-benzoesäure-äthylester, Smp. 220-223 ° C.

2-Chlor-4-fluor-5-{3-[2-(äthoxycarbonyl)-1 -methylvinyl]ureido}-benzoesäure-isopropylester mit Natriumisopropylat in Isopropanol den 2-Chlor-4-fluor-5-[3,6-dihydro-4-methyl-2,6-dioxo-1(2H) -pyrimidinyl]-benzoesäure-isopropylester, Smp. 134-136 ° C.

2-Chlor-4-fluor-5-{3-[2 -(methoxycarbonyl)propenyl]-ureido}-benzoesäure-isopropylester mit Natriumisopropylat in Isopropanol/Dimethylformamid-Gemisch den 2-Chlor-4-fluor-5-[3,6-dihydro-5-methyl-2,6-dioxo-1-(2H)-pyrimidinyl]-benzoesäure-isopropylester, Smp. 170-173 ° C.

2-Chlor-5-{3-[2-(äthoxycarbonyl)-1 -methyl-propenyl]-ureido}-benzoesäure-äthylester den 2-Chlor-5-[3,6-dihydro-4,5-dimethyl-2,6-dioxo-1(2H) -pyrimidinyl]-benzoesäure-äthylester, Smp. 202-204 ° C.

2-Chlor-4-fluor-5-{3-[2-(äthoxycarbonyl) -1-methylpropenyl]ureido}-benzoesäure-isopropylester mit Natriumisopropylat in Isopropanol/Dimethylformamid-Gemisch den 2-Chlor-4-fluor-5-[3,6-dihydro-4,5-dimethyl-2,6-dioxo-1(2H)-pyrimidinyl]-benzoesäure-isopropylester, Smp. 155-157 ° C.

5-{3-[2-(Aethoxycarbonyl)-1-cyclopenten-1-yl]ureido}-2-nitrobenzoesäure-äthylester mit Natriumäthylat in Aethanol den 2-Nitro-5-(1,2,4,5,6,7-hexahydro-2,4-dioxo -3H-cyclopenta[d]pyrimidin-3-yl)-benzoesäure-äthylester, Smp. 205-208 ° C.

2-Chlor-4-fluor-5-{3-[4-(methoxycarbonyl)-2,5 -dihydrothien-3-yl]ureido}-benzoesäure-isopropylester mit Natriumisopropylat in Isopropanol/Dimethylformamid-Gemisch den 2-Chlor-4-fluor-5-{1,2,5,7-tetrahydro-2,4-dioxothieno-[3,4-d]pyrimidin-3(4H)-yl}-benzoesäure-isopropylester, Smp. 180-183 ° C.

2-Chlor-5-{3-[4-(methoxycarbonyl)-2,5-dihydro-thien-3-yl]ureido}-benzoesäure-äthylester mit Natriumäthylat in Aethanol den 2-Chlor-5-{1,2,5,7-tetrahydro-2,4-dioxo-thieno[3,4-d]pyrimidin-3(4H)-yl}-benzoesäure-äthylester, Smp. 194-196 ° C.

2-Chlor-4-fluor-5-{3-[2-(methoxycarbonyl)-4,5-dihydro-thien-3-yl]ureido}-benzoesäure-isopropylester mit Natriumisopropylat in Isopropanol/Dimethylformamid-Gemisch den 2-Chlor-4-fluor-5-{1,4,6,7-tetrahydro-2,4-dioxo-thieno[3,2-d]pyrimidin]-3(2H)-yl}-benzoesäure-isopropylester, Smp. 252-254 ° C.

2-Chlor-4-fluor-5-{3-[2-(äthoxycarbonyl)-2-fluor-1-methylvinyl]ureido}-benzoesäure-isopropylester mit Natriumisopropylat in Isopropanol/Dimethylformamid-Gemisch den 2-Chlor-4-fluor-5-[3,6-dihydro-5-fluor-4-methyl-2,6-dioxo-1(2H)-pyrimidinyl]-benzoesäure-isopropylester, $^1$H-NMR ($CDCl_3$, 400 MHz) 9,99 ppm (s,1H), 7,84 ppm (d,1H), 7,37 ppm (d,1H), 5,26 ppm (m,1H), 2,18 ppm (d,3H), 1,38 ppm (d,3H), 1,36 ppm (d,3H).

2-Chlor-3-fluor-5-{3-[2-(äthoxycarbonyl)-1-propylvinyl]ureido}-benzoesäure-isopropylester mit Natriumisopropylat in Isopropanol den 2-Chlor-4-fluor-5-[3,6-dihydro-4-propyl-2,6-dioxo -1(2H)-pyrimidinyl]-benzoesäure-isopropylester, Smp. 192-193 °C.

2-Chlor-4-fluor-5-{3-[2-(äthoxycarbonyl)-1-äthylvinyl]ureido}-benzoesäure-isopropylester mit Natriumisopropylat in Isopropanol den 2-Chlor-4-fluor-5-[4-äthyl-3,6-dihydro-2,6 -dioxo-1(2H)-pyrimidinyl]-benzoesäure-isopropylester, Smp. 121-124 °C.

2-Chlor-4-fluor-5-{3-[2-(äthoxycarbonyl)-1-methyl-1-butenyl]ureido}-benzoesäure-isopropylester mit Natriumisopropylat in Isopropanol/Dimethylformamid-Gemisch den 2-Chlor-4-fluor-5-[5-äthyl-3,6-dihydro -4-methyl-2,6-dioxo-1(2H)-pyrimidinyl]-benzoesäure-isopropylester, Smp. 176-178 °C.

2-Chlor-4-fluor-5-{3-[2-(äthoxycarbonyl)-1-äthyl-1-propenyl]ureido} -benzoesäure-isopropylester mit Natriumisopropylat in Isopropanol/Dimethylformamid-Gemisch den 2-Chlor-4-fluor-5-[4-äthyl-3,6-dihydro-5-methyl -2,6-dioxo-1(2H)-pyrimidinyl]-benzoesäure-isopropylester, Smp. 192-194 °C.

2,4-Difluor-5-{3-[2-(äthoxycarbonyl)-1-cyclopenten-1-yl]ureido}-benzoesäure-isopropylester mit Natriumisopropylat in Isopropanol den 2,4-Difluor-5-(1,2,4,5,6,7-hexahydro-2,4-dioxo -3H-cyclopenta[d]pyrimidin-3-yl]-benzoesäure-isopropylester, Smp. 231-234 °C.

2,4-Dichlor-5-{3-[2-(äthoxycarbonyl)-1-cyclopenten-1-yl]ureido}-benzoesäure-isopropylester mit Natriumisopropylat in Isopropanol den 2,4-Dichlor-5-(1,2,4,5,6,7-hexahydro-2,4-dioxo -3H-cyclopenta[d]pyrimidin-3-yl]-benzoesäure-isopropylester, Smp. 186-189 °C.

2-Brom-4-chlor-5-{3-[2-(äthoxycarbonyl)-1-cyclopenten-1-yl]ureido} -benzoesäure-isopropylester mit Natriumisopropylat in Isopropanol den 2-Brom-4-chlor-5-(1,2,4,5,6,7-hexahydro-2,4 -dioxo-3H-cyclopenta[d]-pyrimidin-3-yl)-benzoesäure-isopropylester, Smp. 208-210 °C.

2-Brom-4-fluor-5-{3-[2-(äthoxycarbonyl)-1-cyclopenten-1-yl]ureido} -benzoesäure-isopropylester mit Natriumisopropylat in Isopropanol den 2-Brom-4-fluor-5-(1,2,4,5,6,7-hexahydro-2,4 -dioxo-3H-cyclopenta[d]-pyrimidin-3-yl)-benzoesäure-isopropylester, Smp. 214-216 °C.

2,4-Dibrom-5-{3-[2-(äthoxycarbonyl)-1-cyclopenten-1-yl]ureido}-benzoesäure-isopropylester mit Natriumisopropylat in Isopropanol den 2,4-Dibrom-5-(1,2,4,5,6,7-hexahydro-2,4 -dioxo-3H-cyclopenta[d]pyrimidin-3-yl)-benzoesäure-isopropylester, Smp. 223-226 °C.

2-Chlor-4-fluor-5-{3-[3-(äthoxycarbonyl)-4,5-dihydro-furan-2-yl]ureido} -benzoesäure-isopropylester mit Natriumisopropylat in Isopropanol/Dimethylformamid-Gemisch den 2-Chlor-4-fluor-5-{1,2,5,6-tetrahydro -2,4-dioxo-furo-[2,3-d]pyrimidin-3(4H)-yl} -benzoesäure-isopropylester, Smp. 213-215 °C.

2-Chlor-5-{3-[2-(äthoxycarbonyl) -1-methyl-vinyl]thioureido}-benzoesäure-isopropylester mit Natriumisopropylat in Isopropanol/Dimethylformamid-Gemisch den 2-Chlor-5-[3,6-dihydro-4-methyl -6-oxo-2-thioxo-1-(2H)-pyrimidinyl]-benzoesäure-isopropylester.

2-Chlor-5-{3-[2-(äthoxycarbonyl) -1-trifluormethylvinyl]thioureido}-benzoesäure-isopropylester mit Natriumisopropylat in Isopropanol/Dimethylformamid-Gemisch den 2-Chlor-5-[3,6-dihydro-4 -trifluormethyl-6-oxo-2-thioxo-1(2H)-pyrimidinyl]-benzoesäure-isopropylester.

2-Chlor-5-{3-[2-(äthoxycarbonyl) -1-cyclopenten-1-yl]thioureido}-benzoesäure-isopropylester mit Natriumisopropylat in Isopropanol/Dimethylformamid-Gemisch den 2-Chlor-5-(1,2,4,5,6,7-hexahydro -4-oxo-2-thioxo-3H-cyclopenta[d]pyrimidin-3-yl) -benzoesäure-isopropylester.

Beispiel 2

Eine Lösung von 3,55 g 3-Amino-4,4,4-trifluorcrotonsäure-äthylester in 50 ml n-Hexan wird unter Rühren bei 0-3 °C während 15 Minuten zu 0,85 g einer 55%igen Natriumhydrid-Dispersion in 50 ml Dimethylformamid zugetropft, und das Gemisch wird 30 Minuten nachgerührt. Anschliessend wird während 5 Minuten unter Rühren und Kühlen eine Lösung von 5,0 g 2-Chlor-4-fluor-5-isocyanatobenzoesäure-isopropylester in 100 ml n-Hexan zugetropft. Die Temperatur des Reaktionsgemisches steigt auf 10 °C an, und das Gemisch wird danach eine Stunde bei Raumtemperatur nachgerührt. Das dabei anfallende Zwischenprodukt 2-Chlor-4-fluor-5-{3-[2-(äthoxycarbonyl) -1-trifluormethyl-vinyl]ureido}-benzoesäure-isopropylester wird nicht isoliert.

Man bringt durch Zusatz von konzentrierter Essigsäure das Gemisch auf pH 4, giesst es auf 750 ml Wasser und extrahiert das wässrige Gemisch mit 300 ml Aethylacetat. Die organische Phase wird über wasserfreiem Natriumsulfat getrocknet und anschliessend unter vermindertem Druck zur Trockene eingedampft und der Rückstand aus Diäthyläther/n-Hexan umkristallisiert. Man erhält auf diese Weise den 2-Chlor-4-fluor-5-[3,6-dihydro-4-trifluormethyl -2,6-dioxo-1(2H)-pyrimidinyl]-benzoesäure-isopropylester, Smp. 127-129 °C.

Beispiel 3

In eine Lösung von 7,5 g 3-Oxo-2,4,4,4-tetrafluor-buttersäure-äthylesterin 20 ml Toluol wird bei 75°C unter Rühren Ammoniak bis zur Sättigung eingeleitet. Anschliessend wird das Reaktionsgemisch unter Verwendung eines Wasserabscheiders 5 Stunden auf Rückflusstemperatur erhitzt, wobei das Zwischenprodukt 3-Amino-2,4,4,4-tetrafluorcrotonsäure-äthylester gebildet wird.

Das Reaktionsgemisch wird bei 0°C unter Rühren während 20 Minuten zu einer Suspension von 1,62 g einer 55%igen Natriumhydrid-Dispersion in 80 ml absolutem Dimethylformamid zugetropft und das Ganze 15 Minuten bei 0°C nachgerührt und danach auf -5°C abgekühlt. Man fügt eine Lösung von 9,56 g 2-Chlor-4-fluor-5-isocyanatobenzoesäure-isopropylester in 40 ml n-Hexan zu. Die Temperatur des Reaktionsgemisches steigt auf 10°C an, und das Gemisch wird danach 3 Stunden bei Raumtemperatur nachgerührt. Das dabei anfallende Zwischenprodukt 2-Chlor-4-fluor-5-{3-[2-(äthoxycarbonyl)-2-fluor -1-trifluormethyl-vinyl]ureido}-benzoesäure-isopropylester wird nicht isoliert.

Das Reaktionsgemisch wird auf 1,5 l Wasser, das 20 ml 2N Salzsäure enthält, gegossen und das wässrige Gemisch zweimal mit je 200 ml Aethylacetat extrahiert. Man wäscht die organische Phase mit Wasser, trocknet sie über wasserfreiem Natriumsulfat und dampft sie unter vermindertem Druck zur Trockene ein. Der Rückstand wird mit 100 ml Diäthyläther und einer Lösung von 5 g Natriumhydrogencarbonat in 300 ml Wasser bei 50°C kurz gerührt und abgekühlt. Nach Abtrennen der wässrigen Phase wird die organische Phase dreimal mit einer Lösung von 2,5 g Natriumhydrogencarbonat in 100 ml Wasser ausgeschüttelt, und anschliessend werden die vereinigten wässrigen Lösungen mit 15 ml konzentrierter Salzsäure auf pH 1 gestellt und mit Diäthyläther extrahiert. Dann wird die organische Phase mit Wasser gewaschen, über wasserfreiem Natriumsulfat getrocknet und unter vermindertem Druck zur Trockene eingedampft. Schliesslich wird der Rückstand aus Diäthyläther/n-Hexan umkristallisiert.

Auf diese Weise erhält man den 2-Chlor-4-fluor-5-[3,6-dihydro-5-fluor -4-trifluormethyl-2,6-dioxo-1(2H)-pyrimidinyl]-benzoesäure-isopropylester, Smp. 102-106°C.

Beispiel 4

Eine Lösung von 1,80 g 3-Amino-2,4-difluorcrotonsäure-äthylester in 20 ml absolutem Dimethylformamid wird unter Rühren bei Raumtemperatur während 10 Minuten zu 0,48 g einer 55%igen Natriumhydrid-Dispersion in 25 ml absolutem Dimethylformamid zugetropft, und das Gemisch wird 15 Minuten nachgerührt. Anschliessend werden 2,81 g 2-Chlor-4-fluor-5-isocyanatobenzoesäure-isopropylester zugefügt, wobei die Temperatur des Reaktionsgemisches auf 35°C ansteigt. Danach wird das Gemisch 2 Stunden bei Raumtemperatur nachgerührt. Das dabei anfallende Zwischenprodukt 2-Chlor-4-fluor-5-{3-[2-(äthoxycarbonyl)-2-fluor-1-fluormethyl -vinyl]ureido}-benzoesäure-isopropylester wird nicht isoliert.

Man giesst das Gemisch auf 300 ml Wasser, das 5,5 ml 2N Salzsäure enthält, extrahiert das wässrige Gemisch dreimal mit je 50 ml Aethylacetat und wäscht die vereinigten organischen Phasen mit Wasser, trocknet sie über wasserfreiem Natriumsulfat und dampft sie unter vermindertem Druck zur Trockene ein. Der Rückstand wird an einer Kieselgel-Säule unter Verwendung von Aethylacetat/n-Hexan (1:1) als Laufmittel chromatographisch gereinigt und aus Diäthyläther/n-Hexan umkristallisiert. Man erhält den 2-Chlor-4-fluor-5-[3,6-dihydro-5-fluor -4-fluormethyl-2,6-dioxo-1(2H)-pyrimidinyl]-benzoesäure-isopropylester, Smp. 171-173°C.

Beispiel 5

Eine Lösung von 50,2 g 2-Chlor-5-(1,2,4,5,6,7-hexahydro-2,4-dioxo-3H-cyclopenta[d]pyrimidin-3-yl) -benzoesäure-äthylester in 300 ml absolutem 1,2-Dimethoxyäthan wird unter Rühren bei 20°C während 10 Minuten zu einer Suspension von 3,6 g Natriumhydrid in 100 ml absolutem 1,2-Dimethoxyäthan zugetropft. Das Reaktionsgemisch wird 1 Stunde nachgerührt, mit 21,3 g Methyljodid versetzt und weitere 2 Stunden gerührt. Anschliessend wird mit 0,5 ml Essigsäure neutral gestellt und unter vermindertem Druck zur Trockene eingedampft. Der Rückstand wird in 500 ml Aethylacetat gelöst, die Lösung dreimal mit 250 ml Wasser ausgeschüttelt und die organische Phase über wasserfreiem Natriumsulfat getrocknet und unter vermindertem Druck zur Trockene eingedampft. Der Rückstand wird in 50 ml heissem Methylenchlorid gelöst, geimpft und mit Diäthyläther versetzt. Man erhält den 2-Chlor-5-(1,2,4,5,6,7-hexahydro-1-methyl-2,4-dioxo-3H-cyclopenta[d]pyrimidin-3-yl) -benzoesäure-äthylester, Smp. 141-143°C.

In analoger Weise erhält man beim Einsatz von:

2-Chlor-5-[1,4,5,6,7,8-hexahydro-2,4-dioxo-3(2H) -chinazolinyl]-benzoesäure-äthylester den 2-Chlor-5-[1,4,5,6,7,8-hexahydro-1-methyl-2,4-dioxo-3(2H) -chinazolinyl]-benzoesäure-äthylester, Smp. 108-112°C.

2-Chlor-4-fluor-5-(1,2,4,5,6,7-hexahydro-2,4-dioxo-3H-cyclopenta[d]pyrimidin-3-yl)-benzoesäure-isopropylester den 2-Chlor-4-fluor-5-(1,2,4,5,6,7-hexahydro-1-methyl-2,4-dioxo -3H-cyclopenta[d]pyrimidin-3-yl)-benzoesäure-isopropylester, Smp. 111-113 ° C.

2-Chlor-4-fluor-5-[1,4,5,6,7,8-hexahydro-2,4-dioxo-3(2H)-chinazolinyl]-benzoesäure-isopropylester den 2-Chlor-4-fluor-5-[1,4,5,6,7,8-hexahydro-1-methyl-2,4-dioxo -3(2H)-chinazolinyl]-benzoesäure-isopropylester, Smp. 113-115 ° C.

2-Chlor-5-(1,2,4,5,6,7-hexahydro-2,4-dioxo-3H -cyclopenta[d]pyrimidin-3-yl)-benzoesäure-äthylester und Chlordimethyläther in Dimethylformamid den 2-Chlor-5-(1,2,4,5,6,7-hexahydro-1-methoxymethyl-2,4 -dioxo-3H-cyclopenta[d]pyrimidin-3-yl)-benzoesäure -äthylester, Smp. 103-106 ° C.

2-Chlor-5-(1,2,4,5,6,7-hexahydro-2,4-dioxo-3H -cyclopenta[d]pyrimidin-3-yl)-benzoesäure-äthylester und 3-Brom-1-propin den 2-Chlor-5-{1,2,4,5,6,7-hexahydro-1-(2-propinyl)-2,4-dioxo -3H-cyclopenta[d]pyrimidin-3-yl}-benzoesäure-äthylester, Smp. 121-123 ° C.

2-Chlor-5-[3,6-dihydro-2,6-dioxo-1(2H) -pyrimidinyl]-benzoesäure-äthylester in Dimethylformamid den 2-Chlor-5-[3,6-dihydro-3-methyl-2,6-dioxo-1(2H) -pyrimidinyl]-benzoesäure-äthylester, Smp. 147-148 ° C.

2-Chlor-5-[3,6-dihydro-4-methyl-2,6-dioxo-1(2H) -pyrimidinyl]-benzoesäure-äthylester den 2-Chlor-5-[3,6-dihydro-3,4-dimethyl-2,6-dioxo-1(2H) -pyrimidinyl]-benzoesäure-äthylester, Smp. 120-122 ° C.

2-Chlor-4-fluor-5-[3,6-dihydro-4-methyl-2,6-dioxo-1(2H)-pyrimidinyl]-benzoesäure-isopropylester mit Natriumisopropylat in Isopropanol den 2-Chlor-4-fluor-5-[3,6-dihydro-3,4-dimethyl-2,6-dioxo-1(2H) -pyrimidinyl]-benzoesäure-isopropylester, $^1$H-NMR ($CDCl_3$, 60MHz) 7,88 ppm (d, 1H), 7,38 ppm (d, 1H), 5,77 ppm (s, 1H), 5,30 ppm (m, 1H), 3,50 ppm (s, 3H), 2,36 ppm (s, 3H), 1,40 ppm (d, 6H).

2-Chlor-4-fluor-5-[3,6-dihydro-5-methyl-2,6-dioxo-1(2H)-pyrimidinyl]-benzoesäure-isopropylester in Dimethylformamid den 2-Chlor-4-fluor-5-[3,6-dihydro-3,5-dimethyl-2,6-dioxo -1(2H)-pyrimidinyl]-benzoesäure-isopropylester, Smp. 177-180 ° C.

2-Chlor-5-[3,6-dihydro-4,5-dimethyl-2,6-dioxo-1(2H)-pyrimidinyl]-benzoesäure-äthylester den 2-Chlor-5-[3,6-dihydro-3,4,5-trimethyl-2,6-dioxo-1(2H) -pyrimidinyl]-benzoesäure-äthylester, Smp. 159-161 ° C.

2-Chlor-4-fluor-5-[3,6-dihydro-4,5-dimethyl-2,6-dioxo-1(2H)-pyrimidinyl]-benzoesäure-isopropylester in Dimethylformamid den 2-Chlor-4-fluor-5-[3,6-dihydro-3,4,5-trimethyl-2,6-dioxo -1(2H)-pyrimidinyl]-benzoesäure-isopropylester, Smp. 119-120 ° C.

2-Nitro-5-(1,2,4,5,6,7-hexahydro-2,4-dioxo-3H -cyclopenta[d]pyrimidin-3-yl)-benzoesäure-äthylester mit Natriumäthylat in Aethanol den 2-Nitro-5-(1,2,4,5,6,7-hexahydro-1-methyl-2,4-dioxo-3H -cyclopenta[d]-pyrimidin-3-yl)-benzoesäure-äthylester, Smp. 188-190 ° C.

2-Chlor-4-fluor-5-{1,2,5,7-tetrahydro-2,4 -dioxo-thieno[3,4-d]pyrimidin-3(4H)-yl}-benzoesäure -isopropylester in Dimethylformamid den 2-Chlor-4-fluor-5-{1,2,5,7-tetrahydro-1-methyl-2,4 -dioxo-thieno[3,4-d]pyrimidin-3(4H)-yl}-benzoesäure -isopropylester, $^1$H-NMR ($CDCl_3$, 400 MHz) 7,82 ppm (d, 1H), 7,35 ppm (d, 1H), 5,23 ppm (m, 1H), 4,24 ppm (m, 2H), 4,09 ppm (m, 2H), 3,45 ppm (s, 3H), 1,36 ppm (d, 6H).

2-Chlor-5-{1,2,5,7-tetrahydro-2,4 -dioxo-thieno-[3,4-d]pyrimidin-3(4H)-yl}-benzoesäure-äthylester in Dimethylformamid den 2-Chlor-5-{1,2,5,7-tetrahydro-1-methyl-2,4-dioxo-thieno[3,4-d]pyrimidin-3(4H)-yl}-benzoesäure-äthylester, Smp. 203-206 ° C.

2-Chlor-4-fluor-5-{1,4,6,7-tetrahydro-2,4 -dioxo-thieno[3,2-d]pyrimidin-3(2H)-yl}-benzoesäure -isopropylester den 2-Chlor-4-fluor-5-{1,4,6,7-tetrahydro-1-methyl-2,4 - dioxo-thieno[3,2-d]pyrimidin-3(2H)-yl}-benzoesäure-isopropylester, Smp. 156-158 ° C.

2-Chlor-4-fluor-5-[3,6-dihydro-5-fluor -4-methyl-2,6-dioxo-1(2H)-pyrimidinyl]-benzoesäure-isopropylester und Dimethylsulfat in Dimethylformamid den 2-Chlor-4-fluor-5-[3,6-dihydro-3,4-dimethyl -5-fluor-2,6-dioxo-1-(2H)-pyrimidinyl]-benzoesäure-isopropylester, Smp. 112-115 ° C.

2-Chlor-4-fluor-5-[3,6-dihydro-4-propyl -2,6-dioxo-1(2H)-pyrimidinyl]-benzoesäure-isopropylester und Dimethylsulfat mit Natriumisopropylat in Isopropanol den 2-Chlor-4-fluor-5-[3,6-dihydro-3-methyl-4-propyl -2,6-dioxo-1(2H)-pyrimidinyl]-benzoesäure-isopropylester , $^1$H-NMR ($CDCl_3$, 400 MHz) 7,83 ppm (d,1H), 7,34 ppm (d,1H), 5,73 ppm (s,1H), 5,23 ppm (m,1H), 3,45 ppm (s,3H), 2,53 ppm (t,2H), 1,72 ppm (m,2H), 1,36 ppm (d,6H), 1,10 ppm (t,3H).

2-Chlor-4-fluor-5-['3,6-dihydro-4-äthyl -2,6-dioxo-1(2H)-pyrimidinyl]-benzoesäure-isopropylester und Dimethylsulfat mit Natriumisopropylat in Isopropanol den 2-Chlor-4-fluor-5-[4-äthyl-3,6-dihydro -3-methyl-2,6-dioxo-1(2H)-pyrimidinyl]-benzoesäure-isopropylester, $^1$H-NMR ($CDCl_3$, 400 MHz) 7,84 ppm (d,1H), 7,34 ppm (d,1H), 5,75 ppm (s,1H), 5,25 ppm (m,1H), 3,45 ppm (s,3H), 2,61 ppm (m,2H), 1,36 ppm (d,6H), 1,31 ppm (t,3H).

2-Chlor-4-fluor-5-[5-äthyl-3,6-dihydro -4-methyl-2,6-dioxo-1(2H)-pyrimidinyl]-benzoesäure-isopropylester und Dimethylsulfat in Dimethylformamid den 2-Chlor-4-fluor-5-[5-äthyl-3,6-dihydro-3,4-dimethyl -2,6-dioxo-1-(2H)-pyrimidinyl]-benzoesäure-isopropylester, $^1$H-NMR ($CDCl_3$, 400 MHz) 7,83 ppm (d,1H), 7,33 ppm (d,1H), 5,23 ppm (m,1H), 3,48 ppm (s,3H), 2,51 ppm (m,2H), 2,34 ppm (s,3H), 1,35 ppm (2xd,6H), 1,09 ppm (t,3H).

2-Chlor-4-fluor-5-[4-äthyl-3,6-dihydro -5-methyl-2,6-dioxo-1(2H)-pyrimidinyl]-benzoesäure-isopropylester

und Dimethylsulfat in Dimethylformamid den 2-Chlor-4-fluor-5-[4-äthyl-3,6-dihydro-3,5-dimethyl -2,6-dioxo-1-(2H)-pyrimidinyl]-benzoesäure-isopropylester, $^1$H-NMR ($CDCl_3$, 400 MHz) 7,82 ppm (d,1H), 7,33 ppm (d,1H), 5,23 ppm (m,1H), 3,51 ppm (s,3H), 2,71 ppm (m,2H), 2,04 ppm (s,3H), 1,35 ppm (d,6H), 1,28 ppm (t,3H).

2-Chlor-4-fluor-5-[3,6-dihydro -2,6-dioxo-4-trifluormethyl-1(2H)-pyrimidinyl]-benzoesäure-isopropylester und Dimethylsulfat in Dimethylformamid den 2-Chlor-4-fluor-5-[3,6-dihydro-3-methyl -4-trifluormethyl-2,6-dioxo-1(2H)-pyrimidinyl]-benzoesäure-isopropylester, $^1$H-NMR ($CDCl_3$, 400 MHz) 7,84 ppm (d,1H), 7,37 ppm (d,1H), 6,38 ppm (s,1H), 5.25 ppm (m,1H), 3,57 ppm (d,3H), 1,36 ppm (d,6H).

2-Chlor-4-fluor-5-[5-cyano-3,6-dihydro -2,6-dioxo-1(2H)-pyrimidinyl]-benzoesäure-isopropylester und Dimethylsulfat in Dimethylformamid den 2-Chlor-4-fluor-5-[5-cyano-3,6-dihydro-3-methyl -2,6-dioxo-1(2H)-pyrimidinyl]-benzoesäure-isopropylester, $^1$H-NMR ($CDCl_3$, 400 MHz) 8,00 ppm (s,1H), 7,83 ppm (d,1H), 7,38 ppm (d,1H), 5,25 ppm (m,1H), 3,55 ppm (s,2H), 1,37 ppm (2xd,6H).

2-Chlor-4-fluor-5-[5-cyano-3,6-dihydro -4-methyl-2,6-dioxo-1(2H)-pyrimidinyl]-benzoesäure-isopropylester und Dimethylsulfat in Dimethylformamid den 2-Chlor-4-fluor-5-[5-cyano-3,6-dihydro-3,4-dimethyl -2,6-dioxo-1(2H)-pyrimidinyl]-benzoesäure-isopropylester, Smp. 171-173 ° C.

2-Chlor-4-fluor-5-[3,6-dihydro-5-fluor-4-trifluormethyl-2,6-dioxo -1(2H)-pyrimidinyl]-benzoesäure-isopropylester und Dimethylsulfat in Dimethylformamid den 2-Chlor-4-fluor-5-[3,6-dihydro-5-fluor-3-methyl -4-trifluormethyl-2,6-dioxo-1(2H)-pyrimidinyl]-benzoesäure-isopropylester, Smp. 66-68 ° C.

2,4-Difluor-5-(1,2,4,5,6,7-hexahydro-2,4-dioxo-3H-cyclopenta[d]pyrimidin -3-yl)-benzoesäure-isopropylester und Dimethylsulfat mit Natriumisopropylat in Isopropanol den 2,4-Difluor-5-(1,2,4,5,6,7-hexahydro -1-methyl-2,4-dioxo-3H-cyclopent[d]pyrimidin-3-yl)-benzoesäure-isopropylester, Smp. 112-115 ° C.

2,4-Dichlor-5-(1,2,4,5,6,7-hexahydro -2,4-dioxo-3H-cyclopenta[d]pyrimidin-3-yl)-benzoesäure-isopropylester und Dimethylsulfat mit Natriumisopropylat in Isopropanol den 2,4-Dichlor-5-(1,2,4,5,6,7-hexahydro-1-methyl -2,4-dioxo-3H-cyclopenta[d]pyrimidin-3-yl)-benzoesäure-isopropylester, $^1$H-NMR ($CDCl_3$, 400 MHz) 7,78 ppm (s,1H), 7,65 ppm (s,1H), 5,23 ppm (m,1H), 3,42 ppm (s,3H), 2,96 ppm (m,2H), 2,82 ppm (m,2H), 2,17 ppm (m,2H), 1,35 ppm (d,6H).

2-Brom-4-chlor-5-(1,2,4,5,6,7-hexahydro -2,4-dioxo-3H-cyclopenta[d]pyrimidin-3-yl)-benzoesäure-isopropylester und Dimethylsulfat mit Natriumisopropylat in Isopropanol den 2-Brom-4-chlor-5-(1,2,4,5,6,7-hexahydro -1-methyl-2,4-dioxo-3H-cyclopenta[d]pyrimidin-3-yl)-benzoesäure-isopropylester, $^1$H-NMR ($CDCl_3$, 400 MHz) 7,86 ppm (s,1H), 7,74 ppm (s,1H), 5,23 ppm (m,1H), 3,42 ppm (s,3H), 2,96 ppm (m,2H), 2,82 ppm (m, 2H), 2,18 ppm (m,2H), 1,35 ppm (d,6H).

2-Brom-4-fluor-5-(1,2,4,5,6,7-hexahydro -2,4-dioxo-3H-cyclopenta[d]pyrimidin-3-yl)-benzoesäure-isopropylester und Dimethylsulfat mit Natriumisopropylat in Isopropanol den 2-Brom-4-fluor-5-(1,2,4,5,6,7-hexahydro -1-methyl-2,4-dioxo-3H-cyclopenta[d]pyrimidin-3-yl)-benzoesäure-isopropylester, Smp. 116-120 ° C.

2,4-Dibrom-5-(1,2,4,5,6,7-hexahydro-2,4 -dioxo-3H-cyclopenta[d]pyrimidin-3-yl)-benzoesäure-isopropylester und Dimethylsulfat mit Natriumisopropylat in Isopropanol den 2,4-Dibrom-5-(1,2,4,5,6,7-hexahydro-1-methyl -2,4-dioxo-3H-cyclopenta[d]pyrimidin-3-yl)-benzoesäure-isopropylester, $^1$H-NMR ($CDCl_3$, 400 MHz) 8,03 ppm (s,1H), 4,72 ppm (s,1H), 5,23 ppm (m,1H), 2,96 ppm (m,2H), 2,83 ppm (m,2H), 2,17 ppm (m,2H), 1,35 ppm (d,6H).

2-Chlor-4-fluor-5-[3,6-dihydro-5-fluor -4-fluormethyl-2,6-dioxo-1(2H)-pyrimidinyl]-benzoesäure-isopropylester und Dimethylsulfat in Dimethylformamid den 2-Chlor-4-fluor-5-[3,6-dihydro-5-fluor -4-fluormethyl-3-methyl-2,6-dioxo-1(2H)-pyrimidinyl]-benzoesäure-isopropylester, Smp. 85-89 ° C.

2-Chlor-4-fluor-5-(1,2,5,6-tetrahydro -2,4-dioxo-furo-[2,3-d]pyrimidin-3(4H)-yl)-benzoesäure-isopropylester den 2-Chlor-4-fluor-5-{1,2,5,6-tetrahydro-1-methyl -2,4-dioxo-furo[2,3-d]pyrimidin-3(4H)-yl}-benzoesäure-isopropylester, Smp. 170-173 ° C.

Beispiel 6

Eine Lösung von 3,6 g 2-Chlor-4-fluor-5-(1,2,4,5,6,7-hexahydro-2,4-dioxo-3H-cyclopenta[d]pyrimidin-3-yl)-benzoesäure-isopropylester in 50 ml absolutem Dimethylformamid wird mit 0,43 g einer 55%igen Natriumhydrid-Dispersion 2 Stunden bei Raumtemperatur gerührt. Anschliessend wird eine Lösung von 0,93 g Acetylchlorid in 10 ml absolutem Dimethylformamid während 10 Minuten zugetropft und das Gemisch 2 Stunden nachgerührt. Das Reaktionsgemisch wird in 100 ml Aethylacetat gelöst und die Lösung gründlich mit Wasser gewaschen. Die organische Phase wird über wasserfreiem Natriumsulfat getrocknet und zur Trockene eingedampft. Der Rückstand wird an einer Kieselgel-Säule unter Verwendung von Methylenchlorid/Aethylacetat (3:1) als Laufmittel chromatographisch gereinigt. Man erhält den 2-Chlor-4-fluor-5-(1,2,4,5,6,7-hexahydro-1-acetyl -2,4-dioxo-3H -cyclopenta[d]pyrimidin-3-yl)-benzoesäure-isopropylester, $^1$H-NMR ($CDCl_3$, 400 MHz) 7,86 ppm (d, 1H), 7,37 ppm (d, 1H), 5,24 ppm (m, 1H), 3,13 ppm (m, 2H),

2,75 ppm (m, 2H), 2,68 ppm (s, 3H), 2,12 ppm (m, 2H), 1,36 ppm (d, 6H).

In analoger Weise erhält man beim Einsatz von:

2-Chlor-4-fluor-5-(1,2,4,5,6,7-hexahydro-2,4-dioxo-3H -cyclopenta[d]pyrimidin-3-yl)-benzoesäure-isopropylester und Chlorameisensäure-methylester den 2-Chlor-4-fluor-5-{1,2,4,5,6,7-hexahydro-1-methoxycarbonyl -2,4-dioxo-3H-cyclopenta[d]pyrimidin-3-yl}-benzoesäure -isopropylester, $^{1}$H-NMR ($CDCl_3$, 400 MHz) 7,84 ppm (d, 1H), 7,35 ppm (d, 1H), 5,24 ppm (m, 1H), 4,03 ppm (s, 3H), 3,02 ppm (m, 2H), 2,79 ppm (m, 2H), 2,16 ppm (m, 2H), 1,36 ppm (d, 6H).

2-Chlor-4-fluor-5-[3,6-dihydro-4-methyl -2,6-dioxo-1(2H)-pyrimidinyl]-benzoesäure-isopropylester den 2-Chlor-4-fluor-5-[3-acetyl-3,6-dihydro-4-methyl -2,6-dioxo-1(2H)-pyrimidinyl]-benzoesäure-isopropylester, Smp. 168-170 ° C.

Beispiel 7

Zu einer Lösung von 15,0 g 2-Chlor-4-fluor-5-[3,6-dihydro-3,4-dimethyl-2,6-dioxo-1(2H)-pyrimidinyl]-benzoesäure-isopropylester in 100 ml Essigsäure werden bei Raumtemperatur 6,3 g Sulfurylchlorid während 1 Minute unter Rühren zugetropft, währenddessen die Temperatur auf ca. 30 ° C ansteigt. Anschliessend wird 15 Minuten bei Raumtemperatur nachgerührt und das Reaktionsgemisch unter vermindertem Druck zur Trockene eingedampft. Der Rückstand wird in Aethylacetat gelöst und die Lösung der Reihe nach mit wässriger Natriumhydrogencarbonat-Lösung und Wasser gewaschen. Die organische Phase wird über wasserfreiem Natriumsulfat getrocknet und zur Trockene eingedampft. Der Rückstand wird aus Methylenchlorid/Diäthyläther umkristallisiert. Man erhält den 2-Chlor-4-fluor-5-[5-chlor-3,6-dihydro-3,4 -dimethyl-2,6-dioxo-1(2H)-pyrimidinyl]-benzoesäure-isopropylester, Smp. 150-153 ° C.

In analoger Weise erhält man beim Einsatz von:

2-Chlor-4-fluor-5-[3,6-dihydro-4-methyl -2,6-dioxo-1(2H)-pyrimidinyl]-benzoesäure-isopropylester den 2-Chlor-4-fluor-5-[5-chlor-3,6-dihydro -4-methyl-2,6-dioxo-1(2H)-pyrimidinyl]-benzoesäure-isopropylester, Smp. 198-201 ° C.

Beispiel 8

Zu einer Lösung von 3,4 g 2-Chlor-4-fluor-5-[3,6-dihydro-4-methyl-2,6 -dioxo-1(2H)-pyrimidinyl]-benzoesäure-isopropylester in 20 ml Essigsäure wird unter Rühren bei 25 ° C während 25 Minuten eine Lösung von 1,7 g Brom in 20 ml Essigsäure zugetropft. Das Reaktionsgemisch wird 1 Stunde nachgerührt und unter vermindertem Druck zurt Trockene eingedampft. Der Rückstand wird in Diäthyläther gelöst und mit wässriger Natriumhydrogencarbonat-Lösung, danach mit Wasser gewaschen. Die organische Phase wird über wasserfreiem Natriumsulfat getrocknet und zur Trockene eingedampft. Der Rückstand wird aus Diäthyläther/n-Hexan umkristallisiert. Man erhält den 2-Chlor-4-fluor-5-[5-brom-3,6-dihydro-4-methyl-2,6-dioxo -1(2H)-pyrimidinyl]-benzoesäure-isopropylester, Smp. 187-189 ° C.

In analoger Weise erhält man bei Einsatz von:

2-Chlor-4-fluor-5-[3,6-dihydro-3,4-dimethyl-2,6-dioxo-1(2H)-pyrimidinyl]-benzoesäure-isopropylester den 2-Chlor-4-fluor-5-[5-brom-3,6-dihydro-3,4-dimethyl-2,6-dioxo-1(2H)-pyrimidinyl]-benzoesäure-isopropylester, Smp. 127-129 ° C.

Beispiel 9

1,50 g 2-Chlor-4-fluor-5-[3,6-dihydro -3,4-dimethyl-2,6-dioxo-1(2H)-pyrimidinyl]-benzoesäure-isopropylester in 10 ml Essigsäure werden mit 0,70 g Jod versetzt und 1 Stunde bei Raumtemperatur gerührt. Anschliessend wird das Reaktionsgemisch mit 0,67 g 100%iger Salpetersäure versetzt und 3 Stunden bei Raumtemperatur gerührt. Das Reaktionsgemisch wird auf 150 ml Eiswasser gegossen und mit 100 ml Aethylacetat extrahiert. Man wäscht die organische Phase mit 150 ml Wasser, danach mit 150 ml wässriger Natriumhydrogencarbonat-Lösung und schliesslich mit 150 ml gesättigter, wässriger Natriumhydrogensulfit-Lösung. Die obere Phase wird über wasserfreiem Natriumsulfat getrocknet und unter vermindertem Druck zur Trockene eingedampft. Der Rückstand wird aus Methylenchlorid/n-Hexan umkristallisiert. Man erhält den 2-Chlor-4-fluor-5-[3,6-dihydro-3,4-dimethyl -5-jod-2,6-dioxo-1(2H)-pyrimidinyl]-benzoesäure-isopropylester, Smp. 147-150 ° C.

In analoger Weise erhält man beim Einsatz von:

2-Chlor-4-fluor-5-[3,6-dihydro-4-methyl -2,6-dioxo-1(2H)-pyrimidinyl]-benzoesäure-isopropylester den 2-Chlor-4-fluor-5-[3,6-dihydro-5-jod -4-methyl-2,6-dioxo-1(2H)-pyrimidinyl]-benzoesäure-isopropylester, Smp. 211-213 ° C.

Beispiel 10

5,0 g 2-Chlor-4-fluor-5-[3,6-dihydro-3,4-dimethyl-2,6-dioxo-1(2H) -pyrimidinyl]-benzoesäure-isopropylester und 5,7 g Chlordimethyläther werden im Autoklav 24 Stunden (ca. 8-9 Atm.) auf 100°C erhitzt. Nach Abkühlen des Reaktionsgemisches wird dies mit Methylenchlorid versetzt und unter vermindertem Druck bei 50°C zur Trockene eingedampft. Man erhält so den 2-Chlor-4-fluor-5-[5-chlormethyl -3,6-dihydro-3,4-dimethyl-2,6-dioxo-1(2H)-pyrimidinyl]-benzoesäure-isopropylester, der für allfällige weitere Reaktionen nicht gereinigt werden muss. $^1$H-NMR ($CDCl_3$, 400 MHz) 7,83 ppm (d,1H), 7,34 ppm (d,1H), 5,24 ppm (m,1H), 4,64 ppm (d,1H), 4,54 ppm (d,1H), 3,52 ppm (s,3H), 2,48 ppm (s,3H), 1,37 ppm (d,3H), 1,35 ppm (d,3H).

Beispiel 11

Eine Lösung von 3,5 g 2-Chlor-4-fluor-5-[3,6-dihydro-3,5-dimethyl-2,6 -dioxo-1(2H)-pyrimidinyl]-benzoesäure-isopropylester in 100 ml Tetrachlorkohlenstoff wird mit 1,8 g N-Bromsuccinimid und etwas Dibenzoylperoxid unter Rühren 1 Stunde auf Rückflusstemperatur erhitzt. Das Reaktionsgemisch wird mit einer 150W Birne beleuchtet. Das Succinimid wird abgenutscht und das Filtrat unter vermindertem Druck zur Trockene eingedampft. Der Rückstand wird an einer Kieselgel-Säule unter Verwendung von Methylenchlorid/Aethylacetat (7:1) als Laufmittel chromatographisch gereinigt. Man erhält den 2-Chlor-4-fluor-5-[5-brommethyl-3,6-dihydro-3-methyl-2,6 -dioxo-1(2H)-pyrimidinyl]-benzoesäure-isopropylester, $^1$H-NMR ($CDCl_3$, 400 MHz) 7,84 ppm (d, 1H), 7,58 ppm (s, 1H), 7,36 ppm (d, 1H), 5,24 ppm (m, 1H), 4,35 ppm (d, 1H), 4,29 ppm (d, 1H), 3,48 ppm (s, 3H), 1,37 ppm (2d, 6H).

Beispiel 12

Eine Lösung von 1,14 g 2-Chlor-4-fluor-5-[5-chlormethyl-3,6-dihydro -3,4-dimethyl-2,6-dioxo-1(2H)-pyrimidinyl]-benzoesäure-isopropylester in 20 ml 1,2-Dimethoxyäthan wird mit einer Lösung von 0,47 g Natriumhydrogencarbonat in 10 ml Wasser 3 Stunden bei Raumtemperatur gerührt. Das Lösungsmittel wird unter vermindertem Druck abdestilliert und der Rückstand mit Aethylacetat extrahiert. Die organische Phase wird über wasserfreiem Natriumsulfat getrocknet und zur Trockene eingedampft. Der Rückstand wird an einer Kieselgel-Säule unter Verwendung von Methylenchlorid/Aethylacetat (2:1) als Laufmittel chromatographisch gereinigt. Man erhält den 2-Chlor-4-fluor-5-[3,6-dihydro-3,4-dimethyl -5-hydroxymethyl-2,6-dioxo-1-(2H)-pyrimidinyl]-benzoesäure-isopropylester, $^1$H-NMR ($CDCl_3$), 400 MHz) 7,83 ppm (d,1H), 7,35 ppm (d,1H), 5,24 ppm (m,1H), 4,59 ppm (d,1H), 4,54 ppm (d,1H), 3,50 ppm (s,3H), 2,44 ppm (s,3H), 2,34 ppm (s, ca. 1H, sehr breit), 1,36 ppm (d,6H).

Beispiel 13

1,14 g 2-Chlor-4-fluor-5-[5-chlormethyl -3,6-dihydro-3,4-dimethyl-2,6-dioxo-1(2H)-pyrimidinyl]-benzoesäure-isopropylester werden in 10 ml Methanol gelöst, und die Lösung wird 45 Minuten auf 60°C erhitzt. Das Reaktionsgemisch wird unter vermindertem Druck zur Trockene eingedampft und der Rückstand in Aethylacetat gelöst und mit wässriger Natriumhydrogencarbonat-Lösung ausgeschüttelt. Die organische Phase wird über wasserfreiem Natriumsulfat getrocknet und unter vermindertem Druck zur Trockene eingedampft und der Rückstand an einer Kieselgel-Säule unter Verwendung von Diäthyläther/Aethylacetat (15:1) als Laufmittel chromatographisch gereinigt. Man erhält den 2-Chlor-4-fluor-5-[3,6-dihydro-3,4-dimethyl -5-methoxymethyl-2,6-dioxo-1(2H)-pyrimidinyl]-benzoesäure-isopropylester, $^1$H-NMR ($CDCl_3$, 400 MHz) 7,82 ppm (d,1H), 7,33 ppm (d,1H), 5,23 ppm (m,1H), 4,40 ppm (d,1H), 4,33 ppm (d,1H), 3,49 ppm (s,3H), 3,39 ppm (s,3H), 2,43 ppm (s,3H), 1,35 ppm (d,6H).

In analoger Weise erhält man beim Einsatz von:

2-Chlor-4-fluor-5-[5-chlormethyl-3,6-dihydro -3,4-dimethyl-2,6-dioxo-1(2H)-pyrimidinyl]-benzoesäure-isopropylester mit Isopropanol den 2-Chlor-4-fluor-5-[3,6-dihydro-3,4-dimethyl -5-isopropoxymethyl-2,6-dioxo-1-(2H)-pyrimidinyl]-benzoesäure-isopropylester, $^1$H-NMR ($CDCl_3$, 400 MHz) 7,82 ppm (d,1H), 7,32 ppm (d,1H), 5,22 ppm (m,1H), 4,45 ppm (d,1H), 4,34 ppm (d,1H), 3,69 ppm (m,1H), 3,48 ppm (s,3H), 2,44 ppm (s,3H), 1,35 ppm (d,6H), 1,21 ppm (d,6H).

Beispiel 14

1,14 g 2-Chlor-4-fluor-5-[5-chlormethyl -3,6-dihydro-3,4-dimethyl-2,6-dioxo-1(2H)-pyrimidinyl]-benzoesäure -isopropylester und 0.21 g Natrium-methanthiolat werden in 5 ml Dimethylformamid 16 Stunden bei

Raumtemperatur gerührt. Das Reaktionsgemisch wird auf 100 ml Wasser gegossen und das wässrige Gemisch mit 50 ml Aethylacetat extrahiert. Die organische Phase wird mit Wasser gewaschen, über wasserfreiem Natriumsulfat getrocknet und unter vermindertem Druck zur Trockene eingedampft. Der Rückstand wird an einer Kieselgel-Säule unter Verwendung von Diäthyläther/n-Hexan (7:1) als Laufmittel chromatographisch gereinigt. Man erhält den 2-Chlor-4-fluor-5-[3,6-dihydro-3,4-dimethyl -5-methylthiomethyl-2,6-dioxo-1(2H)-pyrimidinyl]-benzoesäure-isopropylester, $^1$H-NMR ($CDCl_3$, 400 MHz) 7,83 ppm (d,1H), 7,33 ppm (d,1H), 5,23 ppm (m,1H), 3,67 ppm (d,1H), 3,62 ppm (d,1H), 3,50 ppm (s,3H), 2,43 ppm (s,3H), 2,17 ppm (s,3H), 1,36 ppm (2xd,6H).

## Beispiel 15

3,40 g 5-[6-Amino-5-cyano-2-oxo-1(2H)-pyrimidinyl]-2-chlor-4-fluorbenzoesäure-isopropylester werden in einer Lösung von 100 ml Isopropanol und 10 ml 2N Salzsäure 1 Stunde bei Raumtemperatur gerührt. Das Reaktionsgemisch wird unter vermindertem Druck weitgehend eingeengt und mit Aethylacetat extrahiert. Die organische Phase wird mit wässriger Natriumhydrogencarbonat-Lösung, danach mit Wasser gewaschen und über wasserfreiem Natriumsulfat getrocknet. Anschliessend wird unter vermindertem Druck zur Trockene eingedampft, und der Rückstand in Diäthyläther gelöst, mit Kohle behandelt und zur Trockene eingedampft. Der Rückstand wird aus Diäthyläther/n-Hexan umkristallisiert. Man erhält den 2-Chlor-4-fluor-5-[5-cyano-3,6-dihydro -2,6-dioxo-1(2H)-pyrimidinyl]-benzoesäure-isopropylester, Smp. 143-145 ° C.

In analoger Weise erhält man beim Einsatz von:

5-[6-Amino-5-cyano-4-methyl-2-oxo -1(2H)-pyrimidinyl]-2-chlor-4-fluorbenzoesäure-isopropylester den 2-Chlor-4-fluor-5-[5-cyano-3,6-dihydro -4-methyl-2,6-dioxo-1(2H)-pyrimidinyl]-benzoesäure-isopropylester, Smp. 189-193 ° C.

## Beispiel 16

Eine Lösung von 1,50 g 2-Chlor-4-fluor-5-[3,6-dihydro-4-methyl -2,6-dioxo-1(2H)-pyrimidinyl]-benzoesäure-isopropylester in 30 ml Methylenchlorid wird bei Raumtemperatur unter Rühren mit 2,0 ml 100%iger Salpetersäure versetzt. Die Lösung wird anschliessend mit 5 Tropfen konzentrierter Schwefelsäure versetzt und 48 Stunden bei Raumtemperatur gerührt. Das Reaktionsgemisch wird auf 150 ml Eiswasser gegossen, mit 100 ml Aethylacetat verdünnt und viermal mit 150 ml Wasser gewaschen. Die organische Phase wird über wasserfreiem Natriumsulfat getrocknet und unter vermindertem Druck zur Trockene eingedampft. Der Rückstand wird aus Diäthyläther umkristallisiert. Man erhält 2-Chlor-4-fluor-5-[3,6-dihydro-4-methyl-5-nitro -2,6-dioxo-1(2H)-pyrimidinyl]-benzoesäure-isopropylester, Smp. 202-205 ° C

In analoger Weise erhält man beim Einsatz von:

2-Chlor-4-fluor-5-[3,6-dihydro-3,4-dimethyl -2,6-dioxo-1(2H)-pyrimidinyl]-benzoesäure-isopropylester den 2-Chlor-4-fluor-5-[3,6-dihydro-3,4-dimethyl-5-nitro -2,6-dioxo-1(2H)-pyrimidinyl]-benzoesäure-isopropylester, Smp. 129-131 ° C.

## Beispiel 17

Zu 2,1 g Ammoniumrhodamid in 80 ml Essigsäure werden unter Rühren bei 10-15 ° C 1,86 g Brom in 10 ml Essigsäure während 15 Minuten zugetropft. Anschliessend werden 1,5 g 2-Chlor-4-fluor-5-[3,6-dihydro-4-methyl -2,6-dioxo-1(2H)-pyrimidinyl]-benzoesäure-isopropylester in 15 ml Essigsäure während 5 Minuten bei 10-15 ° C zugetropft, und das Reaktionsgemisch wird 1 Stunde bei Raumtemperatur nachgerührt und anschliessend unter vermindertem Druck weitgehend eingedampft. Man löst den Rückstand in Aethylacetat, nutscht die unlöslichen Anteile ab und wäscht das Filtrat mit wässriger Natriumhydrogencarbonat-Lösung, danach mit Wasser. Die organische Phase wird über wasserfreiem Natriumsulfat getrocknet und unter vermindertem Druck zur Trockene eingedampft. Der Rückstand wird an einer Kieselgel-Säule unter Verwendung von Methylenchlorid/Aethylacetat (3:1) als Laufmittel chromatographisch gereinigt. Das Produkt wird aus Methylenchlorid/Diäthyläther umkristallisiert. Man erhält den 2-Chlor-4-fluor-5-[3,6-dihydro-4-methyl-5-thiocyanato -2,6-dioxo-1(2H)-pyrimidinyl]-benzoesäure-isopropylester, Smp. 159-161 ° C.

In analoger Weise erhält man beim Einsatz von:

2-Chlor-4-fluor-5-[3,6-dihydro-3,4-dimethyl -2,6-dioxo-1(2H)-pyrimidinyl]-benzoesäure-isopropylester den 2-Chlor-4-fluor-5-[3,6-dihydro-3,4-dimethyl-5-thiocyanato-2,6-dioxo-1(2H)-pyrimidinyl]-benzoesäure-isopropylester, $^1$H-NMR ($CDCl_3$, 400 MHz) 7,83 ppm (d,1H), 7,36 ppm (d,1H), 5,25 ppm (m,1H), 3,59 ppm (s,3H), 2,80 ppm (s,3H), 1,38 ppm (d,3H), 1,36 ppm (d,3H).

Beispiel 18

3,2 g fein pulverisierter 2-Chlor-5-(1,2,4,5,6,7-hexahydro-1-methyl-2,4-dioxo-3H -cyclopenta[d]pyrimidin-3-yl)-benzoesäure und 3,5 g frisch destilliertes Thionylchlorid in 60 ml absolutem Benzol werden unter Rühren 5 bis 6 Stunden auf Rückflusstemperatur erhitzt, und zwar bis zur Bildung einer klaren Lösung. Das Reaktionsgemisch wird zur Trockene eingedampft, das Säurechlorid in 40 ml absolutem Methylenchlorid gelöst, 1,0 g 1-Methoxy-2-propanol und 0,9 g Pyridin zugefügt und das Ganze 1 Stunde bei 23° C gerührt. Anschliessend wird das Reaktionsgemisch zur Trockene eingedampft, der Rückstand in Aethylacetat gelöst und die Lösung gründlich mit Wasser gewaschen. Die organische Phase wird über wasserfreiem Natriumsulfat getrocknet und zur Trockene eingedampft. Der Rückstand wird an 300 g Kieselgel unter Verwendung von Aethylacetat/Methylenchlorid (1:3) als Laufmittel chromatographisch gereinigt. Man erhält den 2-Chlor-5-(1,2,4,5,6,7-hexahydro-1-methyl-2,4-dioxo-3H-cyclopenta[d]pyrimidin-3-yl)-benzoesäure-(2-methoxy-1-methyläthyl)ester, $^{1}$H-NMR ($CDCl_3$, 400 MHz) 7,72 ppm (d, 1H), 7,55 ppm (d, 1H), 7,27 ppm (q, 1H), 5,32 ppm (m, 1H), 3,57 ppm (q, 1H), 3,48 ppm (q, 1H), 3,39 ppm (s, 3H), 3,37 ppm (s, 3H), 2,93 ppm (m, 2H), 2,80 ppm (m, 2H), 2,15 ppm (m, 2H), 1,35 ppm (d, 3H).

In analoger Weise erhält man beim Einsatz von:

2-Chlor-5-(1,2,4,5,6,7-hexahydro-1-methyl-2,4-dioxo-3H -cyclopenta[d]pyrimidin-3-yl)-benzoesäure über das entsprechende Säurechlorid und tert.Butanol den 2-Chlor-5-(1,2,4,5,6,7-hexahydro-1-methyl-2,4-dioxo-3H -cyclopenta[d]pyrimidin-3-yl)-benzoesäure-tert.butylester, Smp. 189-191° C.

2-Chlor-4-fluor-5-(1,2,4,5,6,7-hexahydro -1-methyl-2,4-dioxo-3H-cyclopenta[d]pyrimidin-3-yl)-benzoesäure über das entsprechende Säurechlorid und 1-Methoxy-2-propanol den 2-Chlor-4-fluor-5-(1,2,4,5,6,7-hexahydro-1-methyl -2,4-dioxo-3H-cyclopenta[d]pyrimidin-3-yl)-benzoesäure-(2-methoxy-1 -methyläthyl)-ester, $^{1}$H-NMR ($CDCl_3$, 400 MHz) 7,84 ppm (d,1H), 7,34 ppm (d,1H), 5,30 ppm (m,1H), 3,56 ppm (q,1H), 3,47 ppm (q,1H), 3,40 ppm (s,3H), 3,37 ppm (s,3H), 2,94 ppm (m,2H), 2,80 ppm (m,2H), 2,16 ppm (m,2H), 1,34 ppm (d,3H).

2-Chlor-4-fluor-5-(1,2,4,5,6,7-hexahydro -1-methyl-2,4-dioxo-3H-cyclopenta[d]pyrimidin-3-yl)-benzoesäure über das entsprechende Säurechlorid und tert.Butanol den 2-Chlor-4-fluor-5-(1,2,4,5,6,7-hexahydro-1-methyl -2,4-dioxo-3H-cyclopenta[d]pyrimidin-3-yl)-benzoesäure-tert.butylester, $^{1}$H-NMR ($CDCl_3$, 400 MHz) 7,76 ppm (d,1H), 7,31 ppm (d,1H), 3,41 pp (s,3H), 2,94 ppm (m,2H), 2,81 ppm (m,2H), 2,17 ppm (m,2H), 1,57 ppm (s,9H).

Beispiel 19

Eine Lösung von 3,6 g 2-Chlor-5-[1,4,5,6,7,8-hexahydro-1-methyl-2,4 -dioxo-3(2H)-chinazolinyl--benzoesäure-äthylester in 70 ml Aethanol wird mit 0,6 g Natriumhydroxid in 70 ml Wasser 10 Minuten bei 60° C gehalten und anschliessend 1 Stunde nachgerührt. Die Lösung wird unter vermindertem Druck weitgehend eingedampft und der Rückstand mit 2N Salzsäure auf pH 1 gebracht. Die Fällung wird fünfmal mit jeweils 50 ml Diäthyläther ausgeschüttelt, und die organischen Phasen werden über wasserfreiem Natriumsulfat getrocknet und unter vermindertem Druck zur Trockene eingedampft. Man erhält die 2-Chlor-5-[1,4,5,6,7,8-hexahydro-1-methyl-2,4-dioxo -3(2H)-chinazolinyl]-benzoesäure.

Die freie Säure wird mit 0,84 g Natriumhydrogencarbonat in 50 ml Wasser gerührt und die Lösung unter vermindertem Druck zur Trockene eingedampft. Der Rückstand wird dreimal mit jeweils 50 ml absolutem Aethanol zur Trockene eingedampft und mit etwas Diäthyläther angerieben, und die Kristalle werden abgenutscht und unter vermindertem Druck bei 70° C getrocknet. Man erhält das 2-Chlor-5-[1,4,5,6,7,8-hexahydro-1-methyl -2,4-dioxo-3(2H)-chinazolinyl]-benzoesäure-Natriumsalz.

1,78 g des Natriumsalzes werden in 30 ml absolutem Dimethylformamid gelöst, und die Lösung wird mit 1,2 g Isopropylbromid 1 Stunde auf 100° C erhitzt. Anschliessend wird das Lösungsmittel unter vermindertem Druck entfernt, der Rückstand in 100 ml Aethylacetat gelöst, die Lösung mit Wasser ausgeschüttelt und die organische Phase über wasserfreiem Natriumsulfat getrocknet. Nach Entfernung des Lösungsmittels wird der Rückstand aus Diäthyläther/n-Hexan kristallisiert. Man erhält den 2-Chlor-5-[1,4,5,6,7,8-hexahydro-1-methyl-2,4-dioxo-3(2H) -chinazolinyl]-benzoesäure-isopropylester, Smp. 136-138° C.

In analoger Weise erhält man beim Einsatz von:

2-Chlor-5-[1,4,5,6,7,8-hexahydro-2,4-dioxo -3(2H)-chinazolinyl]-benzoesäure-äthylester den 2-Chlor-5-[1,4,5,6,7,8-hexahydro-2,4-dioxo-3(2H) -chinazolinyl]-benzoesäure-isopropylester, Smp. 205-207° C.

2-Chlor-5-(1,2,4,5,6,7-hexahydro-1-methyl-2,4-dioxo-3H -cyclopenta[d]pyrimidin-3-yl)-benzoesäure-äthylester über das Natrium-Salz der entsprechenden Carbonsäure und Methyljodid, den 2-Chlor-5-(1,2,4,5,6,7-hexahydro-1-methyl-2,4-dioxo-3H -cyclopenta[d]pyrimidin-3-yl)-benzoesäure-methylester, Smp. 164-166° C.

2-Chlor-5-(1,2,4,5,6,7-hexahydro-1-methyl-2,4-dioxo-3H -cyclopenta[d]pyrimidin-3-yl)-benzoesäure-äthylester über das Natriumsalz der entsprechenden Carbonsäure und Isopropylbromid den 2-Chlor-5-(1,2,4,5,6,7-hexahydro-1-methyl-2,4-dioxo-3H -cyclopenta[d]pyrimidin-3-yl)-benzoesäure-isopropylester, Smp. 148-150 ° C.

Beispiel 20

Eine Lösung von 26,6 g 2-Chlor-4-fluor-5-(1,2,4,5,6,7-hexahydro-1-methyl -2,4-dioxo-3H-cyclopenta[d]-pyrimidin-3-yl)-benzoesäure-isopropylester in 1,7 l Methanol wird mit einer Lösung von 3,08 g Natriumhydroxid in 700 ml Wasser versetzt. Das Reaktionsgemisch wird 15 Stunden bei Raumtemperatur gerührt, währenddessen 2,1 l Wasser kontinuierlich zugetropft werden. Es wird weitere 15 Stunden nachgerührt, wobei der pH-Wert des Gemisches 9-10 erreicht. Man säuert das Gemisch mit konzentrierter Salzsäure an und engt es unter vermindertem Druck bei ca. 20 ° C auf ein Volumen von ca. 300 ml ein. Die Kristalle werden abgenutscht, mit etwas Wasser nachgewaschen und bei 40-50 ° C unter vermindertem Druck getrocknet. Man erhält die 2-Chlor-4-fluor-5-(1,2,4, 5,6,7-hexahydro-1-methyl -2,4-dioxo-3H-cyclopenta[d]-pyrimidin-3-yl)-benzoesäure, Smp. 270-273 ° C.

18,85 g der Säure werden mit 4,67 g Natriumhydrogencarbonat in 300 ml Wasser bei 50 ° C 30 Minuten gerührt. Der unlösliche Anteil wird abgenutscht und das Filtrat unter vermindertem Druck bei 40-50 ° C zur Trockene eingedampft. Man löst den Rückstand in 50 ml Methanol und versetzt die Lösung mit 100 ml Benzol und dampft sie unter vermindertem Druck zur Trockene ein. Anschliessend wird mit weiteren 100 ml Benzol zur Trockene eingedampft. Man erhält das 2-Chlor-4-fluor-5-(1,2,4,5,6,7-hexahydro-1-methyl -2,4-dioxo-3H-cyclopenta[d]pyrimidin-3-yl)-benzoesäure-Natriumsalz.

1,9 g des Natriumsalzes werden in 15 ml absolutem Dimethylformamid gelöst und mit 0,6 g 3-Brom-1-propen 2 Stunden bei 70 ° C gerührt. Das Lösungsmittel wird unter vermindertem Druck abdestilliert und der Rückstand in Aethylacetat/Diäthyläther gelöst und mit Wasser ausgeschüttelt. Die organische Phase wird über wasserfreiem Natriumsulfat getrocknet und unter vermindertem Druck zur Trockene eingedampft. Man erhält den 2-Chlor-4-fluor-5-(1,2,4,5,6,7-hexahydro-1-methyl -2,4-dioxo-3H-cyclopenta[d]pyrimidin-3-yl)-benzoesäure-2-propenylester, $^1$H-NMR ($CDCl_3$, 400 MHz) 7,90 ppm (d,1H), 7,35 ppm (d,1H), 6,05-5,94 ppm (m,1H), 5,40 ppm (m,1H), 5,29 ppm (m,1H), 4,79 ppm (m,2H), 3,41 ppm (s,3H), 2,94 ppm (m,2H), 2,81 ppm (m,2H), 2,16 ppm (m,2H).

In analoger Weise erhält man beim Einsatz von:

Natriumsalz der 2-Chlor-4-fluor-5-(1,2,4,5,6,7-hexahydro-1-methyl -2,4-dioxo-3H-cyclopenta[d]pyrimidin-3-yl)-benzoesäure und Chlordimethyläther den 2-Chlor-4-fluor-5-(1,2,4,5,6,7-hexahydro-1-methyl -2,4-dioxo-3H-cyclopenta[d]pyrimidin-3-yl)-benzoesäure-methoxymethylester, $^1$H-NMR ($CDCl_3$, 400 MHz) 7,93 ppm (d,1H), 7,36 ppm (d,1H), 5,45 ppm (s,2H), 3,54 ppm (s,3H), 3,41 ppm (s,3H), 2,95 ppm (m,2H), 2,81 ppm (m,2H), 2,17 ppm (m,2H).

Natriumsalz der 2-Chlor-4-fluor-5-(1,2,4,5,6,7-hexahydro-1-methyl -2,4-dioxo-3H-cyclopenta[d]pyrimidin-3-yl)-benzoesäure und 3-Brom-1-propin den 2-Chlor-4-fluor-5-(1,2,4,5,6,7-hexahydro-1-methyl -2,4-dioxo-3H-cyclopenta[d]pyrimidin-3-yl)-benzoesäure -2-propinylester, Smp. 193-195 ° C.

Natriumsalz der 2-Chlor-4-fluor-5-(1,2,4,5,6,7-hexahydro-1-methyl -2,4-dioxo-3H-cyclopenta[d]pyrimidin-3-yl)-benzoesäure und Methyljodid den 2-Chlor-4-fluor-5-(1,2,4, 5,6,7-hexahydro-1-methyl -2,4-dioxo-3H-cyclopenta[d]pyrimidin-3-yl)-benzoesäure-methylester, Smp. 138-141 ° C.

II. Herstellung der Verbindungen der Formel II:

Beispiel 21

48,4 g 2-Chlor-5-ureido-benzoesäure-äthylester und 31,2 g Cyclopentanon-2-carbonsäure-äthylester werden in 500 ml Benzol und 2 g Toluol-4-sulfonsäure-Monohydrat 6 Stunden am Rückfluss erhitzt. Das sich bildende Wasser wird mittels eines Wasserabscheiders entfernt. Anschliessend wird das Reaktionsgemisch zur Trockene eingedampft, der Rückstand in 700 ml Diäthyläther gelöst und die Lösung filtriert. Das Filtrat wird zur Trockene eingedampft und der Rückstand an 1,5 kg Kieselgel unter Verwendung von Diäthyläther/n-Hexan (1:2) als Laufmittel chromatographisch gereinigt. Man erhält den 2-Chlor-5-{3-[2-(äthoxycarbonyl)-1 -cyclopenten-1-yl]ureido}-benzoesäure-äthylester als farblose Kristalle. Das Produkt wird aus Diäthyläther/n-Hexan umkristallisiert, Smp. 110-112 ° C.

In analoger Weise erhält man beim Einsatz von:

2-Chlor-5-ureidobenzoesäure-äthylester und Cyclohexanon-2-carbonsäure-äthylester den 2-Chlor-5-{3-[2-(äthoxycarbonyl)-1-cyclohexen -1-yl]ureido}-benzoesäure-äthylester, Smp. 119-122 ° C.

2-Chlor-4-fluor-5-ureidobenzoesäure-isopropylester und Cyclopentanon-2-carbonsäure-äthylester den 2-Chlor-4-fluor-5-{3-[2-(äthoxycarbonyl)-1-cyclopenten -1-yl]ureido}-benzoesäure-isopropylester, Smp. 146-149 °C.

2-Chlor-4-fluor-5-ureidobenzoesäure-isopropylester und Cyclohexanon-2-carbonsäure-äthylester den 2-Chlor-4-fluor-5-{3-[2-(äthoxycarbonyl)-1-cyclohexen -1-yl]ureido}-benzoesäure-isopropylester, Smp. 129-130 °C.

2-Nitro-5-ureidobenzoesäure-äthylester und Cyclopentanon-2-carbonsäure-äthylester den 5-{3-[2-(Aethoxycarbonyl)-1-cyclopenten-1-yl]ureido}-2 -nitrobenzoesäure-äthylester, Smp. 152-155 °C.

2-Chlor-4-fluor-5-ureidobenzoesäure-isopropylester und 3-Oxotetrahydrothiophen-2-carbonsäure-methylester den 2-Chlor-4-fluor-5-{3-[2-(methoxycarbonyl)-4,5-dihydro-thien-3-yl]ureido}-benzoesäure-isopropylester, Smp. 161-163 °C.

2-Chlor-4-fluor-5-ureidobenzoesäure-isopropylester und 3-Oxotetrahydrothiophen-4-carbonsäure-methylester den 2-Chlor-4-fluor-5-{3-[4-(methoxycarbonyl) -2,5-dihydro-thien-3-yl]ureido}-benzoesäure-isopropylester.

2-Chlor-5-ureidobenzoesäure-äthylester und 3-Oxotetrahydrothiophen-4-carbonsäure-methylester den 2-Chlor-5-{3-[4-(methoxycarbonyl) -2,5-dihydro-thien-3-yl]ureido}-benzoesäure-äthylester.

2-Chlor-4-fluor-5-ureidobenzoesäure-isopropylester und 3-Oxo-kapronsäure-äthylester den 2-Chlor-4-fluor-5-{3-[2-(äthoxycarbonyl) -1-propylvinyl]ureido}-benzoesäure-isopropylester.

2-Chlor-4-fluor-5-ureidobenzoesäure-isopropylester und 3-Oxo-n-valeriansäure-äthylester unter Verwendung von feinpulverisiertem Amberlyst®-15 (ein freie Sulfongruppen aufweisendes, organisches, polymerisches Harz) als Katalysator den 2-Chlor-4-fluor-5-{3-[2-(äthoxycarbonyl) -1-äthylvinyl]ureido}-benzoesäure-isopropylester, Smp. 123-126 °C.

2,4-Difluor-5-ureidobenzoesäure-isopropylester und Cyclopentanon-2-carbonsäure-äthylester in Toluol den 2,4-Difluor-5-{3-[2-(äthoxycarbonyl) -1-cyclopenten-1-yl]ureido}-benzoesäure-isopropylester, Smp. 149-151 °C.

2,4-Dichlor-5-ureidobenzoesäure-isopropylester und Cyclopentanon-2-carbonsäure-äthylester unter Verwendung von feinpulverisiertem Amberlyst®-15 als Katalysator den 2,4-Dichlor-5-{3-[2-(äthoxycarbonyl)-1-cyclopenten-1-yl]ureido}-benzoesäure-isopropylester, Smp. 140-142 °C.

2-Brom-4-chlor-5-ureidobenzoesäure-isopropylester und Cyclopentanon-2-carbonsäure-äthylester in Toluol den 2-Brom-4-chlor-5-{3-[2-(äthoxycarbonyl) -1-cyclopenten-1-yl]ureido}-benzoesäure-isopropylester, Smp. 131-132 °C.

2,4-Dibrom-5-ureidobenzoesäure-isopropylester und Cyclopentanon-2-carbonsäure-äthylester in Toluol den 2,4-Dibrom-5-{3-[2-(äthoxycarbonyl)-1-cyclopenten -1-yl]ureido}-benzoesäure-isopropylester, Smp. 157-158 °C.

2-Brom-4-fluor-5-ureidobenzoesäure-isopropylester und Cyclopentanon-2-carbonsäure-äthylester in Toluol den 2-Brom-4-fluor-5-{3-[2-(äthoxycarbonyl)-1-cyclopenten -1-yl]ureido}-benzoesäure-isopropylester, Smp. 150-154 °C.

4-Brom-2-fluor-5-ureidobenzoesäure-isopropylester und Cyclopentanon-2-carbonsäure-äthylester in Toluol den 4-Brom-2-fluor-5-{3-[2-(äthoxycarbonyl)-1-cyclopenten-1-yl]ureido}-benzoesäure-isopropylester, Smp. 166-168 °C.

Beispiel 22

4,7 g 3-Amino-2-methylcrotonsäure-äthylester, gelöst in 15 ml absolutem Diäthyläther, wird bei 23 °C unter Rühren mit einer Lösung von 6,7 g 2-Chlor-5-isocyanatobenzoesäure-äthylester versetzt und 2 Stunden nachgerührt. Das Reaktionsgemisch wird unter vermindertem Druck zur Trockene eingedampft und der Rückstand an 400 g Kieselgel unter Verwendung von Diäthyläther/n-Hexan (1:1) als Laufmittel chromatographisch gereinigt. Man erhält den 2-Chlor-5-{3-[2-(äthoxycarbonyl)-1 -methylpropenyl]ureido}-benzoesäure-äthylester, Smp. 88-91 °C.

In analoger Weise erhält man bei Einsatz von:

2-Chlor-4-fluor-5-isocyanatobenzoesäure-isopropylester und 3-Amino-2-methylcrotonsäure-äthylester den 2-Chlor-4-fluor-5-{3-[2-(äthoxycarbonyl) -1-methylpropenyl]ureido}-benzoesäure-isopropylester, Smp. 145-147 °C.

2-Chlor-5-isocyanatobenzoesäure-äthylester und 3-Aminocrotonsäure-äthylester den 2-Chlor-5-{3-[2-(äthoxycarbonyl) -1-methylvinyl]ureido}-benzoesäure-äthylester.

2-Chlor-4-fluor-5-isocyanatobenzoesäure-isopropylester und 3-Aminocrotonsäure-äthylester den 2-Chlor-4-fluor-5-{3-[2-(äthoxycarbonyl) -1-methylvinyl]ureido}-benzoesäure-isopropylester, Smp. 147-150 °C.

2-Chlor-4-fluor-5-isocyanatobenzoesäure-isopropylester und 3-Amino-2-fluorcrotonsäure-äthylester den

2-Chlor-4-fluor-5-{3-[2-(äthoxycarbonyl)-2-fluor -1-methylvinyl]-ureido}-benzoesäure-isopropylester, Smp. 150-152 ° C.

2-Chlor-4-fluor-5-isocyanatobenzoesäure-isopropylester und 3-Amino-2-äthylcrotonsäure-äthylester in Dimethylformamid den 2-Chlor-4-fluor-5-{3-[2-(äthoxycarbonyl)-1-methyl-1-butenyl]ureido} -benzoesäure-isopropylester, Smp. 112-115 ° C.

2-Chlor-4-fluor-5-isocyanatobenzoesäure-isopropylester und 3-Amino-2-methyl-2-pentensäure-äthylester den 2-Chlor-4-fluor-5-{3-[2-(äthoxycarbonyl)-1-äthyl -1-propenyl]ureido}-benzoesäure-isopropylester, Smp. 132-133 ° C.

2-Chlor-4-fluor-5-isocyanatobenzoesäure-isopropylester und 2-Amino-4,5-dihydro-3-carbonsäure-äthylester den 2-Chlor-4-fluor-5-{3-[3-(äthoxycarbonyl)-4,5-dihydrofuran-2-yl]ureido}-benzoesäure-isopropylester.

Beispiel 23

6,0 g 2-Chlor-5-ureidobenzoesäure-äthylester und 6,4 g 3-Aethoxyacrylsäure-äthylester werden in 100 ml 1,2-Dimethoxyäthan auf Rückflusstemperatur erhitzt und anschliessend mit 12 ml 2N Salzsäure 5 Minuten bei dieser Temperatur erhitzt. Das Reaktionsgemisch wird unter vermindertem Druck zur Trockene eingedampft und der Rückstand an 300 g Kieselgel unter Verwendung von Aethylacetat/n-Hexan (1:3) als Laufmittel chromatographisch gereinigt. Man erhält den 2-Chlor-5-{3-[2 -(äthoxycarbonyl)vinyl]ureido}-benzoesäure-äthylester, Smp. 116-117 ° C.

Beispiel 24

27,4 g 2-Chlor-4-fluor-5-ureidobenzoesäure-isopropylester und 13,0 g 3-Methoxy-2-methyl-acrylsäure-methylester werden in 250 ml Benzol mit 1,9 g Toluol-4-sulfonsäure-Monohydrat 2 Stunden auf Rückflusstemperatur erhitzt. Das Reaktionsgemisch wird unter vermindertem Druck zur Trockene eingedampft und der Rückstand mit 400 ml Diäthyläther gerührt. Der unlösliche Teil wird abgenutscht und das Filtrat unter vermindertem Druck zur Trockene eingedampft. Der Rückstand wird an 1 kg Kieselgel unter Verwendung von Aethylacetat/n-Hexan (1:3) als Laufmittel chromatographisch gereinigt. Man erhält den 2-Chlor-4-fluor-5-{3-[2-(methoxycarbonyl)propenyl]ureido} -benzoesäure-isopropylester, Smp. 189-190 ° C.

III. Herstellung der Verbindungen der Formel VI:

Beispiel 25

Zu 0,71 g einer 55%igen Natriumhydrid-Dispersion in 25 ml Dimethylformamid werden 1,52 g 3-Amino-2-cyanacrylsäurenitril zugefügt, und das Gemisch wird 30 Minuten bei 30 ° C gerührt. Nach Beendigung der Wasserstoffentwicklung wird das Gemisch mit 4,20 g 2-Chlor-4-fluor-5-isocyanatobenzoesäure-isopropylester versetzt, währenddessen die Temperatur auf 30 ° C ansteigt. Anschliessend wird 2 Stunden bei Raumtemperatur nachgerührt, das Reaktionsgemisch auf Wasser gegossen, und das wässrige Gemisch mit Essigsäure angesäuert und zweimal mit 100 ml Aethylacetat extrahiert. Die organische Phase wird mit Wasser gewaschen, über wasserfreiem Natriumsulfat getrocknet und unter vermindertem Druck zur Trockene eingedampft. Der Rückstand wird in 50 ml Methylenchlorid gelöst und durch Zugabe von Diäthyläther und Kühlen auf 0 ° C zur Kristallisation gebracht. Man erhält den 5-[6-Amino-5-cyano-2-oxo-1(2H)-pyrimidinyl]-2-chlor-4-fluorbenzoesäure-isopropylester, Smp. 212-213 ° C.

In analoger Weise erhält man beim Einsatz von:

3-Amino-2-cyanatocrotonsäurenitril und 2-Chlor-4-fluor-5-isocyanatobenzoesäure-isopropylester den 5-[6-Amino-5-cyano-4-methyl-2-oxo -1(2H)-pyrimidinyl]-2-chlor-4-fluor-benzoesäure-isopropylester, Smp. 255-257 ° C.

IV. Formulierungsbeispiele:

Beispiel 26

Zur Herstellung eines 50% Spritzpulvers werden die nachstehend aufgeführten Bestandteile miteinander vermischt:

| | |
|---|---|
| Verbindung der Formel I' oder II | 50 g |
| Kieselsäure, hydratisiert | 5 g |
| Natrium-laurylsulfat | 1 g |
| Natrium-lignosulfonat | 2 g |
| Kaolin | 42 g |
| | 100 g |

Diese Mischung wird in einer geeigneten Mühle feingemahlen.

**Patentansprüche**

**Patentansprüche für folgende Vertragsstaaten : BE, CH, DE, FR, GB, IT, LI, LU, NL, SE**

**1.** Verbindungen der allgemeinen Formel

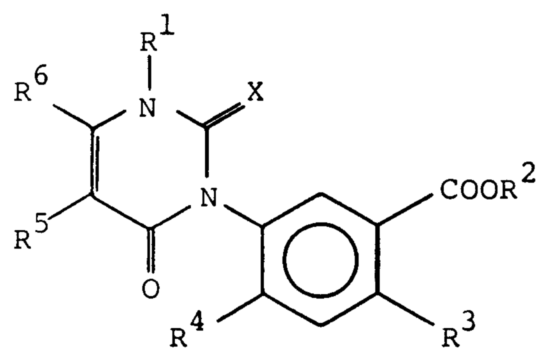

I

worin

| | |
|---|---|
| $R^1$ | Wasserstoff, $C_{1-4}$-Alkyl, $C_{2-4}$-Alkenyl, $C_{2-4}$-Alkinyl, $C_{2-6}$-Alkoxyalkyl, Formyl, $C_{2-6}$-Alkanoyl oder $C_{2-6}$-Alkoxycarbonyl, |
| $R^2$ | Wasserstoff, $C_{1-6}$-Alkyl, $C_{2-4}$-Alkenyl, $C_{2-4}$-Alkinyl oder $C_{2-6}$-Alkoxyalkyl, |
| $R^3$ | Halogen oder Nitro, |
| $R^4$ | Wasserstoff oder Halogen, |
| $R^5$ | Wasserstoff, Halogen, $C_{1-4}$-Alkyl, Chlormethyl, Brommethyl, Hydroxymethyl, ($C_{1-5}$-Alkoxy)methyl, ($C_{1-5}$-Alkylthio)methyl, Cyano, Nitro oder Thiocyanato, |
| $R^6$ | Wasserstoff, $C_{1-4}$-Alkyl oder $C_{1-4}$-Fluoralkyl, oder |
| $R^5$ und $R^6$ | zusammen Tri- oder Tetramethylen, in welchem ein Methylen durch Sauerstoff oder Schwefel ersetzt sein kann, und welches gegebenenfalls mit $C_{1-3}$-Alkyl substituiert ist, und |
| X | Sauerstoff oder Schwefel bedeuten, mit den Massgaben, dass (i) falls $R^5$ für Fluor steht, $R^6$ ausschliesslich $C_{1-4}$-Alkyl oder $C_{1-4}$-Fluoralkyl bedeutet, und (ii) falls $R^5$ für Cyano steht, $R^6$ ausschliesslich Wasserstoff oder $C_{1-4}$-Alkyl und X ausschliesslich Sauerstoff bedeuten, |

sowie Salze derjenigen Verbindungen der Formel I, in denen $R^1$ und/oder $R^2$ Wasserstoff bedeutet.

**2.** Verbindungen der allgemeinen Formel

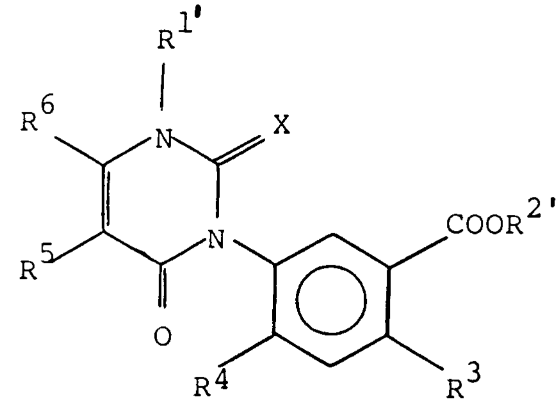

I'

worin

| | |
|---|---|
| $R^{1'}$ | $C_{1-4}$-Alkyl, $C_{2-4}$-Alkenyl, $C_{2-4}$-Alkinyl, $C_{2-6}$-Alkoxyalkyl, Formyl, $C_{2-6}$-Alkanoyl oder $C_{2-6}$-Alkoxycarbonyl, |
| $R^{2'}$ | $C_{1-6}$-Alkyl, $C_{2-4}$-Alkenyl, $C_{2-4}$-Alkinyl oder $C_{2-6}$-Alkoxyalkyl, |
| $R^3$ | Halogen oder Nitro, |
| $R^4$ | Wasserstoff oder Halogen, |
| $R^5$ | Wasserstoff, Halogen, $C_{1-4}$-Alkyl, Chlormethyl, Brommethyl, Hydroxymethyl, ($C_{1-5}$-Alkoxy)methyl, ($C_{1-5}$-Alkylthio)methyl, Cyano, Nitro oder Thiocyanato, |
| $R^6$ | Wasserstoff, $C_{1-4}$-Alkyl oder $C_{1-4}$-Fluoralkyl, |
| | oder |
| $R^5$ und $R^6$ | zusammen Tri- oder Tetramethylen, in welchem ein Methylen durch Sauerstoff oder Schwefel ersetzt sein kann, und welches gegebenenfalls mit $C_{1-3}$-Alkyl substituiert ist, und |
| X | Sauerstoff oder Schwefel bedeuten, mit den Massgaben, dass (i) falls $R^5$ für Fluor steht, $R^6$ ausschliesslich $C_{1-4}$-Alkyl oder $C_{1-4}$-Fluoralkyl bedeutet, und (ii) falls $R^5$ für Cyano steht, $R^6$ ausschliesslich Wasserstoff oder $C_{1-4}$-Alkyl und X ausschliesslich Sauerstoff bedeuten. |

**3.** Verbindungen nach Anspruch 1 oder 2, worin $R^1$ bzw. $R^{1'}$ Methyl bedeutet.

**4.** Verbindungen nach einem der Ansprüche 1 bis 3, worin $R^2$ bzw. $R^{2'}$ $C_{1-6}$-Alkyl oder $C_{2-6}$-Alkoxyalkyl bedeutet.

**5.** Verbindungen nach einem der Ansprüche 1 bis 4, worin $R^3$ Chlor oder Brom bedeutet.

**6.** Verbindungen nach einem der Ansprüche 1 bis 5, worin $R^4$ Fluor bedeutet.

**7.** Verbindungen nach einem der Ansprüche 1 bis 6, worin $R^5$ Wasserstoff, Chlor, Brom, Methyl oder Aethyl bedeutet.

**8.** Verbindungen nach einem der Ansprüche 1 bis 6, worin $R^5$ Fluor bedeutet.

**9.** Verbindungen nach einem der Ansprüche 1 bis 7, worin $R^6$ Methyl, Aethyl oder Trifluormethyl bedeutet.

**10.** Verbindungen nach einem der Ansprüche 1 bis 6, worin $R^5$ und $R^6$ zusammen Tri- oder Tetramethylen bedeuten.

**11.** Eine Verbindung nach Anspruch 1 oder 2, ausgewählt aus:

2-Chlor-4-fluor-5-(1,2,4,5,6,7-hexahydro-1-methyl-2,4-dioxo-3H-cyclopenta[d]pyrimidin-3-yl)-benzoesäure-isopropylester,

2-Chlor-4-fluor-5-[1,4,5,6,7,8-hexahydro-1-methyl-2,4-dioxo-3(2H)-chinazolinyl]-benzoesäure-isopropylester,

2-Chlor-4-fluor-5-[3,6-dihydro-3,4-dimethyl-2,6-dioxo-1(2H)-pyrimidinyl]-benzoesäure-isopropylester,
2-Chlor-4-fluor-5-[5-brom-3,6-dihydro-3,4-dimethyl-2,6-dioxo-1(2H)-pyrimidinyl]-benzoesäure-isopropylester,
2-Chlor-4-fluor-5-[3,6-dihydro-3,4-dimethyl-5-jod-2,6-dioxo-1(2H)-pyrimidinyl]-benzoesäure-isopropylester,
2-Chlor-4-fluor-5-[3,6-dihydro-3,4-dimethyl-5-hydroxymethyl-2,6-dioxo-1(2H)-pyrimidinyl]-benzoesäure-isopropylester,
2-Chlor-4-fluor-5-[4-äthyl-3,6-dihydro-3-methyl-2,6-dioxo-1(2H)-pyrimidinyl]-benzoesäure-isopropylester,
2-Chlor-4-fluor-5-[3,6-dihydro-3-methyl-4-trifluormethyl-2,6-dioxo-1(2H)-pyrimidinyl]-benzoesäure-isopropylester,
2-Brom-4-fluor-5-(1,2,4,5,6,7-hexahydro-1-methyl-2,4-dioxo-3H-cyclopenta[d]pyrimidin-3-yl)-benzoesäure-isopropylester,
2-Chlor-4-fluor-5-[3,6-dihydro-3,4-dimethyl-5-nitro-2,6-dioxo-1(2H)-pyrimidinyl]-benzoesäure-isopropylester,
2-Chlor-4-fluor-5-(1,2,4,5,6,7-hexahydro-1-methyl-2,4-dioxo-3H-cyclopenta[d]pyrimidin-3-yl)-benzoesäure-(2-methoxy-1-methyläthyl)ester und
2-Chlor-4-fluor-5-(1,2,4,5,6,7-hexahydro-1-methyl-2,4-dioxo-3H-cyclopenta[d]pyrimidin-3-yl)-benzoesäure-tert.butylester.

**12.** Eine Verbindung nach Anspruch 1 oder 2, ausgewählt aus:
2-Chlor-4-fluor-5-[3,6-dihydro-3,4-dimethyl-5-fluor-2,6-dioxo-1(2H)-pyrimidinyl]-benzoesäure-isopropylester,
2-Chlor-4-fluor-5-[3,6-dihydro-3-methyl-4-propyl-2,6-dioxo-1(2H)-pyrimidinyl]-benzoesäure-isopropylester und
2-Chlor-4-fluor-5-[3,6-dihydro-5-fluor-3-methyl-4-trifluormethyl-2,6-dioxo-1(2H)-pyrimidinyl]-benzoesäure-isopropylester.

**13.** Eine Verbindung nach Anspruch 1 oder 2, ausgewählt aus:
2-Chlor-5-(1,2,4,5,6,7-hexahydro-2,4-dioxo-3H-cyclopenta[d]pyrimidin-3-yl)-benzoesäure-äthylester,
2-Chlor-4-fluor-5-(1,2,4,5,6,7-hexahydro-2,4-dioxo-3H-cyclopenta[d]pyrimidin-3-yl)-benzoesäure-isopropylester,
2-Chlor-4-fluor-5-[1,4,5,6,7,8-hexahydro-2,4-dioxo-3(2H)-chinazolinyl]-benzoesäure-isopropylester,
2-Chlor-4-fluor-5-[3,6-dihydro-4-methyl-2,6-dioxo-1(2H)-pyrimidinyl]-benzoesäure-isopropylester,
2-Chlor-4-fluor-5-[3,6-dihydro-5-methyl-2,6-dioxo-1(2H)-pyrimidinyl]-benzoesäure-isopropylester,
2-Brom-4-fluor-5-(1,2,4,5,6,7-hexahydro-2,4-dioxo-3H-cyclopenta[d]pyrimidin-3-yl)-benzoesäure-isopropylester,
2-Chlor-4-fluor-5-{1,2,5,7-tetrahydro-2,4-dioxo-thieno-[3,4-d]pyrimidin-3(4H)-yl}-benzoesäure-isopropylester,
2-Chlor-4-fluor-5-[3-äthyl-3,6-dihydro-5-methyl-2,6-dioxo-1(2H)-pyrimidinyl]-benzoesäure-isopropylester,
2-Chlor-5-(1,2,4,5,6,7-hexahydro-1-methyl-2,4-dioxo-3H-cyclopenta[d]pyrimidin-3-yl)-benzoesäure-äthylester,
2-Chlor-5-[1,4,5,6,7,8-hexahydro-1-methyl-2,4-dioxo-3(2H)-chinazolinyl]-benzoesäure-äthylester,
2-Chlor-4-fluor-5-[4-äthyl-3,6-dihydro-2,6-dioxo-1(2H)-pyrimidinyl]-benzoesäure-äthylester,
2-Chlor-5-[3,6-dihydro-3,4-dimethyl-2,6-dioxo-1(2H)-pyrimidinyl]-benzoesäure-äthylester,
2-Chlor-5-[3,6-dihydro-3,4,5-trimethyl-2,6-dioxo-1(2H)-pyrimidinyl]-benzoesäure-äthylester,
2-Chlor-4-fluor-5-[3,6-dihydro-3,4,5-trimethyl-2,6-dioxo-1(2H)-pyrimidinyl]-benzoesäure-isopropylester,
2-Chlor-4-fluor-5-{1,2,5,7-tetrahydro-1-methyl-2,4-dioxo-thieno[3,4-d]pyrimidin-3(4H)-yl}-benzoesäure-isopropylester,
2-Chlor-4-fluor-5-[4-äthyl-3,6-dihydro-3,5-dimethyl-2,6-dioxo-1(2H)-pyrimidinyl]-benzoesäure-isopropylester,
2-Chlor-4-fluor-5-[5-cyano-3,6-dihydro-3,4-dimethyl-2,6-dioxo-1(2H)-pyrimidinyl]-benzoesäure-isopropylester,
2,4-Dichlor-5-(1,2,4,5,6,7-hexahydro-1-methyl-2,4-dioxo-3H-cyclopenta[d]pyrimidin-3-yl)-benzoesäure-isopropylester,
2-Brom-4-chlor-5-(1,2,4,5,6,7-hexahydro-1-methyl-2,4-dioxo-3H-cyclopenta[d]pyrimidin-3-yl)-

benzoesäure-isopropylester,
2-Chlor-4-fluor-5-(1,2,4,5,6,7-hexahydro-1-acetyl-2,4-dioxo-3H-cyclopenta[d]pyrimidin-3-yl)-benzoesäure-isopropylester,
2-Chlor-4-fluor-5-{1,2,4,5,6,7-hexahydro-1-methoxycarbonyl-2,4-dioxo-3H-cyclopenta[d]pyrimidin-3-yl}-benzoesäure-isopropylester,
2-Chlor-4-fluor-5-[5-chlor-3,6-dihydro-3,4-dimethyl-2,6-dioxo-1(2H)-pyrimidinyl]-benzoesäure-isopropylester,
2-Chlor-4-fluor-5-[5-brom-3,6-dihydro-4-methyl-2,6-dioxo-1(2H)-pyrimidinyl]-benzoesäure-isopropylester,
2-Chlor-5-(1,2,4,5,6,7-hexahydro-1-methyl-2,4-dioxo-3H-cyclopenta[d]pyrimidin-3-yl)-benzoesäure-tert.butylester,
2-Chlor-5-[1,4,5,6,7,8-hexahydro-1-methyl-2,4-dioxo-3(2H)-chinazolinyl]-benzoesäure-isopropylester,
2-Chlor-5-[1,4,5,6,7,8-hexahydro-2,4-dioxo-3(2H)-chinazolinyl]-benzoesäure-isopropylester,
2-Chlor-4-fluor-5-(1,2,4,5,6,7-hexahydro-1-methyl-2,4-dioxo-3H-cyclopenta[d]pyrimidin-3-yl)-benzoesäure-2-propenylester,
2-Chlor-4-fluor-5-(1,2,4,5,6,7-hexahydro-1-methyl-2,4-dioxo-3H-cyclopenta[d]pyrimidin-3-yl)-benzoesäure-2-propinylester und
2-Chlor-4-fluor-5-(1,2,4,5,6,7-hexahydro-1-methyl-2,4-dioxo-3H-cyclopenta[d]pyrimidin-3-yl)-benzoesäure-methylester.

**14.** Eine Verbindung nach Anspruch 1 oder 2, ausgewählt aus:
2-Chlor-4-fluor-5-[3,6-dihydro-5-fluor-4-methyl-2,6-dioxo-1(2H)-pyrimidinyl]-benzoesäure-isopropylester,
2-Chlor-4-fluor-5-[3,6-dihydro-5-fluor-4-fluormethyl-2,6-dioxo-1(2H)-pyrimidinyl]-benzoesäure-isopropylester,
2-Chlor-4-fluor-5-[3,6-dihydro-5-fluor-4-fluormethyl-3-methyl-2,6-dioxo-1(2H)-pyrimidinyl]-benzoesäure-isopropylester,
2-Chlor-4-fluor-5-[3,6-dihydro-4-propyl-2,6-dioxo-1(2H)-pyrimidinyl]-benzoesäure-isopropylester,
2-Chlor-4-fluor-5-[5-chlor-3,6-dihydro-4-methyl-2,6-dioxo-1(2H)-pyrimidinyl]-benzoesäure-isopropylester und
2-Chlor-4-fluor-5-[3,6-dihydro-3,4-dimethyl-5-methoxy-methyl-2,6-dioxo-1(2H)-pyrimidinyl]-benzoesäure-isopropylester.

**15.** Verbindungen der allgemeinen Formel

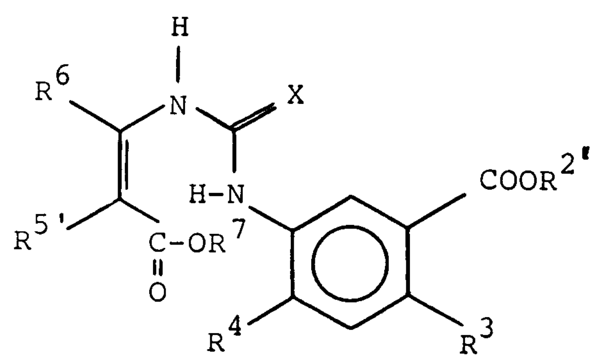

II

worin

| | |
|---|---|
| $R^{2'}$ | $C_{1-6}$-Alkyl, $C_{2-4}$-Alkenyl, $C_{2-4}$-Alkinyl oder $C_{2-6}$-Alkoxyalkyl, |
| $R^3$ | Halogen oder Nitro, |
| $R^4$ | Wasserstoff oder Halogen, |
| $R^{5'}$ | Wasserstoff, Fluor oder $C_{1-4}$-Alkyl, |
| $R^6$ | Wasserstoff, $C_{1-4}$-Alkyl oder $C_{1-4}$-Fluoralkyl, |
| | oder |
| $R^{5'}$ und $R^6$ | zusammen Tri- oder Tetramethylen, in welchem ein Methylen durch Sauerstoff oder Schwefel ersetzt sein kann, und welches gegebenenfalls mit $C_{1-3}$-Alkyl substituiert ist, |

$R^7$ $C_{1-4}$-Alkyl
und
X Sauerstoff oder Schwefel bedeuten, mit der Massgabe, dass falls $R^{5'}$ für Fluor steht, $R^6$ ausschliesslich $C_{1-4}$-Alkyl oder $C_{1-4}$-Fluoralkyl bedeutet.

**16.** 2-Chlor-4-fluor-5-{3-[2-(äthoxycarbonyl)-1-cyclohexen-1-yl]ureido}-benzoesäure-isopropylester.

**17.** 2-Chlor-4-fluor-5-{3-[2-(äthoxycarbonyl)-1-methylpropenyl]ureido}-benzoesäure-isopropylester.

**18.** Unkrautbekämpfungsmittel, dadurch gekennzeichnet, dass es eine wirksame Menge mindestens einer Verbindung der allgemeinen Formel I' gemäss Anspruch 2 sowie Formulierungshilfsstoffe enthält.

**19.** Unkrautbekämpfungsmittel nach Anspruch 18, dadurch gekennzeichnet, dass es eine wirksame Menge mindestens einer der in Anspruch 11 genannten Verbindungen sowie Formulierungshilfsstoffe enthält.

**20.** Unkrautbekämpfungsmittel nach Anspruch 18, dadurch gekennzeichnet, dass es eine wirksame Menge mindestens einer der in Anspruch 12 genannten Verbindungen sowie Formulierungshilfsstoffe enthält.

**21.** Verfahren zur Herstellung von Verbindungen der allgemeinen Formel I gemäss Anspruch 1 und von Salzen derjenigen Verbindungen der Formel I, in denen $R^1$ und/oder $R^2$ Wasserstoff bedeutet, dadurch gekennzeichnet, dass man

a) zwecks Herstellung derjenigen Verbindungen der Formel I, in denen $R^1$ Wasserstoff und $R^2$ $C_{1-6}$-Alkyl, $C_{2-4}$-Alkenyl, $C_{2-4}$-Alkinyl oder $C_{2-6}$-Alkoxyalkyl bedeuten und $R^5$ verschieden von Chlor, Brom, Jod, Chlormethyl, Brommethyl, Hydroxymethyl, ($C_{1-5}$-Alkoxy)methyl, ($C_{1-5}$-Alkylthio)methyl, Cyano, Nitro oder Thiocyanato ist, sowie von Metallsalzen jener Verbindungen der Formel I, in denen $R^1$ Wasserstoff bedeutet, eine Verbindung der allgemeinen Formel

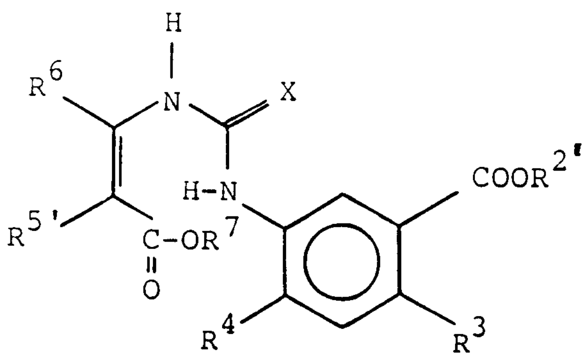

II

worin
$R^{2'}$ $C_{1-6}$-Alkyl, $C_{2-4}$-Alkenyl, $C_{2-4}$-Alkinyl oder $C_{2-6}$-Alkoxyalkyl bedeutet,
$R^3$, $R^4$, $R^6$ und X die oben angegebenen Bedeutungen besitzen,
$R^{5'}$ Wasserstoff, Fluor, $C_{1-4}$-Alkyl oder zusammen mit $R^6$ gegebenenfalls modifiziertes Tri- oder Tetramethylen, wie dies oben näher definiert ist, bedeutet, und
$R^7$ nieder Alkyl bedeutet,

einer basekatalysierten Cyclisierung durch Behandlung mit einer Base unterwirft und gewünschtenfalls eine allfällig erhaltene Metallsalzform des Uracilderivats durch Behandlung mit einer Säure in die entsprechende saure Form ($R^1$ = Wasserstoff) überführt,

b) zwecks Herstellung derjenigen Verbindungen der Formel I, in denen $R^1$ $C_{1-4}$-Alkyl, $C_{2-4}$-Alkenyl, $C_{2-4}$-Alkinyl, $C_{2-6}$-Alkoxyalkyl, Formyl, $C_{2-6}$-Alkanoyl oder $C_{2-6}$--Alkoxycarbonyl bedeutet, ein Uracilderivat der allgemeinen Formel

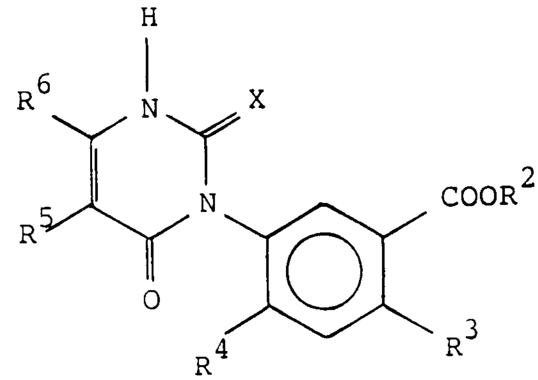

Ia

worin $R^2$, $R^3$, $R^4$, $R^5$, $R^6$ und X die oben angegebenen Bedeutungen besitzen, zur Alkylierung bzw. Acylierung mit einem entsprechenden eine $C_{1-4}$-Alkyl-, $C_{2-4}$-Alkenyl-, $C_{2-4}$-Alkinyl-, $C_{2-6}$-Alkoxyalkyl-, Formyl-, $C_{2-6}$-Alkanoyl- oder $C_{2-6}$-Alkoxycarbonylgruppe enthaltenden Alkylierungs- bzw. Acylierungsmittel behandelt,

c) zwecks Herstellung derjenigen Verbindungen der Formel I, in denen $R^1$ $C_{1-4}$-Alkyl, $C_{2-4}$-Alkenyl, $C_{2-4}$-Alkinyl oder $C_{2-6}$-Alkoxyalkyl bedeutet, $R^5$ verschieden von Chlor, Brom, Jod, Chlormethyl, Brommethyl, Hydroxymethyl, ($C_{1-5}$-Alkoxy)methyl, ($C_{1-5}$-Alkylthio)methyl, Cyano, Nitro oder Thiocyanato ist, und X Schwefel bedeutet, eine Verbindung der allgemeinen Formel

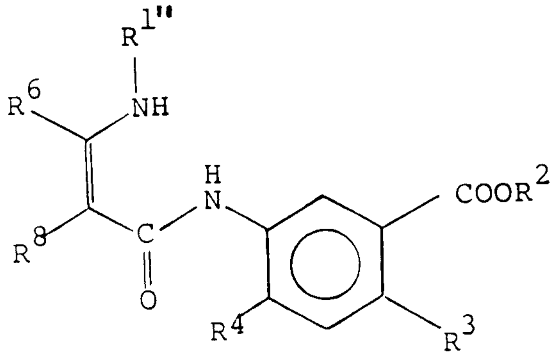

III

worin

| | |
|---|---|
| $R^{1''}$ | $C_{1-4}$-Alkyl, $C_{2-4}$-Alkenyl, $C_{2-4}$-Alkinyl oder $C_{2-6}$-Alkoxyalkyl bedeutet, |
| $R^2$, $R^3$, $R^4$ und $R^6$ | die oben angegebenen Bedeutungen besitzen und |
| $R^8$ | Fluor. $C_{1-4}$-Alkyl, ($C_{1-4}$-Alkoxy)carbonyl oder zusammen mit $R^6$ wie oben definiertes Tri- oder Tetramethylen bedeutet, wobei, im Falle von $R^8$ = Fluor, $R^6$ $C_{1-4}$-Alkyl oder $C_{1-4}$-Fluoralkyl ist, |

mit N,N'-Thiocarbonyldiimidazolid oder Thiophosgen umsetzt und, falls $R^8$ ($C_{1-4}$-Alkoxy)carbonyl bedeutet, das so hergestellte 5-Alkoxycarbonyl-2-thiouracil der allgemeinen Formel

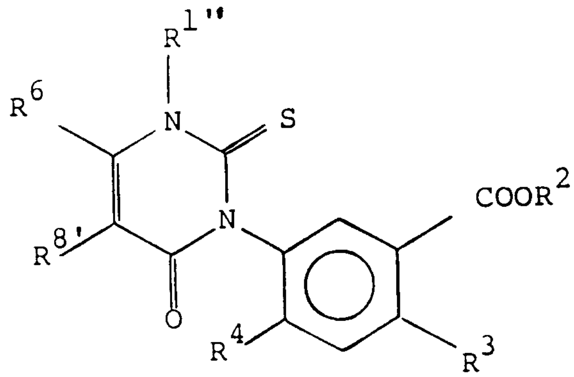

IV

worin

$R^{1''}$, $R^2$, $R^3$, $R^4$ und $R^6$ die oben angegebenen Bedeutungen besitzen und

$R^{8'}$ ($C_{1-4}$-Alkoxy)carbonyl bedeutet,

zur Hydrolyse und Decarboxylierung in Gegenwart von wässriger Salzsäure oder Trifluoressigsäure aufwärmt,

d) zwecks Herstellung derjenigen Verbindungen der Formel I, in denen $R^5$ Chlor, Brom oder Jod bedeutet, ein Uracilderivat der allgemeinen Formel

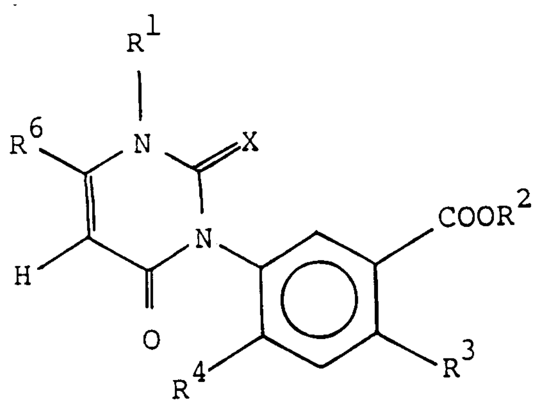

Ib

worin $R^1$, $R^2$, $R^3$, $R^4$, $R^6$ und X die oben angegebenen Bedeutungen besitzen,

zur Chlorierung mit elementarem Chlor oder Sulfurylchlorid, zur Bromierung mit elementarem Brom oder Sulfurylbromid bzw. zur Jodierung mit elementarem Jod behandelt,

e) zwecks Herstellung derjenigen Verbindungen der Formel I, in denen $R^5$ Chlor- oder Brommethyl bedeutet, (i) ein Uracilderivat der oben angegebenen Formel Ib mit Chlor- bzw. Brommethoxymethan behandelt oder (ii) ein 5-Hydroxymethyluracil der allgemeinen Formel

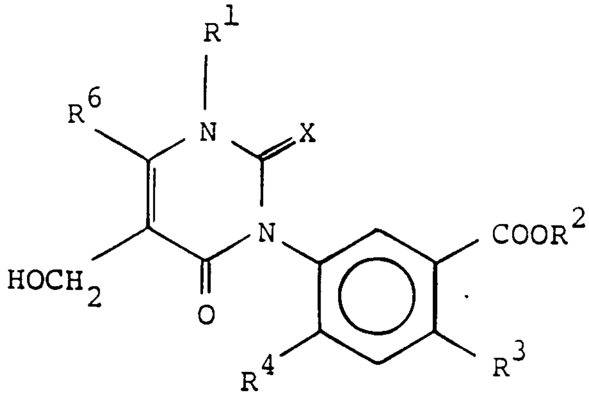

Ic

worin $R^2$, $R^3$, $R^4$, $R^6$ und X die oben angegebenen Bedeutungen besitzen, mit Thionylchlorid bzw. -bromid behandelt oder (iii) ein 5-Methyluracil der allgemeinen Formel

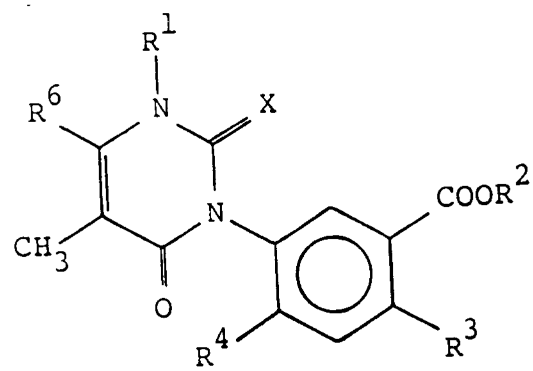

Id

worin $R^1$, $R^2$, $R^3$, $R^4$, $R^6$ und X die oben angegebenen Bedeutungen besitzen, mit N-Chlorsuccinimid bzw. N-Bromsuccinimid behandelt,

f) zwecks Herstellung derjenigen Verbindungen der Formel I, in denen $R^5$ Hydroxymethyl bedeutet, ein 5-Halogenmethyluracil der allgemeinen Formel

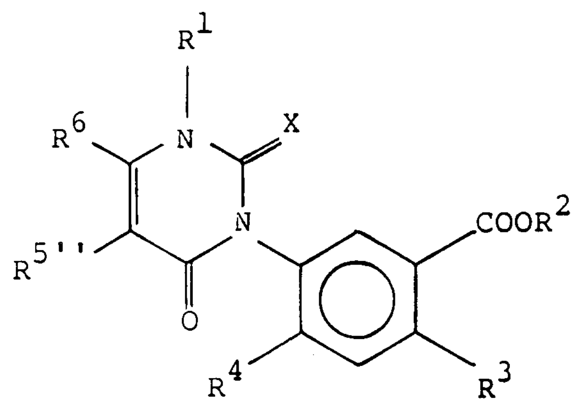

Ie

worin $R^1$, $R^2$, $R^3$, $R^4$, $R^6$ und X die oben angegebenen Bedeutungen besitzen und $R^{5''}$ Chlor-, Brom- oder Jodmethyl bedeutet, zur Hydrolyse mit einer wässrigen Lösung einer anorganischen Base behandelt,

g) zwecks Herstellung derjenigen Verbindungen der Formel I, in denen $R^5$ ($C_{1-5}$-Alkoxy)methyl oder ($C_{1-5}$-Alkylthio)-methyl bedeutet, ein 5-Halogenmethyluracil der oben angegebenen Formel Ie, in der $R^{5''}$ Chlor- oder Brommethyl bedeutet, mit einem Alkalimetallalkoholat oder -thioalkoholat der allgemeinen Formel

$R^9M$ V

worin

$R^9$ $C_{1-5}$-Alkoxy oder $C_{1-5}$-Alkylthio

und M ein Alkalimetall bedeuten,

oder mit einem $C_{1-5}$-Alkanol oder $C_{1-5}$-Alkylmercaptan behandelt,

h) zwecks Herstellung derjenigen Verbindungen der Formel I, in denen $R^5$ ($C_{1-5}$-Alkylthio)methyl bedeutet, ein 5-Hydroxymethyluracil der oben angegebenen Formel Ic mit einem $C_{1-5}$-Alkylmercaptan behandelt,

i) zwecks Herstellung derjenigen Verbindungen der Formel I, in denen $R^1$ Wasserstoff, $R^5$ Cyano, $R^6$ Wasserstoff oder $C_{1-4}$-Alkyl und X Sauerstoff bedeuten, eine Verbindung der allgemeinen Formel

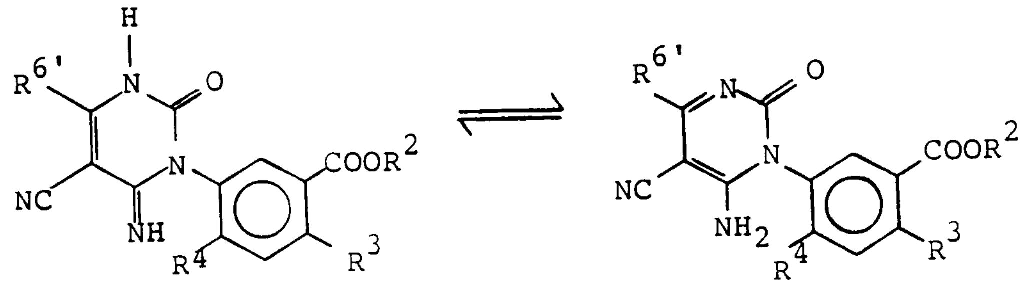

VI

worin $R^2$, $R^3$ und $R^4$ die oben angegebenen Bedeutungen besitzen und $R^{6'}$ Wasserstoff oder $C_{1-4}$-Alkyl bedeutet,
zur säurekatalysierten Hydrolyse mit einer wässrigen Lösung einer Mineralsäure behandelt,
j) zwecks Herstellung derjenigen Verbindungen der Formel I, in denen $R^5$ Nitro bedeutet, ein Uracilderivat der oben angegebenen Formel Ib mit Salpetersäure behandelt,
k) zwecks Herstellung derjenigen Verbindungen der Formel I, in denen $R^5$ Thiocyanato bedeutet, ein Uracilderivat der oben angegebenen Formel Ib mit Thiocyanogen behandelt,
l) Zwecks Herstellung derjenigen Verbindungen der Formel I, in denen $R^2$ Wasserstoff bedeutet, einen Benzoesäureester der allgemeinen Formel

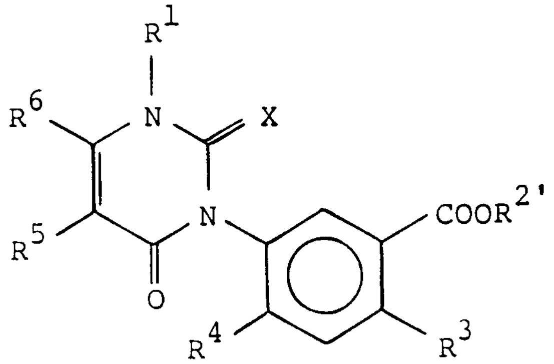

If

worin $R^1$, $R^2$, $R^3$, $R^4$, $R^5$, $R^6$ und X die oben angegebenen Bedeutungen besitzen,
durch Behandlung mit einer wässrigen Lösung eines organischen Lösungsmittels sowie mit einer anorganischen Base zur entsprechenden Benzoesäure hydrolysiert,
m) zwecks Herstellung derjenigen Verbindungen der Formel I, in denen $R^2$ $C_{1-6}$-Alkyl, $C_{2-4}$-Alkenyl, $C_{2-4}$-Alkinyl oder $C_{2-6}$-Alkoxyalkyl bedeutet, eine Benzoesäure der allgemeinen Formel

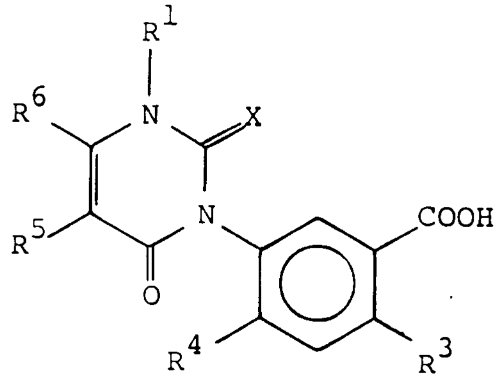

Ig

worin $R^1$, $R^3$, $R^4$, $R^5$, $R^6$ und X die oben angegebenen Bedeutungen besitzen,

oder ein reaktionsfähiges Derivat davon entsprechend verestert, wobei, im Falle der Verwendung eines Salzes der Benzoesäure Ig als reaktionsfähiges Derivat, dieses mit einem $C_{1-6}$-Alkyl-, $C_{2-4}$-Alkenyl-, $C_{2-4}$-Alkinyl- oder $C_{2-6}$-Alkoxyalkyl-chlorid, -bromid, -jodid, -sulfat, -mesylat oder -tosylat umgesetzt wird, und im Falle der Verwendung eines Säurehalogenids oder des entsprechenden O-Acyl-1,3-dicyclohexylisoharnstoffes, N-Acylimidazols oder Säureanhydrids der Benzoesäure Ig als reaktionsfähiges Derivat, dieses mit einem $C_{1-6}$-Alkonol, $C_{2-4}$-Alkenol, $C_{2-4}$-Alkinol oder $C_{2-6}$-Alkoxyalkanol umgesetzt wird, oder

n) zwecks Herstellung derjenigen Verbindungen der Formel I, in denen $R^2$ $C_{2-6}$-Alkyl, $C_{2-4}$-Alkenyl, $C_{2-4}$-Alkinyl oder $C_{2-6}$-Alkoxyalkyl bedeutet, einen Benzoesäureester der oben angegebenen Formel If einer Umesterungsreaktion mit einem Alkanol, Alkenol bzw. Alkinol der allgemeinen Formel

$R^{2''}OH$ VII

worin $R^{2''}$ $C_{2-6}$-Alkyl, $C_{2-4}$-Alkenyl, $C_{2-4}$-Alkinyl oder $C_{2-6}$-Alkoxyalkyl bedeutet,
unterwirft, wobei das Reagenz der Formel VII höher siedend ist als das Alkanol, Alkenol bzw. Alkinol $R^{2'}OH$,
und gewünschtenfalls eine so erhaltene Verbindung der Formel I, in der $R^1$ und/oder $R^2$ Wasserstoff bedeutet, in ein Salz überführt.

**22.** Verfahren nach Anspruch 21, dadurch gekennzeichnet, dass man eine der Varianten a), b), d), e), g), h), i), j), k), l), m) und n) und gegebenenfalls die fakultative Ueberführung in ein Salz durchführt.

**23.** Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 2 bis 7, 9 bis 11 und 13 bzw. eines Mittels gemäss einem der Ansprüche 18 und 19 behandelt.

**24.** Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 8, 12 und 14 bzw. eines Mittels gemäss Anspruch 20 behandelt.

**25.** Verwendung einer Verbindung gemäss einem der Ansprüche 2 bis 7, 9 bis 11 und 13 bzw. eines Mittels gemäss einem der Ansprüche 18 und 19 zur Bekämpfung von Unkräutern.

**26.** Verwendung einer Verbindung gemäss einem der Ansprüche 8, 12 und 14 bzw. eines Mittels gemäss Anspruch 20 zur Bekämpfung von Unkräutern.

**Patentansprüche für folgenden Vertragsstaat : AT**

**1.** Unkrautbekämpfungsmittel, dadurch gekennzeichnet, dass es eine wirksame Menge mindestens einer Verbindung der allgemeinen Formel

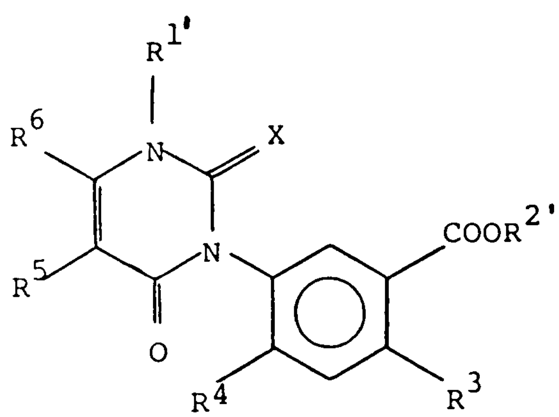

I'

worin

| | |
|---|---|
| $R^{1'}$ | $C_{1-4}$-Alkyl, $C_{2-4}$-Alkenyl, $C_{2-4}$-Alkinyl, $C_{2-6}$-Alkoxyalkyl, Formyl, $C_{2-6}$-Alkanoyl oder $C_{2-6}$-Alkoxycarbonyl, |
| $R^{2'}$ | $C_{1-6}$-Alkyl, $C_{2-4}$-Alkenyl, $C_{2-4}$-Alkinyl oder $C_{2-6}$-Alkoxyalkyl, |
| $R^3$ | Halogen oder Nitro, |
| $R^4$ | Wasserstoff oder Halogen, |
| $R^5$ | Wasserstoff, Halogen, $C_{1-4}$-Alkyl, Chlormethyl, Brommethyl, Hydroxymethyl, ($C_{1-5}$-Alkoxy)methyl, ($C_{1-5}$-Alkylthio)methyl, Cyano, Nitro oder Thiocyanato, |
| $R^6$ | Wasserstoff, $C_{1-4}$-Alkyl oder $C_{1-4}$-Fluoralkyl, oder |
| $R^5$ und $R^6$ | zusammen Tri- oder Tetramethylen, in welchem ein Methylen durch Sauerstoff oder Schwefel ersetzt sein kann, und welches gegebenenfalls mit $C_{1-3}$-Alkyl substituiert ist, und |
| X | Sauerstoff oder Schwefel bedeuten, mit den Massgaben, dass (i) falls $R^5$ für Fluor steht, $R^6$ ausschliesslich $C_{1-4}$-Alkyl oder $C_{1-4}$-Fluoralkyl bedeutet, und (ii) falls $R^5$ für Cyano steht, $R^6$ ausschliesslich Wasserstoff oder $C_{1-4}$-Alkyl und X ausschliesslich Sauerstoff bedeuten, |

sowie Formulierungshilfsstoffe enthält.

**2.** Unkrautbekämpfungsmittel nach Anspruch 1, worin $R^{1'}$ der Formel I' Methyl bedeutet.

**3.** Unkrautbekämpfungsmittel nach Anspruch 1 oder 2, worin $R^{2'}$ der Formel I' $C_{1-6}$-Alkyl oder $C_{2-6}$-Alkoxyalkyl bedeutet.

**4.** Unkrautbekämpfungsmittel nach einem der Ansprüche 1 bis 3, worin $R^3$ der Formel I' Chlor oder Brom bedeutet.

**5.** Unkrautbekämpfungsmittel nach einem der Ansprüche 1 bis 4, worin $R^4$ der Formel I' Fluor bedeutet.

**6.** Unkrautbekämpfungsmittel nach einem der Ansprüche 1 bis 5, worin $R^5$ der Formel I' Wasserstoff, Chlor, Brom, Methyl oder Aethyl bedeutet.

**7.** Unkrautbekämpfungsmittel nach einem der Ansprüche 1 bis 6, worin $R^5$ der Formel I' Fluor bedeutet.

**8.** Unkrautbekämpfungsmittel nach einem der Ansprüche 1 bis 6, worin $R^6$ der Formel I' Methyl, Aethyl oder Trifluormethyl bedeutet.

**9.** Unkrautbekämpfungsmittel nach einem der Ansprüche 1 bis 5, worin zusammen $R^5$ und $R^6$ der Formel I' Tri- oder Tetramethylen bedeuten.

**10.** Unkrautbekämpfungsmittel nach Anspruch 1, dadurch gekennzeichnet, dass es eine wirksame Menge mindestens einer aus der Gruppe

2-Chlor-4-fluor-5-(1,2,4,5,6,7-hexahydro-1-methyl-2,4-dioxo-3H-cyclopenta[d]pyrimidin-3-yl)-benzoesäure-isopropylester,

2-Chlor-4-fluor-5-[1,4,5,6,7,8-hexahydro-1-methyl-2,4-dioxo-3(2H)-chinazolinyl]-benzoesäure-isopropylester,

2-Chlor-4-fluor-5-[3,6-dihydro-3,4-dimethyl-2,6-dioxo-1(2H)-pyrimidinyl]-benzoesäure-isopropylester,

2-Chlor-4-fluor-5-[5-brom-3,6-dihydro-3,4-dimethyl-2,6-dioxo-1(2H)-pyrimidinyl]-benzoesäure-isopropylester,

2-Chlor-4-fluor-5-[3,6-dihydro-3,4-dimethyl-5-jod-2,6-dioxo-1(2H)-pyrimidinyl]-benzoesäure-isopropylester,

2-Chlor-4-fluor-5-[3,6-dihydro-3,4-dimethyl-5-hydroxy-methyl-2,6-dioxo-1(2H)-pyrimidinyl]-benzoesäure-isopropylester,

2-Chlor-4-fluor-5-[4-äthyl-3,6-dihydro-3-methyl-2,6-dioxo-1(2H)-pyrimidinyl]-benzoesäure-isopropylester,

2-Chlor-4-fluor-5-[3,6-dihydro-3-methyl-4-trifluormethyl-2,6-dioxo-1(2H)-pyrimidinyl]-benzoesäure-isopropylester,

2-Brom-4-fluor-5-(1,2,4,5,6,7-hexahydro-1-methyl-2,4-dioxo-3H-cyclopenta[d]pyrimidin-3-yl)-benzoesäure-isopropylester,

2-Chlor-4-fluor-5-[3,6-dihydro-3,4-dimethyl-5-nitro-2,6-dioxo-1(2H)-pyrimidinyl]-benzoesäure-isopropylester,
2-Chlor-4-fluor-5-(1,2,4,5,6,7-hexahydro-1-methyl-2,4-dioxo-3H-cyclopenta[d]pyrimidin-3-yl)-benzoesäure-(2-methoxy-1-methyläthyl)ester und
2-Chlor-4-fluor-5-(1,2,4,5,6,7-hexahydro-1-methyl-2,4-dioxo-3H-cyclopenta[d]pyrimidin-3-yl)-benzoesäure-tert. butylester
ausgewählten Verbindung sowie Formulierungshilfsstoffe enthält.

**11.** Unkrautbekämpfungsmittel nach Anspruch 1, dadurch gekennzeichnet, dass es eine wirksame Menge mindestens einer aus der Gruppe
2-Chlor-4-fluor-5-[3,6-dihydro-3,4-dimethyl-5- fluor-2,6-dioxo-1(2H)-pyrimidinyl]-benzoesäure-isopropylester,
2-Chlor-4-fluor-5-[3,6-dihydro-3-methyl-4-propyl-2,6-dioxo-1(2H)-pyrimidinyl]-benzoesäure-isopropylester und
2-Chlor-4-fluor-5-[3,6-dihydro-5-fluor-3-methyl-4-trifluormethyl-2,6-dioxo-1(2H)-pyrimidinyl]-benzoesäure-isopropylester
ausgewählten Verbindung sowie Formulierungshilfsstoffe enthält.

**12.** Unkrautbekämpfungsmittel nach Anspruch 1, dadurch gekennzeichnet, dass es eine wirksame Menge mindestens einer aus der Gruppe
2-Chlor-5-(1,2,4,5,6,7-hexahydro-1-methyl-2,4-dioxo-3H-cyclopenta[d]pyrimidin-3-yl)-benzoesäure-äthylester,
2-Chlor-5-[1,4,5,6,7,8-hexahydro-1-methyl-2,4-dioxo-3(2H)-chinazolinyl]-benzoesäure-äthylester,
2-Chlor-5-[3,6-dihydro-3,4-dimethyl-2,6-dioxo-1(2H)-pyrimidinyl]-benzoesäure-äthylester,
2-Chlor-5-[3,6-dihydro-3,4,5-trimethyl-2,6-dioxo-1(2H)-pyrimidinyl]-benzoesäure-äthylester,
2-Chlor-4-fluor-5-[3,6-dihydro-3,4,5-trimethyl-2,6-dioxo-1(2H)-pyrimidinyl]-benzoesäure-isopropylester,
2-Chlor-4-fluor-5-{1,2,5,7-tetrahydro-1-methyl-2,4-dioxo-thieno[3,4-d]pyrimidin-3(4H)-yl}-benzoesäure-isopropylester,
2-Chlor-4-fluor-5-[4-äthyl-3,6-dihydro-3,5-dimethyl-2,6-dioxo-1(2H)-pyrimidinyl]-benzoesäure-isopropylester,
2-Chlor-4-fluor-5-[5-cyano-3,6-dihydro-3,4-dimethyl-2,6-dioxo-1(2H)-pyrimidinyl]-benzoesäure-isopropylester,
2,4-Dichlor-5-(1,2,4,5,6,7-hexahydro-1-methyl-2,4-dioxo-3H-cyclopenta[d]pyrimidin-3-yl)-benzoesäure-isopropylester,
2-Brom-4-chlor-5-(1,2,4,5,6,7-hexahydro-1-methyl-2,4-dioxo-3H-cyclopenta[d]pyrimidin-3-yl)-benzoesäure-isopropylester,
2-Chlor-4-fluor-5-(1,2,4,5,6,7-hexahydro-1-acetyl-2,4-dioxo-3H-cyclopenta[d]pyrimidin-3-yl)-benzoesäure-isopropylester,
2-Chlor-4-fluor-5-{1,2,4,5,6,7-hexahydro-1-methoxycarbonyl-2,4-dioxo-3H-cyclopenta[d]pyrimidin-3-yl}-benzoesäure-isopropylester,
2-Chlor-4-fluor-5-[5-chlor-3,6-dihydro-3,4-dimethyl-2,6-dioxo-1(2H)-pyrimidinyl]-benzoesäure-isopropylester,
2-Chlor-5-(1,2.4,5,6,7-hexahydro-1-methyl-2,4-dioxo-3H-cyclopenta[d]pyrimidin-3-yl)-benzoesäure-tert.butylester,
2-Chlor-5-[1,4,5,6,7,8-hexahydro-1-methyl-2,4-dioxo-3(2H)-chinazolinyl]-benzoesäure-isopropylester,
2-Chlor-4-fluor-5-(1,2,4,5,6,7-hexahydro-1-methyl-2,4-dioxo-3H-cyclopenta[d]pyrimidin-3-yl)-benzoesäure-2-propenylester,
2-Chlor-4-fluor-5-(1,2,4,5,6,7-hexahydro-1-methyl-2,4-dioxo-3H-cyclopenta[d]pyrimidin-3-yl)-benzoesäure-2-propinylester und
2-Chlor-4-fluor-5-(1,2,4,5,6,7-hexahydro-1-methyl-2,4-dioxo-3H-cyclopenta[d]pyrimidin-3-yl)-benzoesäure-methylester,
ausgewählten Verbindung sowie Formulierungshilfsstoffe enthält.

**13.** Unkrautbekämpfungsmittel nach Anspruch 1, dadurch gekennzeichnet, dass es eine wirksame Menge von 2-Chlor-4--fluor-5 -[3,6-dihydro-3,4-dimethyl-5-methoxymethyl-2,6--dioxo-1(2H) -pyrimidinyl]-benzoesäure-isopropylester sowie Formulierungshilfsstoffe enthält.

**14.** Verfahren zur Herstellung von Verbindungen der allgemeinen Formel

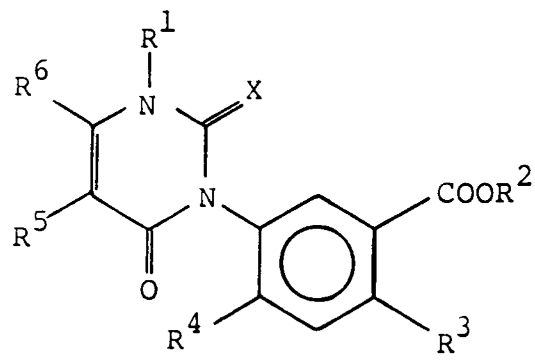

I

worin

| | |
|---|---|
| $R^1$ | Wasserstoff, $C_{1-4}$-Alkyl, $C_{2-4}$-Alkenyl, $C_{2-4}$-Alkinyl, $C_{2-6}$-Alkoxyalkyl, Formyl, $C_{2-6}$--Alkanoyl oder $C_{2-6}$-Alkoxycarbonyl bedeutet, |
| und $R^2$, $R^3$, $R^4$, $R^5$, $R^6$ und X | die in Anspruch 1 angegebenen Bedeutungen besitzen, |

und von Salzen derjenigen Verbindungen der Formel I, in denen $R^1$ und/oder $R^2$ Wasserstoff bedeutet, dadurch gekennzeichnet, dass man

a) zwecks Herstellung derjenigen Verbindungen der Formel I, in denen $R^1$ Wasserstoff und $R^2$ $C_{1-6}$-Alkyl, $C_{2-4}$--Alkenyl, $C_{2-4}$-Alkinyl oder $C_{2-6}$-Alkoxyalkyl bedeuten und $R^5$ verschieden von Chlor, Brom, Jod, Chlormethyl, Brommethyl, Hydroxymethyl, ($C_{1-5}$-Alkoxy)methyl, ($C_{1-5}$--Alkylthio)methyl, Cyano, Nitro oder Thiocyanato ist, sowie von Metallsalzen jener Verbindungen der Formel I, in denen $R^1$ Wasserstoff bedeutet, eine Verbindung der allgemeinen Formel

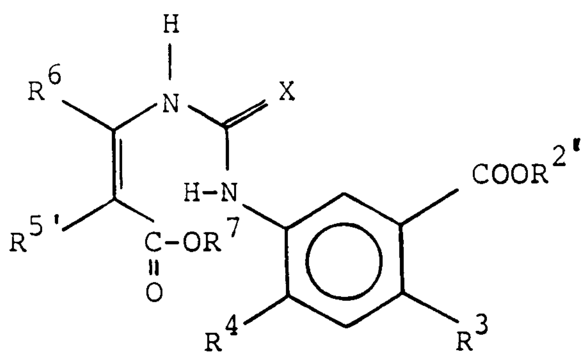

II

worin

| | |
|---|---|
| $R^{2'}$ | $C_{1-6}$-Alkyl, $C_{2-4}$-Alkenyl, $C_{2-4}$-Alkinyl oder $C_{2-6}$-Alkoxyalkyl bedeutet, |
| $R^3$, $R^4$, $R^6$ und X | die in Anspruch 1 angegebenen Bedeutungen besitzen, |
| $R^{5'}$ | Wasserstoff, Fluor, $C_{1-4}$-Alkyl oder zusammen mit $R^6$ gegebenenfalls modifiziertes Tri- oder Tetramethylen, wie dies oben näher definiert ist, bedeutet, und |
| $R^7$ | nieder Alkyl bedeutet, |

einer basekatalysierten Cyclisierung durch Behandlung mit einer Base unterwirft und gewünschtenfalls eine allfällig erhaltene Metallsalzform des Uracilderivats durch Behandlung mit einer Säure in die entsprechende saure Form ($R^1$ = Wasserstoff) überführt,

b) zwecks Herstellung derjenigen Verbindungen der Formel I, in denen $R^1$ $C_{1-4}$-Alkyl, $C_{2-4}$-Alkenyl, $C_{2-4}$-Alkinyl, $C_{2-6}$-Alkoxyalkyl, Formyl, $C_{2-6}$-Alkanoyl oder $C_{2-6}$-Alkoxycarbonyl bedeutet, ein Uracilderivat der allgemeinen Formel

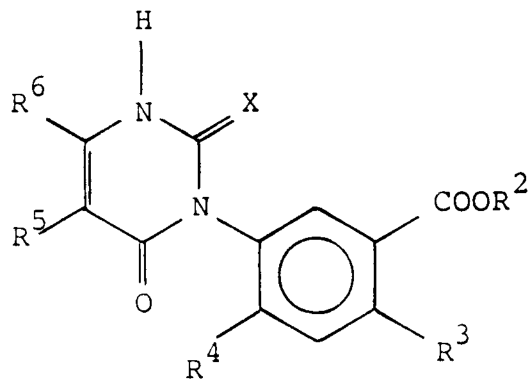

Ia

worin $R^2$, $R^3$, $R^4$, $R^5$, $R^6$ und X die in Anspruch 1 angegebenen Bedeutungen besitzen, zur Alkylierung bzw. Acylierung mit einem entsprechenden eine $C_{1-4}$-Alkyl-, $C_{2-4}$-Alkenyl-, $C_{2-4}$-Alkinyl-, $C_{2-6}$-Alkoxyalkyl-, Formyl-, $C_{2-6}$-Alkanoyl- oder $C_{2-6}$--Alkoxycarbonylgruppe enthaltenden Alkylierungs- bzw. Acylierungsmittel behandelt,

c) zwecks Herstellung derjenigen Verbindungen der Formel I, in denen $R^1$ $C_{1-4}$-Alkyl, $C_{2-4}$-Alkenyl, $C_{2-4}$-Alkinyl oder $C_{2-6}$-Alkoxyalkyl bedeutet, $R^5$ verschieden von Chlor, Brom, Jod, Chlormethyl, Brommethyl, Hydroxymethyl, ($C_{1-5}$-Alkoxy)methyl, ($C_{1-5}$-Alkylthio)methyl, Cyano, Nitro oder Thiocyanato ist, und X Schwefel bedeutet, eine Verbindung der allgemeinen Formel

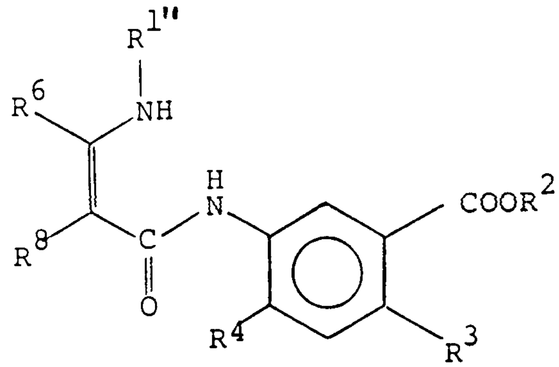

III

worin

| | |
|---|---|
| $R^{1''}$ | $C_{1-4}$-Alkyl, $C_{2-4}$-Alkenyl, $C_{2-4}$-Alkinyl oder $C_{2-6}$-Alkoxyalkyl bedeutet, |
| $R^2$, $R^3$, $R^4$ und $R^6$ | die in Anspruch 1 angegebenen Bedeutungen besitzen und |
| $R^8$ | Fluor, $C_{1-4}$-Alkyl, ($C_{1-4}$-Alkoxy)carbonyl oder zusammen mit $R^6$ wie in Anspruch definiertes Tri- oder Tetramethylen bedeutet, wobei, im Falle von $R^8$ = Fluor, $R^6$ $C_{1-4}$-Alkyl oder $C_{1-4}$-Fluoralkyl ist, |

mit N,N'-Thiocarbonyldiimidazolid oder Thiophosgen umsetzt und, falls $R^8$ ($C_{1-4}$-Alkoxy)carbonyl bedeutet, das so hergestellte 5-Alkoxycarbonyl-2-thiouracil der allgemeinen Formel

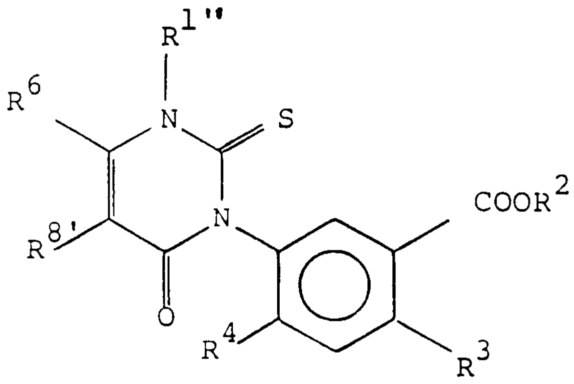

IV

worin

| | |
|---|---|
| $R^{1''}$, $R^2$, $R^3$, $R^4$ und $R^6$ | die oben bzw. in Anspruch 1 angegebenen Bedeutungen besitzen und |
| $R^{8'}$ | ($C_{1-4}$-Alkoxy)carbonyl bedeutet, |

zur Hydrolyse und Decarboxylierung in Gegenwart von wässriger Salzsäure oder Trifluoressigsäure aufwärmt,

d) zwecks Herstellung derjenigen Verbindungen der Formel I, in denen $R^5$ Chlor, Brom oder Jod bedeutet, ein Uracilderivat der allgemeinen Formel

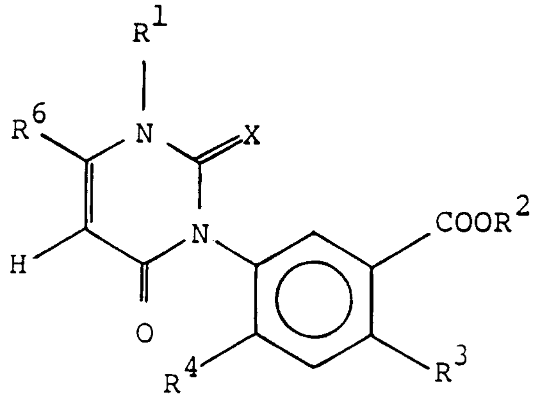

Ib

worin $R^1$, $R^2$, $R^3$, $R^4$, $R^6$ und X die oben bzw. in Anspruch 1 angegebenen Bedeutungen besitzen, zur Chlorierung mit elementarem Chlor oder Sulfurylchlorid, zur Bromierung mit elementarem Brom oder Sulfurylbromid bzw. zur Jodierung mit elementarem Jod behandelt,

e) zwecks Herstellung derjenigen Verbindungen der Formel I, in denen $R^5$ Chlor- oder Brommethyl bedeutet, (i) ein Uracilderivat der oben angegebenen Formel Ib mit Chlor- bzw. Brommethoxymethan behandelt oder (ii) ein 5-Hydroxymethyluracil der allgemeinen Formel

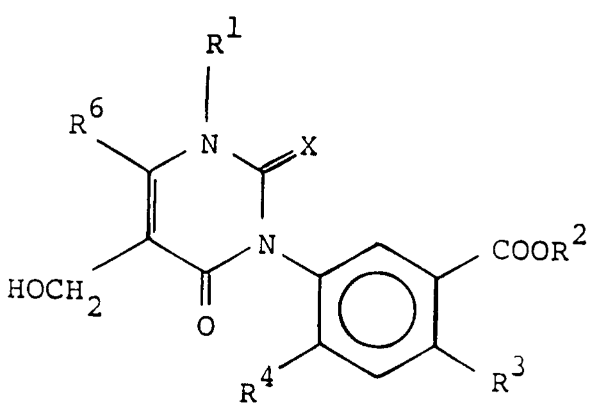

Ic

worin $R^2$, $R^3$, $R^4$, $R^6$ und X die in Anspruch 1 angegebenen Bedeutungen besitzen, mit Thionylchlorid bzw. -bromid behandelt oder (iii) ein 5-Methyluracil der allgemeinen Formel

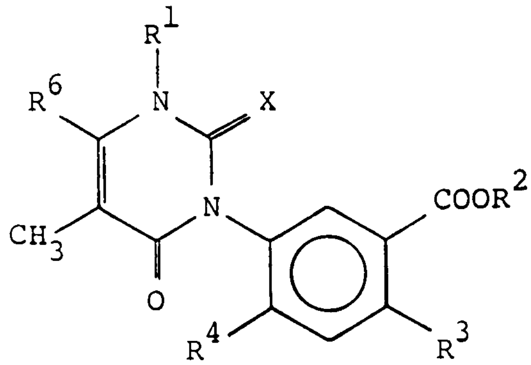

Id

worin $R^1$, $R^2$, $R^3$, $R^4$, $R^6$ und X die oben bzw. in Anspruch 1 angegebenen Bedeutungen besitzen, mit N-Chlorsuccinimid bzw. N-Bromsuccinimid behandelt,

f) zwecks Herstellung derjenigen Verbindungen der Formel I, in denen $R^5$ Hydroxymethyl bedeutet, ein 5-Halogenmethyluracil der allgemeinen Formel

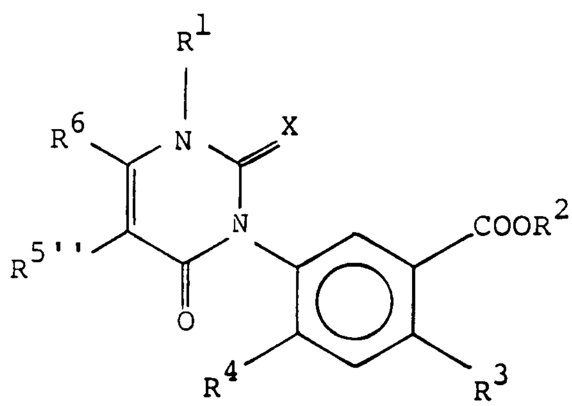

Ie

worin $R^1$, $R^2$, $R^3$, $R^4$, $R^6$ und X die oben bzw. in Anspruch 1 angegebenen Bedeutungen besitzen und $R^{5''}$ Chlor-, Brom- oder Jodmethyl bedeutet,

zur Hydrolyse mit einer wässrigen Lösung einer anorganischen Base behandelt,

g) zwecks Herstellung derjenigen Verbindungen der Formel I, in denen $R^5$ ($C_{1-5}$-Alkoxy)methyl oder ($C_{1-5}$-Alkylthio)methyl bedeutet, ein 5-Halogenmethyluracil der oben angegebenen Formel Ie, in der $R^{5''}$ Chlor- oder Brommethyl bedeutet, mit einem Alkalimetallalkoholat oder -thioalkoholat der allgemeinen Formel

$R^9M$ V

worin

$R^9$ $C_{1-5}$-Alkoxy oder $C_{1-5}$-Alkylthio

und M ein Alkalimetall bedeuten,

oder mit einem $C_{1-5}$-Alkanol oder $C_{1-5}$-Alkylmercaptan behandelt,

h) zwecks Herstellung derjenigen Verbindungen der Formel I, in denen $R^5$ ($C_{1-5}$-Alkylthio)methyl bedeutet, ein 5-Hydroxymethyluracil der oben angegebenen Formel Ic mit einem $C_{1-5}$-Alkylmercaptan behandelt,

i) zwecks Herstellung derjenigen Verbindungen der Formel I, in denen $R^1$ Wasserstoff, $R^5$ Cyano, $R^6$ Wasserstoff oder $C_{1-4}$-Alkyl und X Sauerstoff bedeuten, eine Verbindung der allgemeinen Formel

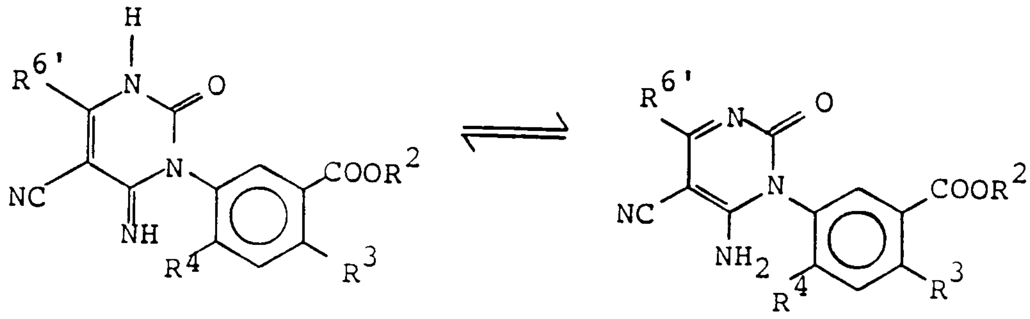

VI

worin $R^2$, $R^3$ und $R^4$ die in Anspruch 1 angegebenen Bedeutungen besitzen und $R^{6'}$ Wasserstoff oder $C_{1-4}$-Alkyl bedeutet,
zur säurekatalysierten Hydrolyse mit einer wässrigen Lösung einer Mineralsäure behandelt,
j) zwecks Herstellung derjenigen Verbindungen der Formel I, in denen $R^5$ Nitro bedeutet, ein Uracilderivat der oben angegebenen Formel Ib mit Salpetersäure behandelt,
k) zwecks Herstellung derjenigen Verbindungen der Formel I, in denen $R^5$ Thiocyanato bedeutet, ein Uracilderivat der oben angegebenen Formel Ib mit Thiocyanogen behandelt,
l) zwecks Herstellung derjenigen Verbindungen der Formel I, in denen $R^2$ Wasserstoff bedeutet, einen Benzoesäureester der allgemeinen Formel

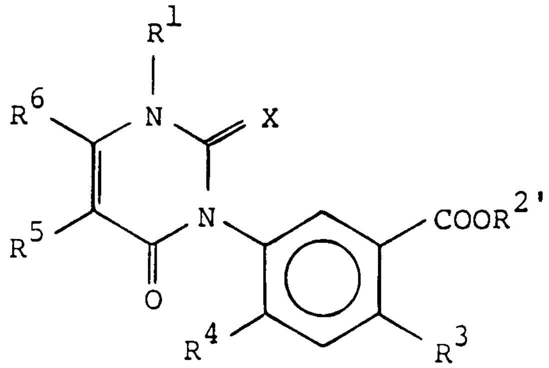

If

worin $R^1$, $R^2$, $R^3$, $R^4$, $R^5$, $R^6$ und X die oben bzw. in Anspruch 1 angegebenen Bedeutungen besitzen,
durch Behandlung mit einer wässrigen Lösung eines organischen Lösungsmittels sowie mit einer anorganischen Base zur entsprechenden Benzoesäure hydrolysiert,
m) zwecks Herstellung derjenigen Verbindungen der Formel I, in denen $R^2$ $C_{1-6}$-Alkyl, $C_{2-4}$-Alkenyl, $C_{2-4}$-Alkinyl oder $C_{2-6}$-Alkoxyalkyl bedeutet, eine Benzoesäure der allgemeinen Formel

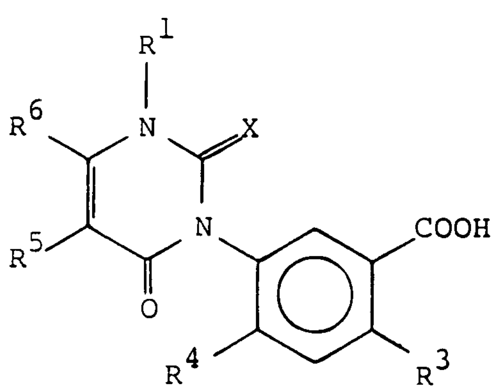

Ig

worin $R^1$, $R^3$, $R^4$, $R^5$, $R^6$ und X die oben bzw. in Anspruch 1 angegebenen Bedeutungen besitzen, oder ein reaktionsfähiges Derivat davon entsprechend verestert, wobei, im Falle der Verwendung eines Salzes der Benzoesäure Ig als reaktionsfähiges Derivat, dieses mit einem $C_{1-6}$-Alkyl-, $C_{2-4}$-Alkenyl-, $C_{2-4}$-Alkinyl- oder $C_{2-6}$-Alkoxyalkyl-chlorid, -bromid, -jodid, -sulfat, -mesylat oder -tosylat umgesetzt O Acyl-1,3-dicyclohexylisoharnstoffes, N-Acylimidazols oder Säureanhydrids der Benzoesäure Ig als reaktionsfähiges Derivat, dieses mit einem $C_{1-6}$-Alkanol, $C_{2-4}$-Alkenol, $C_{2-4}$-Alkinol oder $C_{2-6}$-Alkoxyalkanol umgesetzt wird, oder

n) zwecks Herstellung derjenigen Verbindungen der Formel I, in denen $R^2$ $C_{2-6}$-Alkyl, $C_{2-4}$-Alkenyl, $C_{2-4}$-Alkinyl oder $C_{2-6}$-Alkoxyalkyl bedeutet, einen Benzoesäureester der oben angegebenen Formel If einer Umesterungsreaktion mit einem Alkanol, Alkenol bzw. Alkinol der allgemeinen Formel

R2"OH VII

worin $R^{2''}$ $C_{2-6}$-Alkyl, $C_{2-4}$-Alkenyl, $C_{2-4}$-Alkinyl oder $C_{2-6}$-Alkoxyalkyl bedeutet,
unterwirft, wobei das Reagenz der Formel VII höher siedend ist als das Alkanol, Alkenol bzw. Alkinol $R^{2'}$OH,
und gewünschtenfalls eine so erhaltene Verbindung der Formel I, in der $R^1$ und/oder $R^2$ Wasserstoff bedeutet, in ein Salz überführt.

**15.** Verfahren nach Anspruch 15, dadurch gekennzeichnet, dass man eine der Varianten a), b), d), e), g), h), i), j), k), l), m) und n) und gegebenenfalls die fakultative Ueberführung in ein Salz durchführt.

**16.** Verfahren zur Herstellung eines Unkrautbekämpfungsmittels, dadurch gekennzeichnet, dass man mindestens eine der in Anspruch 1 genannten Verbindungen mit Formulierungshilfsstoffen vermischt.

**17.** Verfahren zur Bekämpfung von Unkräutern, dadurch gekennzeichnet, dass man das gegen Unkräuter zu schützende Gut und/oder die Unkräuter mit einer wirksamen Menge eines Mittels gemäss einem der Ansprüche 1 bis 6, 8 bis 10 und 12 behandelt.

**18.** Verfahren zur Bekämpfung von Unkräutern, dadurch gekennzeichnet, dass man das gegen Unkräuter zu schützende Gut und/oder die Unkräuter mit einer wirksamen Menge eines Mittels gemäss einem der Ansprüche 7, 11 und 13 behandelt.

**19.** Verwendung eines Mittels gemäss einem der Ansprüche 1 bis 6, 8 bis 10 und 12 zur Bekämpfung von Unkräutern.

**20.** Verwendung eines Mittels gemäss einem der Ansprüche 7, 11 und 13 zur Bekämpfung von Unkräutern.

## Claims

### Claims for the following Contracting States : BE, CH, DE, FR, GB, IT, LI, LU, NL, SE

**1.** Compounds of the general formula

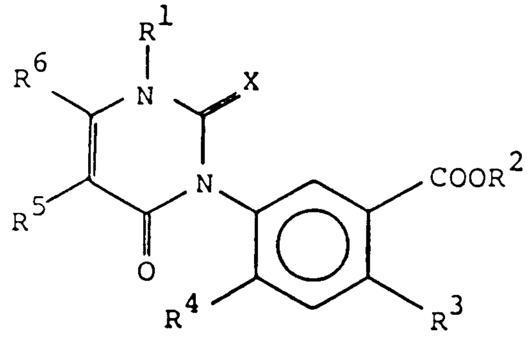

I

wherein
$R^1$ signifies hydrogen, $C_{1-4}$alkyl, $C_{2-4}$alkenyl, $C_{2-4}$alkynyl, $C_{2-6}$alkoxyalkyl, formyl,

$C_{2-6}$alkanoyl or $C_{2-6}$alkoxycarbonyl,

$R^2$ signifies hydrogen, $C_{1-6}$alkyl, $C_{2-4}$alkenyl, $C_{2-4}$alkynyl or $C_{2-6}$alkoxyalkyl,

$R^3$ signifies halogen or nitro,

$R^4$ signifies hydrogen or halogen,

$R^5$ signifies hydrogen, halogen, $C_{1-4}$alkyl, chloromethyl, bromomethyl, hydroxymethyl, ($C_{1-5}$alkoxy)methyl, ($C_{1-5}$alkylthio)methyl, cyano, nitro or thiocyanato,

$R^6$ signifies hydrogen, $C_{1-4}$alkyl or $C_{1-4}$fluoroalkyl,

or

$R^5$ and $R^6$ together signify tri- or tetramethylene in which one methylene can be replaced by oxygen or sulphur and which is optionally substituted with $C_{1-3}$alkyl,

and

X signifies oxygen or sulphur,

with the provisos that (i) $R^6$ signifies exclusively $C_{1-4}$alkyl or $C_{1-4}$fluoroalkyl when $R^5$ stands for fluorine and (ii) $R^6$ signifies exclusively hydrogen or $C_{1-4}$alkyl and X signifies exclusively oxygen when $R^5$ stands for cyano,

as well as salts of those compounds of the formula I in which $R^1$ and/or $R^2$ signifies hydrogen.

**2.** Compounds of the general formula

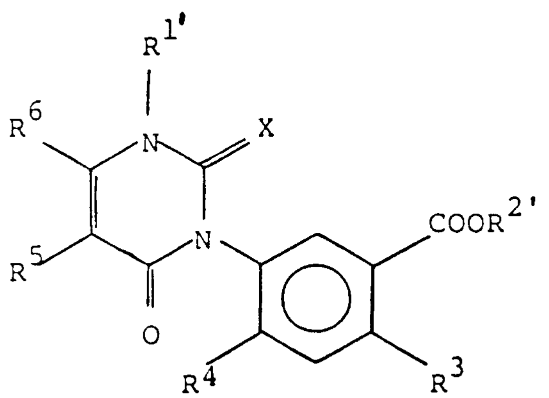

I'

wherein

$R^{1'}$ signifies $C_{1-4}$alkyl, $C_{2-4}$alkenyl, $C_{2-4}$alkynyl, $C_{2-6}$alkoxyalkyl, formyl, $C_{2-6}$alkanoyl or $C_{2-6}$alkoxycarbonyl,

$R^{2'}$ signifies $C_{1-6}$alkyl, $C_{2-4}$alkenyl, $C_{2-4}$alkynyl or $C_{2-6}$alkoxyalkyl,

$R^3$ signifies halogen or nitro,

$R^4$ signifies hydrogen or halogen,

$R^5$ signifies hydrogen, halogen, $C_{1-4}$alkyl, chloromethyl, bromomethyl, hydroxymethyl, ($C_{1-5}$alkoxy)methyl, ($C_{1-5}$alkylthio)methyl, cyano, nitro or thiocyanato,

$R^6$ signifies hydrogen, $C_{1-4}$alkyl or $C_{1-4}$fluoroalkyl,

or

$R^5$ and $R^6$ together signify tri- or tetramethylene in which one methylene can be replaced by oxygen or sulphur and which is optionally substituted with $C_{1-3}$alkyl,

and

X signifies oxygen or sulphur,

with the provisos that (i) $R^6$ signifies exclusively $C_{1-4}$alkyl or $C_{1-4}$fluoroalkyl when $R^5$ stands for fluorine and (ii) $R^6$ signifies exclusively hydrogen or $C_{1-4}$alkyl and X signifies exclusively oxygen when $R^5$ stands for cyano.

**3.** Compounds according to claim 1 or 2, wherein $R^1$ or $R^{1'}$ signifies methyl.

**4.** Compounds according to any one of claims 1 to 3, wherein $R^2$ or $R^{2'}$ signifies $C_{1-6}$alkyl or $C_{2-6}$alkoxyalkyl.

**5.** Compounds according to any one of claims 1 to 4, wherein $R^3$ signifies chlorine or bromine.

6. Compounds according to any one of claims 1 to 5, wherein $R^4$ signifies fluorine.

7. Compounds according to any one of claims 1 to 6, wherein $R^5$ signifies hydrogen, chlorine, bromine, methyl or ethyl.

8. Compounds according to any one of claims 1 to 6, wherein $R^5$ signifies fluorine.

9. Compounds according to any one of claims 1 to 7, wherein $R^6$ signifies methyl, ethyl or trifluoromethyl.

10. Compounds according to any one of claims 1 to 6, wherein $R^5$ and $R^6$ together signify tri- or tetramethylene.

11. A compound according to claim 1 or 2, selected from:
isopropyl 2-chloro-4-fluoro-5-(1,2,4,5,6,7-hexahydro-1-methyl-2,4-dioxo-3H-cyclopenta[d]pyrimidin-3-yl)benzoate,
isopropyl 2-chloro-4-fluoro-5-[1,4,5,6,7,8-hexahydro-1-methyl-2,4-dioxo-3(2H)-quinazolinyl]benzoate,
isopropyl 2-chloro-4-fluoro-5-[3,6-dihydro-3,4-dimethyl-2,6-dioxo-1(2H)-pyrimidinyl]benzoate,
isopropyl 2-chloro-4-fluoro-5-[5-bromo-3,6-dihydro-3,4-dimethyl-2,6-dioxo-1(2H)-pyrimidinyl]-benzoate,
isopropyl 2-chloro-4-fluoro-5-[3,6-dihydro-3,4-dimethyl-5-iodo-2,6-dioxo-1(2H)-pyrimidinyl]benzoate,
isopropyl 2-chloro-4-fluoro-5-[3,6-dihydro-3,4-dimethyl-5-hydroxymethyl-2,6-dioxo-1(2H)-pyrimidinyl]benzoate,
isopropyl 2-chloro-4-fluoro-5-[3,6-dihydro-4-ethyl-3-methyl-2,6-dioxo-1(2H)-pyrimidinyl]benzoate,
isopropyl 2-chloro-4-fluoro-5-[3,6-dihydro-3-methyl-4-trifluoromethyl-2,6-dioxo-1(2H)-pyrimidinyl]-benzoate,
isopropyl 2-bromo-4-fluoro-5-[1,2,4,5,6,7-hexahydro-1-methyl-2,4-dioxo-3H-cyclopenta[d]pyrimidin-3-yl)benzoate,
isopropyl 2-chloro-4-fluoro-5-[3,6-dihydro-3,4-dimethyl-5-nitro-2,6-dioxo-1(2H)-pyrimidinyl]benzoate,
2-methoxy-1-methylethyl 2-chloro-4-fluoro-5-(1,2,3,4,5,6,7-hexahydro-1-methyl-2,4-dioxo-3H-cyclopenta[d]pyrimidin-3-yl)benzoate and
tert-butyl 2-chloro-4-fluoro-5-(1,2,4,5,6,7-hexahydro-1-methyl-2,4-dioxo-3H-cyclopenta[d]pyrimidin-3-yl)benzoate.

12. A compound according to claim 1 or 2, selected from:
isopropyl 2-chloro-4-fluoro-5-[3,6-dihydro-3,4-dimethyl-5-fluoro-2,6-dioxo-1-(2H)-pyrimidinyl]-benzoate,
isopropyl 2-chloro-4-fluoro-5-[3,6-dihydro-3-methyl-4-propyl-2,6-dioxo-1(2H)-pyrimidinyl]benzoate and
isopropyl 2-chloro-4-fluoro-5-[3,6-dihydro-5-fluoro-3-methyl-4-trifluoromethyl-2,6-dioxo-1(2H)-pyrimidinyl]benzoate.

13. A compound according to claim 1 or 2, selected from:
ethyl 2-chloro-5-(1,2,4,5,6,7-hexahydro-2,4-dioxo-3H-cyclopenta[d]pyrimidin-3-yl)benzoate,
isopropyl 2-chloro-4-fluoro-5-(1,2,4,5,6,7-hexahydro-2,4-dioxo-3H-cyclopenta[d]pyrimidin-3-yl)-benzoate,
isopropyl 2-chloro-4-fluoro-5-[1,4,5,6,7,8-hexahydro-2,4-dioxo-3(2H)-quinazolinyl]benzoate,
isopropyl 2-chloro-4-fluoro-5-[3,6-dihydro-4-methyl-2,6-dioxo-1(2H)-pyrimidinyl]benzoate,
isopropyl 2-chloro-4-fluoro-5-[3,6-dihydro-5-methyl-2,6-dioxo-1(2H)-pyrimidinyl]benzoate,
isopropyl 2-bromo-4-fluoro-5-(1,2,4,5,6,7-hexahydro-2,4-dioxo-3H-cyclopenta[d]pyrimidin-3-yl)-benzoate,
isopropyl 2-chloro-4-fluoro-5-(1,2,5,7-tetrahydro-2,4-dioxothieno[3,4-d]pyrimidin-3(4H)-yl]benzoate,
isopropyl 2-chloro-4-fluoro-5-[3,6-dihydro-4-ethyl-5-methyl-2,6-dioxo-1(2H)-pyrimidinyl]benzoate,
ethyl 2-chloro-5-(1,2,4,5,6,7-hexahydro-1-methyl-2,4-dioxo-3H-cyclopenta[d]pyrimidin-3-yl)benzoate,
ethyl 2-chloro-5-[1,4,5,6,7,8-hexahydro-1-methyl-2,4-dioxo-3(2H)-quinazolinyl]benzoate,
ethyl 2-chloro-4-fluoro-5-[3,6-dihydro-4-ethyl-2,6-dioxo-1(2H)-pyrimidinyl]benzoate,
ethyl 2-chloro-5-[3,6-dihydro-3,4-dimethyl-2,6-dioxo-1(2H)-pyrimidinyl]benzoate,
ethyl 2-chloro-5-[3,6-dihydro-3,4,5-trimethyl-2,6-dioxo-1(2H)-pyrimidinyl]benzoate,
isopropyl 2-chloro-4-fluoro-5-[3,6-dihydro-3,4,5-trimethyl-2,6-dioxo-1(2H)-pyrimidinyl]benzoate,

isopropyl 2-chloro-4-fluoro-5-[1,2,5,7-tetrahydro-1-methyl-2,4-dioxothieno[3,4-d]pyrimidin-3(4H)-yl]-benzoate,
isopropyl 2-chloro-4-fluoro-5-[3,6-dihydro-3,5-dimethyl-4-ethyl-2,6-dioxo-1-(2H)-pyrimidinyl]-benzoate,
isopropyl 2-chloro-4-fluoro-5-[5-cyano-3,6-dihydro-3,4-dimethyl-2,6-dioxo-1-(2H)-pyrimidinyl]-benzoate,
isopropyl 2,4-dichloro-5-(1,2,4,5,6,7-hexahydro-1-methyl-2,4-dioxo-3H-cyclopenta[d]pyrimidin-3-yl)-benzoate,
isopropyl 2-bromo-4-chloro-5-(1,2,4,5,6,7-hexahydro-1-methyl-2,4-dioxo-3H-cyclopenta[d]pyrimidin-3-yl)benzoate,
isopropyl 2-chloro-4-fluoro-5-(1,2,4,5,6,7-hexahydro-1-acetyl-2,4-dioxo-3H-cyclopenta[d]pyrimidin-3-yl)benzoate,
isopropyl 2-chloro-4-fluoro-5-{1,2,4,5,6,7-hexahydro-1-methoxycarbonyl-2,4-dioxo-3H-cyclopenta[d]-pyrimidin-3-yl}benzoate,
isopropyl 2-chloro-4-fluoro-5-[5-chloro-3,6-dihydro-3,4-dimethyl-2,6-dioxo-1(2H)-pyrimidinyl]-benzoate,
isopropyl 2-chloro-4-fluoro-5-[5-bromo-3,6-dihydro-4-methyl-2,6-dioxo-1(2H)-pyrimidinyl]benzoate,
tert-butyl 2-chloro-5-(1,2,4,5,6,7-hexahydro-1-methyl-2,4-dioxo-3H-cyclopenta[d]pyrimidin-3-yl)-benzoate,
isopropyl 2-chloro-5-[1,4,5,6,7,8-hexahydro-1-methyl-2,4-dioxo-3(2H)-quinazolinyl]benzoate,
isopropyl 2-chloro-5-[1,4,5,6,7,8-hexahydro-2,4-dioxo-3(2H)-quinazolinyl]benzoate,
2-propenyl 2-chloro-4-fluoro-5-(1,2,4,5,6,7-hexahydro-1-methyl-2,4-dioxo-3H-cyclopenta[d]pyrimidin-3-yl)benzoate,
2-propynyl 2-chloro-4-fluoro-5-(1,2,4,5,6,7-hexahydro-1-methyl-2,4-dioxo-3H-cyclopenta[d]pyrimidin-3-yl)benzoate
and
methyl 2-chloro-4-fluoro-5-(1,2,4,5,6,7-hexahydro-1-methyl-2,4-dioxo-3H-cyclopenta[d]pyrimidin-3-yl)benzoate.

**14.** A compound according to claim 1 or 2, selected from:
isopropyl 2-chloro-4-fluoro-5-[3,6-dihydro-5-fluoro-4-methyl-2,6-dioxo-1(2H)-pyrimidinyl]benzoate,
isopropyl 2-chloro-4-fluoro-5-[3,6-dihydro-5-fluoro-4-fluoromethyl-2,6-dioxo-1(2H)-pyrimidinyl]-benzoate,
isopropyl 2-chloro-4-fluoro-5-[3,6-dihydro-5-fluoro-4-fluoromethyl-3-methyl-2,6-dioxo-1(2H)-pyrimidinyl]benzoate,
isopropyl 2-chloro-4-fluoro-5-[3,6-dihydro-4-propyl-2,6-dioxo-1(2H)-pyrimidinyl]benzoate,
isopropyl 2-chloro-4-fluoro-5-[5-chloro-3,6-dihydro-4-methyl-2,6-dioxo-1(2H)-pyrimidinyl]benzoate
and
isopropyl 2-chloro-4-fluoro-5-[3,6-dihydro-3,4-dimethyl-5-methoxymethyl-2,6-dioxo-1(2H)-pyrimidinyl]benzoate.

**15.** Compounds of the general formula

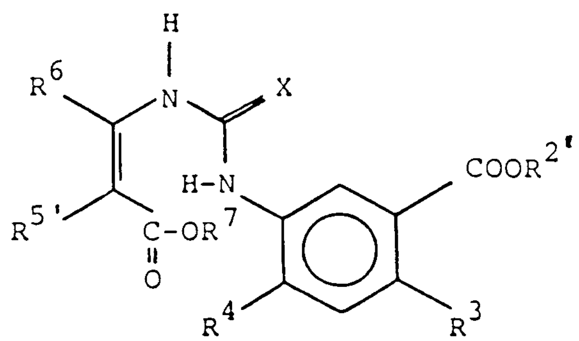

II

wherein

| | |
|---|---|
| $R^{2'}$ | signifies $C_{1-6}$alkyl, $C_{2-4}$alkenyl, $C_{2-4}$alkynyl or $C_{2-6}$alkoxyalkyl, |
| $R^3$ | signifies halogen or nitro, |

| | |
|---|---|
| $R^4$ | signifies hydrogen or halogen, |
| $R^{5'}$ | signifies hydrogen, fluorine or $C_{1-4}$alkyl, |
| $R^6$ | signifies hydrogen, $C_{1-4}$alkyl or $C_{1-4}$fluoroalkyl, |
| | or |
| $R^{5'}$ and $R^6$ | together signify tri- or tetramethylene in which one methylene can be replaced by oxygen or sulphur and which is optionally substituted with $C_{1-3}$alkyl, |
| $R^7$ | signifies $C_{1-4}$alkyl |
| | and |
| X | signifies oxygen or sulphur, |
| | with the proviso that $R^6$ signifies exclusively $C_{1-4}$alkyl or $C_{1-4}$fluoroalkyl when $R^{5'}$ stands for fluorine. |

**16.** Isopropyl 2-chlro-4-fluoro-5-(3[2(ethoxycarbonyl)-1-cyclohexen-1-yl]ureido)benzoate

**17.** Isopropyl 2-chloro-4-fluoro-5(3[2(ethoxycarbonyl)-1-methylpropenyl]ureido)benzoate.

**18.** Weed control composition, characterized in that it contains an effective amount of at least one compound of the general formula I' according to claim 2 as well as formulation adjuvants.

**19.** Weed control composition according to claim 18, characterized in that it contains an effective amount of at least one of the compounds stated in claim 11 as well as formulation adjuvants.

**20.** Weed control composition according to claim 18, characterized in that it contains an effective amount of at least one of the compounds stated in claim 12 as well as formulation adjuvants.

**21.** Process for the preparation of compounds of the general formula I according to claim 1 and of salts of those compounds of the formula I in which $R^1$ and/or $R^2$ signifies hydrogen, which comprises

a) for the preparation of those compounds of the formula I in which $R^1$ signifies hydrogen and $R^2$ signifies $C_{1-6}$alkyl, $C_{2-4}$alkenyl, $C_{2-4}$alkynyl or $C_{2-6}$alkoxyalkyl and $R^5$ has a signification other than chlorine, bromine, iodine, chloromethyl, bromomethyl, hydroxymethyl, ($C_{1-5}$alkoxy)methyl, ($C_{1-5}$alkylthio)methyl, cyano, nitro or thiocyanato, as well as of metal salts of those compounds of the formula I in which $R^1$ signifies hydrogen, subjecting a compound of the general formula

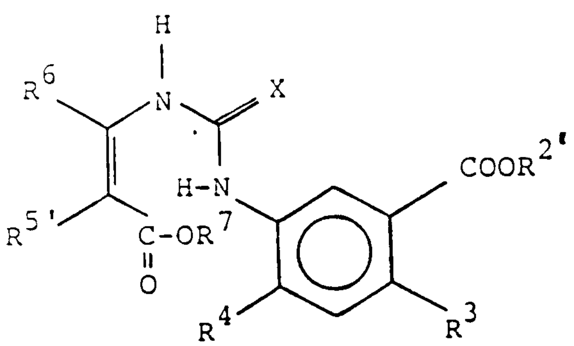

II

wherein

| | |
|---|---|
| $R^{2'}$ | signifies $C_{1-6}$alkyl, $C_{2-4}$alkenyl, $C_{2-4}$alkynyl or $C_{2-6}$alkoxyalkyl, |
| $R^3$, $R^4$, $R^6$ and X | have the significations given above, |
| $R^{5'}$ | signifies hydrogen, fluorine, $C_{1-4}$alkyl or, together with $R^6$, signifies tri- or tetramethylene which is optionally modified, as more precisely defined above, |
| | and |
| $R^7$ | signifies lower alkyl |

to a base-catalyzed cyclization by treatment with a base and, if desired, converting a metal salt form of the uracil derivative, which may be obtained, into the corresponding acid form ($R^1$ = hydrogen) by treatment with an acid,

b) for the preparation of those compounds of the formula I in which $R^1$ signifies $C_{1-4}$alkyl,

$C_{2-4}$alkenyl, $C_{2-4}$alkynyl, $C_{2-6}$alkoxyalkyl, formyl, $C_{2-6}$alkanoyl or $C_{2-6}$alkoxycarbonyl, treating a uracil derivative of the general formula

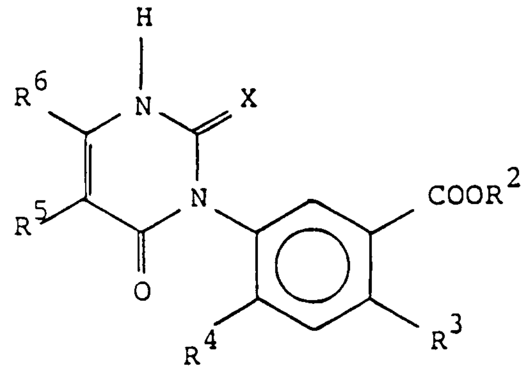

Ia

wherein $R^2$, $R^3$, $R^4$, $R^5$, $R^6$ and X have the significations given above, to bring about alkylation or acylation with a corresponding alkylating or acylating agent containing a $C_{1-4}$alkyl, $C_{2-4}$alkenyl, $C_{2-4}$alkynyl, $C_{2-6}$alkoxyalkyl, formyl, $C_{2-6}$alkanoyl or $C_{2-6}$alkoxycarbonyl group,

c) for the preparation of those compounds of the formula I in which $R^1$ signifies $C_{1-4}$alkyl, $C_{2-4}$alkenyl, $C_{2-4}$alkynyl or $C_{2-6}$alkoxyalkyl, $R^5$ is different from chlorine, bromine, iodine, chloromethyl, bromomethyl, hydroxymethyl, ($C_{1-5}$alkoxy)methyl, ($C_{1-5}$alkylthio)methyl, cyano, nitro or thiocyanato and X signifies sulphur, reacting a compound of the general formula

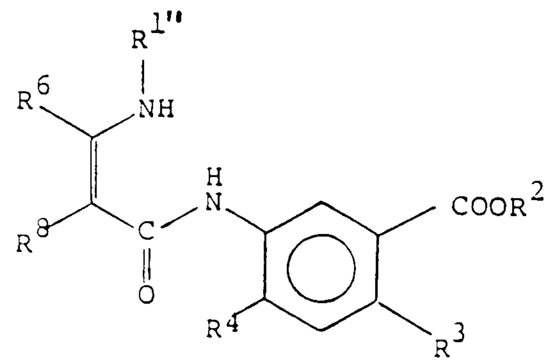

III

wherein

| | |
|---|---|
| $R^{1''}$ | signifies $C_{1-4}$alkyl, $C_{2-4}$alkenyl, $C_{2-4}$alkynyl or $C_{2-6}$alkoxyalkyl, |
| $R^2$, $R^3$, $R^4$ and $R^6$ | have the significations given above, and |
| $R^8$ | signifies fluorine, $C_{1-4}$alkyl, ($C_{1-4}$alkoxy)carbonyl or, together with $R^6$, signifies tri- or tetramethylene which is as defined above, where, in the case that $R^8$ signifies fluorine, $R^6$ is $C_{1-4}$alkyl or $C_{1-4}$fluoroalkyl, |

with N,N'-thiocarbonyldiimidazolide or thiophosgene and, when $R^8$ signifies ($C_{1-4}$alkoxy)carbonyl, heating the 5-alkoxycarbonyl-2-thiouracil thus prepared of the general formula

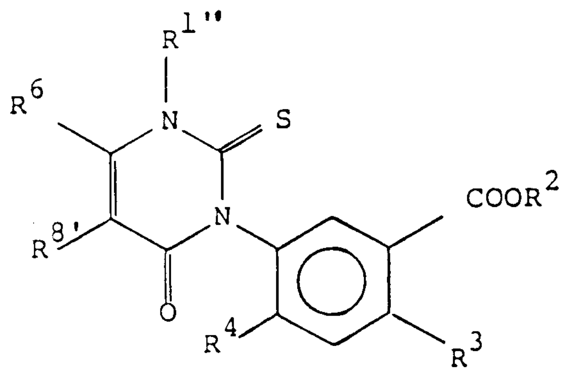

IV

wherein $R^{1''}$, $R^2$, $R^3$, $R^4$ and $R^6$ have the significations given above and $R^{8'}$ signifies ($C_{1-4}$alkoxy)-carbonyl,
to bring about hydrolysis and decarboxylation in the presence of aqueous hydrochloric acid or trifluoroacetic acid,
d) for the preparation of those compounds of the formula I in which $R^5$ signifies chlorine, bromine or iodine, treating a uracil derivative of the general formula

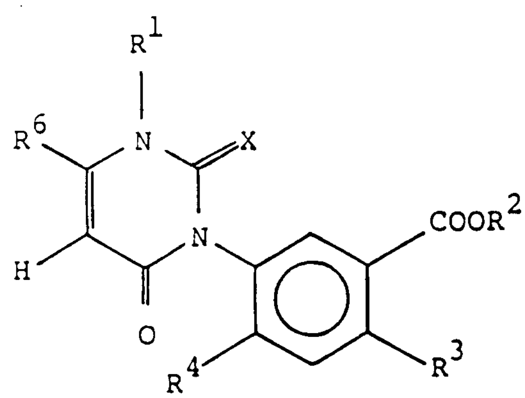

Ib

wherein $R^1$, $R^2$, $R^3$, $R^4$, $R^6$ and X have the significations given above, to bring about chlorination with elemental chloride or sulfuryl chloride, by bromination with elemental bromine or sulfuryl bromide or iodisation with elemental iodine,
e) for the preparation of those compounds of the formula I in which $R^5$ signifies chloro- or bromomethyl, (i) treating a uracil derivative of the formula Ib given above with chloro- or bromomethoxymethane or (ii) treating a 5-hydroxymethyluracil of the general formula

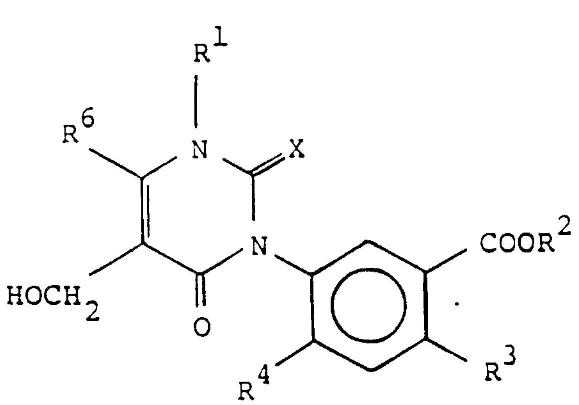

Ic

wherein $R^2$, $R^3$, $R^4$, $R^6$ and X have the significations given above, with thionyl chloride or bromide or (iii) treating a 5-methyluracil of the general formula

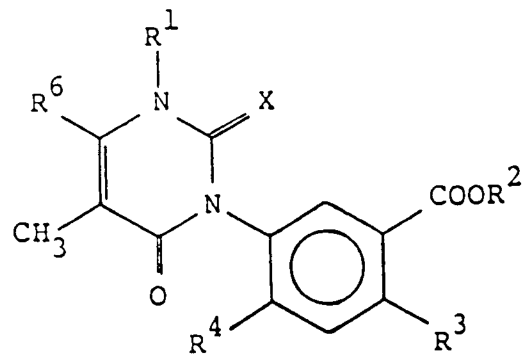

Id

wherein $R^1$, $R^2$, $R^3$, $R^4$, $R^6$ and X have the significations given above, with N-chlorosuccinimide or N-bromosuccinimide,

f) for the preparation of those compounds of the formula I in which $R^5$ signifies hydroxymethyl, treating a 5-halomethyluracil of the general formula

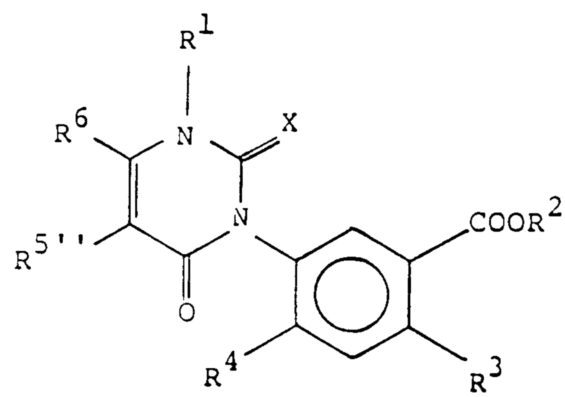

Ie

wherein $R^1$, $R^2$, $R^3$, $R^4$, $R^6$ and X have the significations given above and $R^{5''}$ signifies chloro-, bromo- or iodomethyl,

to bring about hydrolysis with an aqueous solution of an inorganic base,

g) for the preparation of those compounds of the formula I in which $R^5$ signifies ($C_{1-5}$alkoxy)methyl or ($C_{1-5}$alkylthio)methyl, treating a 5-halomethyluracil of the formula Ie given above, in which $R^{5''}$ signifies chloro- or bromomethyl, with an alkali metal alcoholate or thioalcoholate of the general formula

$R^9M$ V

wherein $R^9$ signifies $C_{1-5}$alkoxy or $C_{1-5}$alkylthio and M signifies an alkali metal,

or with a $C_{1-5}$alkanol or $C_{1-5}$alkylmercaptan,

h) for the preparation of those compounds of the formula I in which $R^5$ signifies ($C_{1-5}$alkylthio)-methyl, treating a 5-hydroxymethyluracil of the formula Ic given above with a $C_{1-5}$alkylmercaptan,

i) for the preparation of those compounds of the formula I in which $R^1$ signifies hydrogen, $R^5$ signifies cyano, $R^6$ signifies hydrogen or $C_{1-4}$alkyl and X signifies oxygen, treating a compound of the general formula

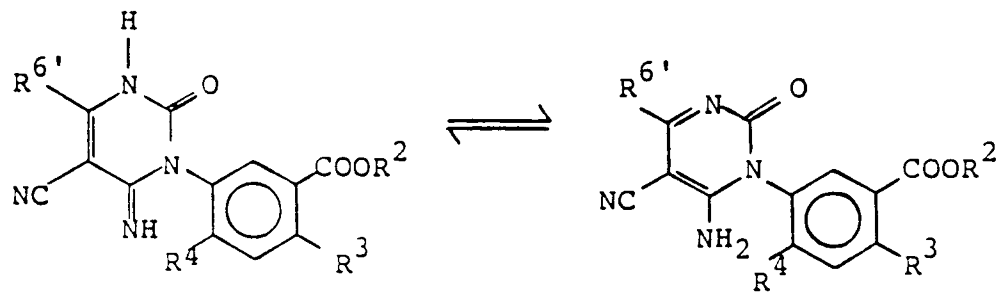

VI

wherein $R^2$, $R^3$ and $R^4$ have the significations given above and $R^{6'}$ signifies hydrogen or $C_{1-4}$alkyl, to bring about an acid-catalyzed hydrolysis with an aqueous solution of an inorganic acid,

j) for the preparation of those compounds of the formula I in which $R^5$ signifies nitro, treating a uracil derivative of the formula Ib given above with nitric acid,

k) for the preparation of those compounds of the formula I in which $R^5$ signifies thiocyanato, treating a uracil derivative of the formula Ib given above with thiocyanogen,

l) for the preparation of those compounds of the formula I in which $R^2$ signifies hydrogen, hydrolysing a bensoic acid ester of the general formula

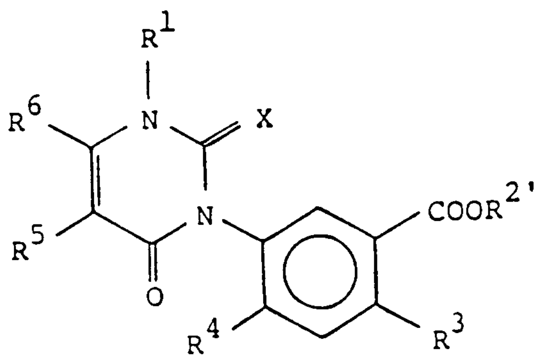

If

wherein $R^1$, $R^{2'}$, $R^3$, $R^4$, $R^5$, $R^6$ and X have the significations given above, to the corresponding benzoic acid by treatment with an aqueous solution of an organic solvent and with an inorganic base,

m) for the preparation of those compounds of the formula I in which $R^2$ signifies $C_{1-6}$alkyl, $C_{2-4}$alkenyl, $C_{2-4}$alkynyl or $C_{2-6}$alkoxyalkyl, appropriately esterifying a benzoic acid of the general formula

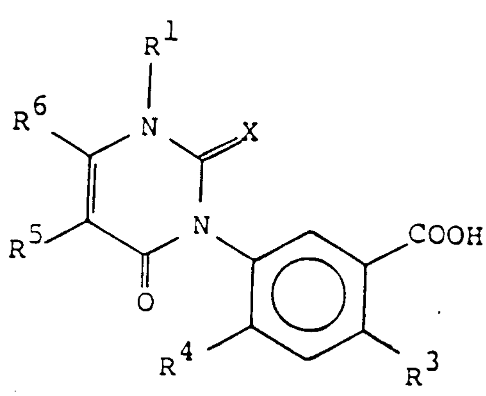

Ig

wherein $R^1$, $R^3$, $R^4$, $R^5$, $R^6$ and X have the significations given above, or a reactive derivative thereof,

where, in the case in which a salt of the benzoic acid Ig is used as the reactive derivative, it is reacted with a chloride, bromide, iodide, sulphate, mesylate or tosylate of a $C_{1-6}$alkyl, $C_{2-4}$alkenyl $C_{2-4}$alkynyl or $C_{2-6}$alkoxyalkyl and, in the case in which an acid halide or the corresponding O-acyl-1,3-dicyclohexylisourea, N-acylimidazole or acid anhydride of the benzoic acid Ig is used as the reactive derivative, it is reacted with a $C_{1-6}$alkanol, $C_{2-4}$alkenol, $C_{2-4}$alkynol or $C_{2-6}$alkoxyalkanol, or

n) for the preparation of those compounds of the formula I in which $R^2$ signifies $C_{2-6}$alkyl, $C_{2-4}$alkenyl, $C_{2-4}$alkynyl or $C_{2-6}$alkoxyalkyl, subjecting a benzoic acid ester of the formula If given above to a transesterification reaction with an alkanol, alkenol or alkynol of the general formula

$R^{2''}OH$ VII

wherein $R^{2''}$ signifies $C_{2-6}$alkyl, $C_{2-4}$alkenyl, $C_{2-4}$alkynyl or $C_{2-6}$alkoxyalkyl,
where the reagent of the formula VII is higher boiling than the alkanol, alkenol or alkynol $R^{2'}OH$, and, if desired, converting a compound thus obtained of the formula I in which $R^1$ and/or $R^2$ signifies hydrogen into a salt.

**22.** Process according to claim 21, characterized in that one of the variants a), b), d), e), g), h), i), j), k), l), m) and n) and if appropriate the optional conversion into a salt is carried out.

**23.** Method for the control of weeds, characterized in that the locus to be protected against weeds and/or the weeds are treated one with an effective amount of a compound in accordance with any one of claims 2 to 7, 9 to 11 and 13 or of a composition in accordance with either of claims 18 and 19.

**24.** Method for the control of weeds, characterized in that the locus to be protected against weeds and/or the weeds are treated with an effective amount of a compound in accordance with any one of claims 8, 12 and 14 or of a composition in accordance with claim 20.

**25.** The use of a compound in accordance with any one of claims 2 to 7, 9 to 11 and 13 or of a composition in accordance with either of claims 18 and 19 for the control of weeds.

**26.** The use of a compound in accordance with any one of claims 8, 12 and 14 or of a composition in accordance with claim 20 for the control of weeds.

**Claims for the following Contracting State : AT**

**1.** Weed control composition, characterized in that it contains an effective amount of one or more compounds of the general formula

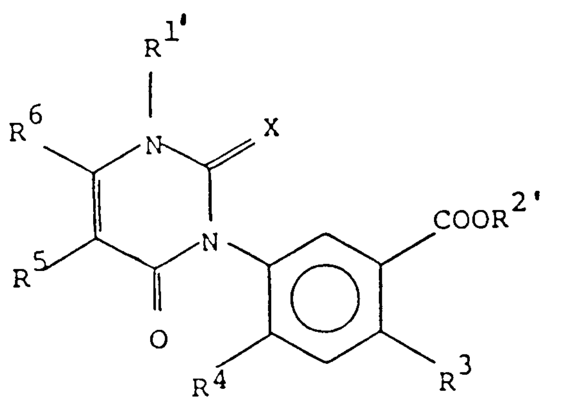

I'

wherein

| | |
|---|---|
| $R^{1'}$ | signifies $C_{1-4}$alkyl, $C_{2-4}$alkenyl, $C_{2-4}$alkynyl, $C_{2-6}$alkoxyalkyl, formyl, $C_{2-6}$alkanoyl or $C_{2-6}$alkoxycarbonyl, |
| $R^{2'}$ | signifies $C_{1-6}$alkyl $C_{2-4}$alkenyl, $C_{2-4}$alkynyl or $C_{2-6}$alkoxyalkyl, |
| $R^3$ | signifies halogen or nitro, |

| | |
|---|---|
| $R^4$ | signifies hydrogen or halogen, |
| $R^5$ | signifies hydrogen, halogen, $C_{1-4}$alkyl, chloromethyl, bromomethyl, hydroxymethyl, ($C_{1-5}$alkoxy)methyl$_1$ ($C_{1-5}$alkylthio)methyl, cyano, nitro or thiocyanato, |
| $R^6$ | signifies hydrogen, $C_{1-4}$alkyl or $C_{1-4}$fluoroalkyl, |
| | or |
| $R^5$ and $R^6$ | together signify tri- or tetramethylene in which one methylene can be replaced by oxygen or sulphur and which is optionally substituted with $C_{1-3}$alkyl, |
| | and |
| X | signifies oxygen or sulphur, |
| | with the provisos that (i) $R^5$ signifies exclusively $C_{1-4}$alkyl or $C_{1-4}$fluoroalkyl when $R^5$ stands for fluorine and (ii) $R^6$ signifies exclusively hydrogen or $C_{1-4}$alkyl and X signifies exclusively oxygen when $R^5$ stands for cyano, |

and formulation adjuvants.

2. Weed control composition according to claim 1, wherein $R^{1'}$ in the formula I' signifies methyl.

3. Weed control composition according to claim 1 or 2, wherein $R^{2'}$ in the formula I' signifies $C_{1-6}$alkyl or $C_{2-6}$alkoxyalkyl.

4. Weed control composition according to any one of claims 1 to 3, wherein $R^3$ in the formula I' signifies chlorine or bromine.

5. Weed control composition according to any one of claims 1 to 4, wherein $R^4$ in the formula I' signifies fluorine.

6. Weed control composition according to any one of claims 1 to 5, wherein $R^5$ in the formula I' signifies hydrogen, chlorine, bromine, methyl or ethyl.

7. Weed control composition according to any one of claims 1 to 6, wherein $R^5$ in the formula I' signifies fluorine.

8. Weed control composition according to any one of claims 1 to 6, wherein $R^6$ in the formula I' signifies methyl, ethyl or trifluoromethyl.

9. Weed control composition according to any one of claims 1 to 5, wherein $R^5$ and $R^6$ in the formula I' together signify tri- or tetramethylene.

10. Weed control composition according to claim 1, characterized in that it contains an effective amount of one or more compounds selected from the group
isopropyl 2-chloro-4-fluoro-5-(1,2,4,5,6,7-hexahydro-1-methyl-2,4-dioxo-3H-cyclopenta[d]pyrimidin-3-yl)benzoate,
isopropyl 2-chloro-4-fluoro-5-[1,4,5,6,7,8-hexahydro-1-methyl-2,4-dioxo-3(2H)-quinazolinyl]benzoate,
isopropyl 2-chloro-4-fluoro-5-[3,6-dihydro-3,4-dimethyl-2,6-dioxo-1(2H)-pyrimidinyl]benzoate,
isopropyl 2-chloro-4-fluoro-5-[5-bromo-3-,6-dihydro-3,4-dimethyl-2,6-dioxo-1(2H)-pyrimidinyl]-benzoate,
isopropyl 2-chloro-4-fluoro-5-[3,6-dihydro-3,4-dimethyl-5-iodo-2,6-dioxo-1(2H)-pyrimidinyl]benzoate,
isopropyl 2-chloro-4-fluoro-5-[3,6-dihydro-3,4-dimethyl-5-hydroxymethyl-2,6-dioxo-1(2H)-pyrimidinyl]benzoate,
isopropyl 2-chloro-4-fluoro-5-[3,6-dihydro-4-ethyl-3-methyl-2,6-dioxo-1(2H) -pyrimidinyl]benzoate,
isopropyl 2-chloro-4-fluoro-5-[3,6-dihydro-3-methyl-4-trifluoromethyl-2,6-dioxo-1(2H)-pyrimidinyl]-benzoate,
isopropyl 2-bromo-4-fluoro-5-(1,2,4,5,6,7-hexahydro-1-methyl-2,4-dioxo-3H-cyclopenta[d]pyrimidin-3-yl)benzoate,
isopropyl 2-chloro-4-fluoro-5-[3,6-dihydro-3,4-dimethyl-5-nitro-2,6-dioxo-1(2H)-pyrimidinyl]benzoate,
2-methoxy-1-methylethyl 2-chloro-4-fluoro-5-(1,2,4,5,6,7-hexahydro-1-methyl-2,4-dioxo-3H-cyclopenta[d]pyrimidin-3-yl)benzoate and
tert-butyl 2-chloro-4-fluoro-5-(1,2,4,5,6,7-hexahydro-1-methyl-2,4-dioxo-3H-cyclopenta[d]pyrimidin-3-yl)benzoate

and formulation adjuvants.

11. Weed control composition according to claim 1, characterized in that it contains an effective amount of one or more compounds selected from the group
isopropyl 2-chloro-4-fluoro-5-[3,6-dihydro-3,4-dimethyl-5-fluoro-2,6-dioxo-1(2H)-pyrimidinyl]-benzoate,
isopropyl 2-chloro-4-fluoro-5-[3,6-dihydro-3-methyl-4-propyl-2,6-dioxo-1(2H)-pyrimidinyl]benzoate and
isopropyl 2-chloro-4-fluoro-5-[3,6-dihydro-5-fluoro-3-methyl-4-trifluoromethyl-2,6-dioxo-1(2H)-pyrimidinyl]benzoate
and formulation adjuvants.

12. Weed control composition according to claim 1, characterized in that it contains an effective amount of one or more compounds selected from the group
ethyl 2-chloro-5-(1,2,4,5,6,7-hexahydro-1-methyl-2,4-dioxo-3H-cyclopenta[d]pyrimidin-3-yl)benzoate,
ethyl 2-chloro-5-[1,4,5,6,7,8-hexahydro-1-methyl-2,4-dioxo-3(2H)-quinazolinyl]benzoate
ethyl 2-chloro-5-[3,6-dihydro-3,4-dimethyl-2,6-dioxo-1(2H) -pyrimidinyl]benzoate,
ethyl 2-chloro-5-[3,6-dihydro-3,4,5-trimethyl-2,6-dioxo-1(2H) -pyrimidinyl]benzoate,
isopropyl 2-chloro-4-fluoro-5-[3,6dihydro-3,4,5-trimethyl-2,6-dioxo-1(2H)-pyrimidinyl]benzoate,
isopropyl 2-chloro-4-fluoro-5-[1,2,5,7-tetrahydro-1-methyl-2,4-dioxothieno[3,4-d]pyrimidin-3(4H)-yl]-benzoate,
isopropyl 2-chloro-4-fluoro-5-[3,6-dihydro-3,5-dimethyl-4-ethyl-2,6-dioxo-1(2H)-pyrimidinyl]benzoate,
isopropyl 2-chloro-4-fluoro-5-[5-cyano-3,6-dihydro-3,4-dimethyl-2,6-dioxo-1(2H) -pyrimidinyl]-benzoate,
isopropyl 2,4-dichloro-5-(1,2,4,5,6,7-hexahydro-1-methyl-2,4-dioxo-3H-cyclopenta[d]pyrimidin-3-yl)-benzoate,
isopropyl 2-bromo-4-chloro-5-(1,2,4,5,6,7-hexahydro-1-methyl-2,4-dioxo-3H-cyclopenta[d]pyrimidin-3-yl)benzoate,
isopropyl 2-chloro-4-fluoro-5-(1,2,4,5,6,7-hexahydro-1-acetyl-2,4-dioxo-3H-cyclopenta[d]pyrimidin-3-yl)benzoate,
isopropyl 2-chloro-4-fluoro-5-[1,2,4,5,6,7-hexahydro-1-methoxycarbonyl-2,4-dioxo-3H-cyclopenta[d]-pyrimidin-3-yl)benzoate,
isopropyl 2-chloro-4-fluoro-5-[5-chloro-3,6-dihydro-3,4-dimethyl-2,6-dioxo-1(2H)-pyrimidinyl]-benzoate,
tert-butyl 2-chloro-5-(1,2,4,5,6,7-hexahydro-1-methyl-2,4-dioxo-3H-cyclopenta[d]pyrimidin-3-yl)-benzoate,
isopropyl 2-chloro-5-[1,4,5,6,7,8-hexahydro-1-methyl-2,4-dioxo-3(2H)-quinazolinyl]benzoate,
2-propenyl 2-chloro-4-fluoro-5-(1,2,4,5,6,7-hexahydro-1-methyl-2,4-dioxo-3H-cyclopenta[d]pyrimidin-3-yl)benzoate,
2-propynyl 2-chloro-4-fluoro-5-(1,2,4,5,6,7-hexahydro-1-methyl-2,4-dioxo-3H-cyclopenta[d]pyrimidin-3-yl)benzoate
and
methyl 2-chloro-4-fluoro-5-(1,2,4,5,6,7-hexahydro-1-methyl-2,4-dioxo-3H-cyclopenta[d]pyrimidin-3-yl)benzoate
and formulation adjuvants.

13. Weed control composition according to claim 1, characterized in that it contains an effective amount of isopropyl 2-chloro-4-fluoro-5-[3,6-dihydro-3,4-dimethyl-5-methoxymethyl-2,6-dioxo-1(2H)-pyrimidinyl]-benzoate and formulation adjuvants.

14. Process for the preparation of compounds of the general formula

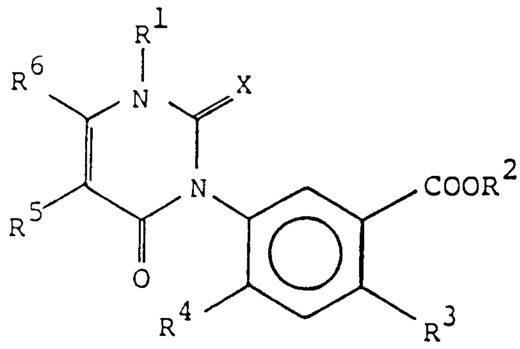

I

wherein

$R^1$ signifies hydrogen, $C_{1-4}$alkyl, $C_{2-4}$alkenyl, $C_{2-4}$alkynyl, $C_{2-6}$alkoxyalkyl, formyl, $C_{2-6}$alkanoyl or $C_{2-6}$alkoxycarbonyl,

and $R^2$, $R^3$, $R^4$, $R^5$, $R^6$ and X have the significations given in claim 1 and of salts of those compounds of the formula I in which $R^1$ and/or $R^2$ signifies hydrogen, which comprises

a) for the preparation of those compounds of the formula I in which $R^1$ signifies hydrogen and $R^2$ signifies $C_{1-6}$alkyl, $C_{2-4}$alkenyl, $C_{2-4}$alkynyl or $C_{2-6}$alkoxyalkyl and $R^5$ has a signification other than chlorine, bromine, iodine, chloromethyl, bromomethyl, hydroxymethyl, ($C_{1-5}$alkoxy)methyl, ($C_{1-5}$alkylthio)methyl, cyano, nitro or thiocyanato, as well as of metal salts of those compounds of the formula I in which $R^1$ signifies hydrogen, subjecting a compound of the general formula

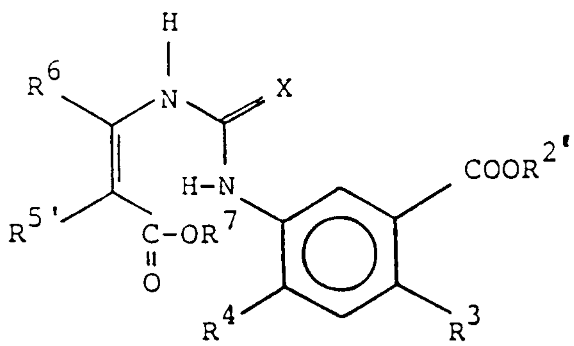

II

wherein

| | |
|---|---|
| $R^{2'}$ | signifies $C_{1-6}$alkyl, $C_{2-4}$alkenyl, $C_{2-4}$alkynyl or $C_{2-6}$alkoxyalkyl, |
| $R^3$, $R^4$, $R^6$ and X | have the significations given in claim 1, |
| $R^{5'}$ | signifies hydrogen, fluorine, $C_{1-4}$alkyl or, together with $R^6$, signifies tri- or tetramethylene which is optionally modified, as more precisely defined above, and |
| $R^7$ | signifies lower alkyl |

to a base-catalyzed cyclization by treatment with a base and, if desired, converting a metal salt form of the uracil derivative, which may be obtained, into the corresponding acid form ($R^1$ = hydrogen) by treatment with an acid,

b) for the preparation of those compounds of the formula I in which $R^1$ signifies $C_{1-4}$alkyl, $C_{2-4}$alkenyl, $C_{2-4}$alkynyl $C_{2-6}$alkoxyalkyl, formyl, $C_{2-6}$alkanoyl or $C_{2-6}$alkoxycarbonyl, treating a uracil derivative of the general formula

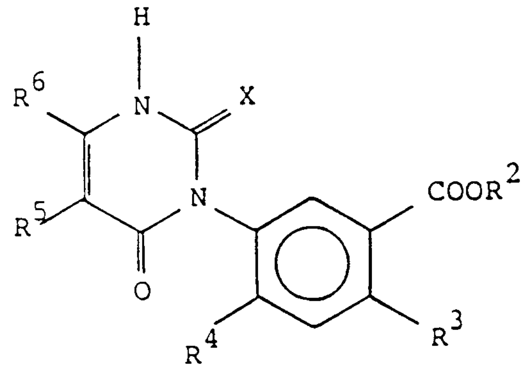

Ia

wherein $R^2$, $R^3$, $R^4$, $R^5$, $R^6$ and X have the significations given in claim 1, to bring about alkylation or acylation with a corresponding alkylating or acylating agent containing a $C_{1-4}$alkyl, $C_{2-4}$alkenyl, $C_{2-4}$alkynyl, $C_{2-6}$alkoxyalkyl, formyl, $C_{2-6}$alkanoyl or $C_{2-6}$alkoxycarbonyl group,

c) for the preparation of those compounds of the formula I in which $R^1$ signifies $C_{1-4}$alkyl, $C_{2-4}$alkenyl, $C_{2-4}$alkynyl or $C_{2-6}$alkoxyalkyl, $R^5$ is different from chlorine, bromine, iodine, chloromethyl, bromomethyl, hydroxymethyl, ($C_{1-5}$alkoxy)methyl, ($C_{2-5}$alkylthio)methyl, cyano, nitro or thiocyanato and X signifies sulphur, reacting a compound of the general formula

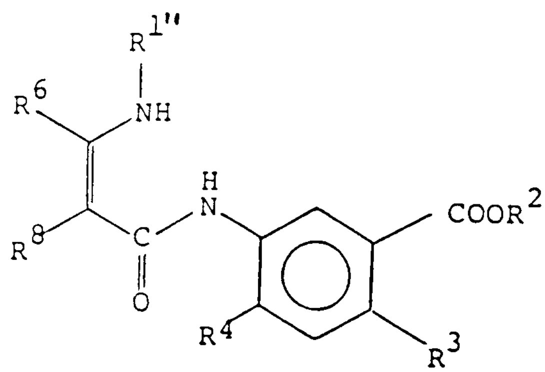

III

wherein

| | |
|---|---|
| $R^{1''}$ | signifies $C_{1-4}$alkyl, $C_{2-4}$alkenyl, $C_{2-4}$alkynyl or $C_{2-6}$alkoxyalkyl, |
| $R^2$, $R^3$, $R^4$ and $R^6$ | have the significations given in claim 1, and |
| $R^8$ | signifies fluorine, $C_{1-4}$alkyl, ($C_{1-4}$alkoxy)carbonyl or, together with $R^5$, signifies tri- or tetramethylene which is as defined in claim 1, where, in the case that $R^8$ signifies fluorine, $R^6$ is $C_{1-4}$alkyl or $C_{1-4}$fluoroalkyl, |

with N,N'-thiocarbonyldiimidazolide or thiophosgene and, when $R^8$ signifies ($C_{1-4}$alkoxy)carbonyl, heating the 5-alkoxycarbonyl-2-thiouracil thus prepared of the general formula

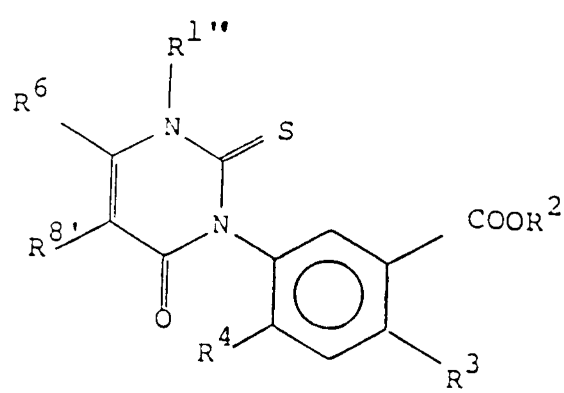

IV

wherein $R^{1''}$, $R^2$, $R^3$, $R^4$ and $R^6$ have the significations given above and in claim 1 and $R^{8'}$ signifies ($C_{1-4}$alkoxy)carbonyl, to bring about hydrolysis and decarboxylation in the presence of aqueous hydrochloric acid or trifluoroacetic acid,

d) for the preparation of those compounds of the formula I in which $R^5$ signifies chlorine, bromine or iodine, treating a uracil derivative of the general formula

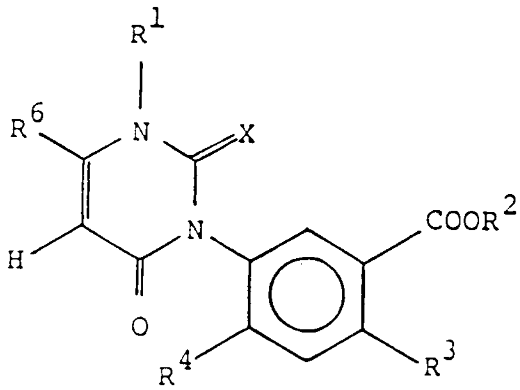

Ib

wherein $R^1$, $R^2$, $R^3$, $R^4$, $R^6$ and X have the signification s given above and in claim 1, to bring about chlorination with elemental chloride or sulfuryl chloride, by bromination with elemental bromine or sulfuryl bromide or iodisation with elemental iodine

e) for the preparation of those compounds of the formula I in which $R^5$ signifies chloro- or bromomethyl, (i) treating a uracil derivative of the formula Ib given above with chloro- or bromomethoxymethane or (ii) treating a 5-hydroxymethyluracil of the general formula

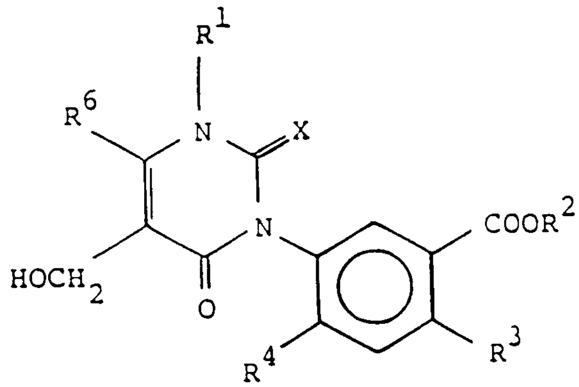

Ic

wherein $R^2$, $R^3$, $R^4$, $R^6$ and X have the significations given in claim 1, with thionyl chloride or bromide or (iii) treating a 5-methyluracil of the general formula

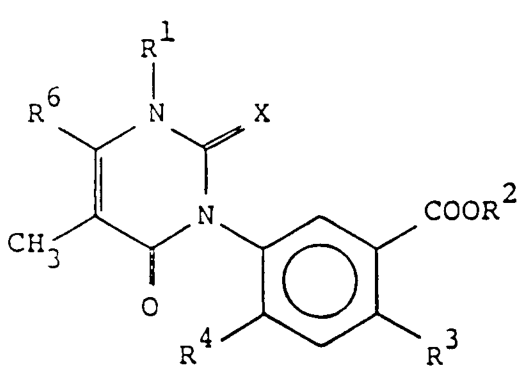

Id

wherein $R^1$, $R^2$, $R^3$, $R^4$, $R^6$ and X have the significations given above and in claim 1, with N-chlorosuccinimide or N-bromosuccinimide,

f) for the preparation of those compounds of the formula I in which $R^5$ signifies hydroxymethyl, treating a 5-halomethyluracil of the general formula

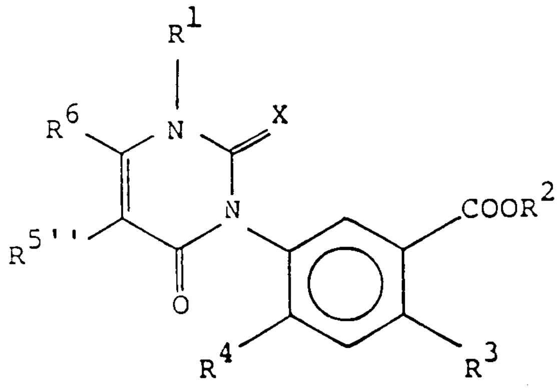

Ie

wherein $R^1$, $R^2$, $R^3$, $R^4$, $R^6$ and X have the significations given above and in claim 1 and $R^{5''}$ signifies chloro-, bromo- or iodomethyl,

to bring about hydrolysis with an aqueous solution of an inorganic base,

g) for the preparation of those compounds of the formula I in which $R^5$ signifies ($C_{1-5}$alkoxy)methyl or ($C_{1-5}$alkylthio)methyl, treating a 5-halomethyluracil of the formula Ie given above, in which $R^{5''}$ signifies chloro- or bromomethyl, with an alkali metal alcoholate or thioalcoholate of the general formula

$R^9M$ V

wherein $R^9$ signifies $C_{1-5}$alkoxy or $C_{1-5}$alkylthio and M signifies an alkali metal,

or with a $C_{1-5}$alkanol or $C_{1-5}$alkylmercaptan,

h) for the preparation of those compounds of the formula I in which $R^5$ signifies ($C_{1-5}$alkylthio)-methyl, treating a 5-hydroxymethyluracil of the formula Ic given above with a $C_{1-5}$alkylmercaptan,

i) for the preparation of those compounds of the formula I in which $R^1$ signifies hydrogen, $R^5$ signifies cyano, $R^6$ signifies hydrogen or $C_{1-4}$alkyl and X signifies oxygen, treating a compound of the general formula

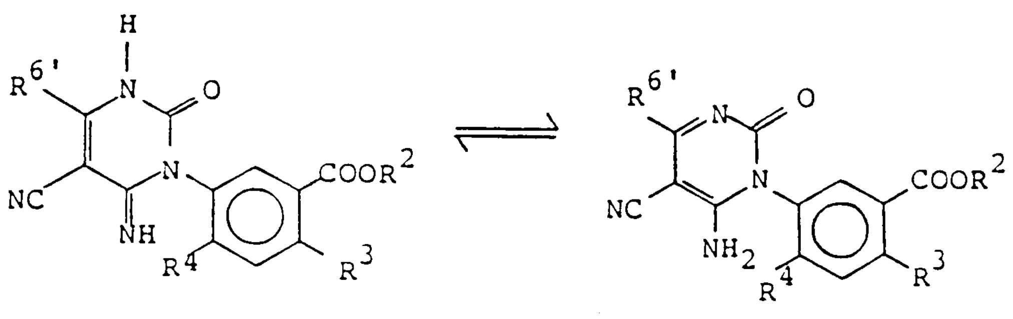

VI

wherein $R^2$, $R^3$ and $R^4$ have the significations given in claim 1 and $R^{6'}$ signifies hydrogen or $C_{1-4}$alkyl,

to bring about an acid-catalyzed hydrolysis with an aqueous solution of an inorganic acid,

j) for the preparation of those compounds of the formula I in which $R^5$ signifies nitro, treating a uracil derivative of the formula Ib given above with nitric acid,

k) for the preparation of those compounds of the formula I in which $R^5$ signifies thiocyanato, treating a uracil derivative of the formula Ib given above with thiocyanogen,

l) for the preparation of those compounds of the formula I in which $R^2$ signifies hydrogen, hydrolysing a benzoic acid ester of the general formula

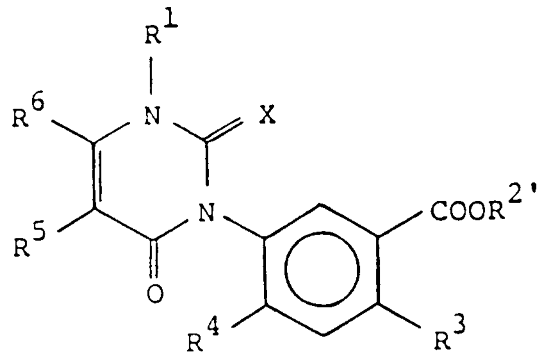

If

wherein $R^1$, $R^{2'}$, $R^3$, $R^4$, $R^5$, $R^6$ and X have the significations given above and in claim 1, to the corresponding benzoic acid by treatment with an aqueous solution of an organic solvent and with an inorganic base,

m) for the preparation of those compounds of the formula I in which $R^2$ signifies $C_{1-6}$alkyl, $C_{2-4}$alkenyl, $C_{2-4}$alkynyl or $C_{2-6}$alkoxyalkyl, appropriately esterifying a benzoic acid of the general formula

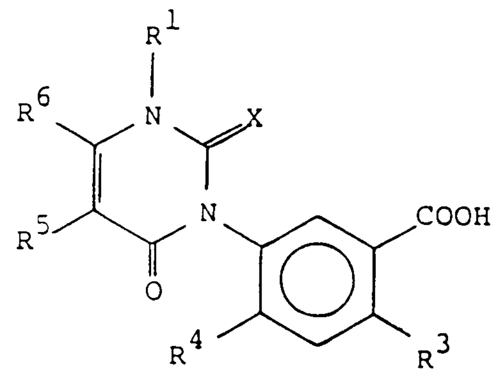

Ig

wherein $R^1$, $R^3$, $R^4$, $R^5$, $R^6$ and X have the significations given above and in claim 1, or a reactive derivative thereof, where, in the case in which a salt of the benzoic acid Ig is used as the reactive derivative, it is reacted with a chloride, bromide, iodide, sulphate, mesylate or tosylate of a $C_{1-6}$alkyl, $C_{2-4}$alkenyl, $C_{2-4}$alkynyl or $C_{2-6}$alkoxyalkyl and, in the case in which an acid halide or the corresponding O-acyl-1,3-dicyclohexylisourea, N-acylimidazole or acid anhydride of the benzoic acid Ig is used as the reactive derivative, it is reacted with a $C_{1-6}$alkanol, $C_{2-4}$alkenol, $C_{2-4}$alkynol or $C_{2-6}$alkoxyalkanol, or

n) for the preparation of those compounds of the formula I in which $R^2$ signifies $C_{2-6}$alkyl, $C_{2-4}$alkenyl, $C_{2-4}$alkynyl or $C_{2-6}$alkoxyalkyl, subjecting a benzoic acid ester of the formula If given above to a transesterification reaction with an alkanol, alkenol or alkynol of the general formula

$R^{2''}OH$ VII

wherein $R^{2''}$ signifies $C_{2-6}$alkyl, $C_{2-4}$alkenyl, $C_{2-4}$alkynyl or $C_{2-6}$alkoxyalkyl, where the reagent of the formula VII is higher boiling than the alkanol, alkenol or alkynol $R^{2'}OH$, and, if desired, converting a compound thus obtained of the formula I in which $R^1$ and/or $R^2$ signifies hydrogen into a salt.

**15.** Process according to claim 14, characterized in that one of the variants a), b), d), e), g), h), i), j), k), l), m) and n) and if desired the optional conversion into a salt is carried out.

16. Method for the preparation of a weed control composition, characterized in that one or more of the compounds stated in claim 1 are mixed with formulation adjuvants.

17. Method for the control of weeds, characterized in that the locus to be protected against weeds and/or the weeds are treated with an effective amount of a composition in accordance with any one of claims 1 to 6, 8 to 10 and 12.

18. Method for the control of weeds, characterized in that the locus to be protected against weeds and/or the weeds are treated with an effective amount of a composition in accordance with any one of claims 7, 11 and 13.

19. The use of a composition in accordance with any one of claims 1 to 6, 8 to 10 and 12 for the control of weeds.

20. The use of a composition in accordance with any one of claims 7, 11 and 13 for the control of weeds.

**Revendications**
**Revendications pour les Etats contractants suivants : BE, CH, DE, FR, GB, IT, LI, LU, NL, SE**

1. Composés de formule générale

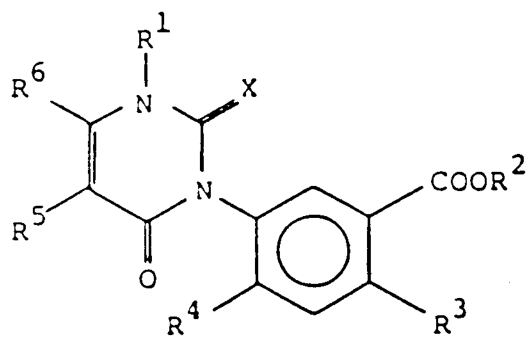

I

dans laquelle

| | |
|---|---|
| $R^1$ | représente l'hydrogène, un groupe alkyle en C 1-C 4, alcényle en C 2-C 4, alcynyle en C 2-C 4, alcoxyalkyle en C 2-C 6, formyle, alcanoyle en C 2-C 6 ou alcoxycarbonyle en C 2-C 6, |
| $R^2$ | représente l'hydrogène, un groupe alkyle en C 1-C 6, alcényle en C 2-C 4, alcynyle en C 2-C 4 ou alcoxyalkyle en C 2-C 6, |
| $R^3$ | représente un halogène ou un groupe nitro, |
| $R^4$ | représente l'hydrogène ou un halogène, |
| $R^5$ | représente l'hydrogène, un halogène, un groupe alkyle en C 1-C 4, chlorométhyle, bromométhyle, hydroxyméthyle, (alcoxy en C 1-C 5)-méthyle, (alkylthio en C 1-C 5)-méthyle, cyano, nitro ou thiocyanato, |
| $R^6$ | représente l'hydrogène, un groupe alkyle en C 1-C 4 ou fluoralkyle en C 1-C 4, ou bien |
| $R^5$ et $R^6$ | forment ensemble un groupe tri- ou tétraméthylène dans lequel un groupe méthylène peut être remplacé par l'oxygène ou le soufre, et qui est éventuellement substitué par un groupe alkyle en C 1-C 3, et |
| X | représente l'oxygène ou le soufre, |
| | sous réserve que (i) lorsque $R^5$ représente le fluor, $R^6$ représente exclusivement un groupe alkyle en C 1-C 4 ou fluoralkyle en C 1-C 4, et (ii) lorsque $R^5$ représente un groupe cyano, $R^6$ représente exclusivement l'hydrogène ou un groupe alkyle en C 1-C 4 et X exclusivement l'oxygène, |

et les sels des composés de formule I dans laquelle $R^1$ et/ou $R^2$ représentent l'hydrogène.

2. Composés de formule générale

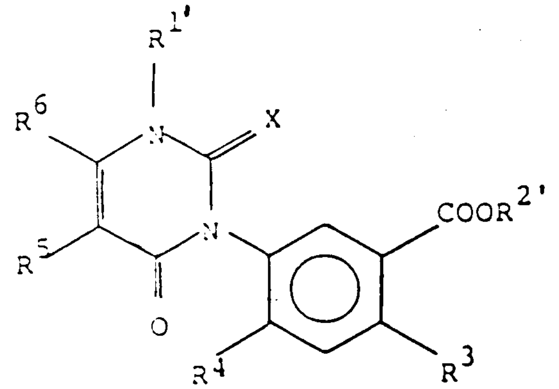

I'

dans laquelle

| | |
|---|---|
| $R^{1'}$ | représente un groupe alkyle en C 1-C 4, alcényle en C 2-C 4, alcynyle en C 2-C 4, alcoxyalkyle en C 2-C 6, formyle, alcanoyle en C 2-C 6 ou alcoxycarbonyle en C 2-C 6, |
| $R^{2'}$ | représente un groupe alkyle en C 1-C 6, alcényle en C 2-C 4, alcynyle en C 2-C 4 ou alcoxyalkyle en C 2-C 6, |
| $R^3$ | représente un halogène ou un groupe nitro, |
| $R^4$ | représente l'hydrogène ou un halogène, |
| $R^5$ | représente l'hydrogène, un halogène, un groupe alkyle en C 1-C 4, chlorométhyle, bromométhyle, hydroxymétnyle, (alcoxy en C 1-C 5)-méthyle, (alkylthio en C 1-C 5)-méthyle, cyano, nitro ou thiocyanato, |
| $R^6$ | représente l'hydrogène, un groupe alkyle en C 1-C 4 ou fluoralkyle en C 1-C 4, ou bien |
| $R^5$ et $R^6$ | forment ensemble un groupe tri- ou tétraméthylène dans lequel un groupe méthylène peut être remplacé par l'oxygène ou le soufre et qui est éventuellement substitué par un groupe alkyle en C 1-C 3, et |
| X | représente l'oxygène ou le soufre, sous réserve que (i) lorsque $R^5$ représente le fluor, $R^6$ représente exclusivement un groupe alkyle en C 1-C 4 ou fluoralkyle en C 1-C 4 et (ii) lorsque $R^5$ représente un groupe cyano, $R^6$ représente exclusivement l'hydrogène ou un groupe alkyle en C 1-C 4 et X exclusivement l'oxygène. |

**3.** Composés selon revendications 1 ou 2, dans lesquels $R^1$ et $R^{1'}$ représentent des groupes méthyle.

**4.** Composés selon l'une des revendications 1 à 3, dans lesquels $R^2$ et $R^{2'}$ représentent des groupes alkyle en C 1-C 6 ou alcoxyalkyle en C 2-C 6.

**5.** Composés selon l'une des revendications 1 à 4, dans lesquels $R^3$ représente le chlore ou le brome.

**6.** Composés selon l'une des revendications 1 à 5, dans lesquels $R^4$ représente le fluor.

**7.** Composés selon l'une des revendications 1 à 6, dans lesquels $R^5$ représente l'hydrogène, le chlore, le brome, un groupe méthyle ou éthyle.

**8.** Composés selon l'une des revendications 1 à 6, dans lesquels $R^5$ représente le fluor.

**9.** Composés selon l'une des revendications 1 à 7, dans lesquels $R^6$ représente un groupe méthyle, éthyle ou trifluorométhyle.

**10.** Composés selon l'une des revendications 1 à 6, dans lesquels $R^5$ et $R^6$ forment ensemble un groupe tri- ou tétra-méthylène.

**11.** Un composé selon le revendication 1 ou 2, choisi parmi les suivants :
le 2-chloro-4-fluoro-5-(1,2,4,5,6,7-hexahydro-1-méthyl-2,4-dioxo-3H-cyclopenta[d]pyrimidine-3-yl)-benzoate d'isopropyle,

le 2-chloro-4-fluoro-5-[1,4,5,6,7,8-hexahydrol-méthyl-2,4-dioxo-3-(2H)-quinazolinyl]-benzoate d'isopropyle,
le 2-chloro-4-fluoro-5-[3,6-dihydro-3,4-diméthyl-2,6-dioxo-1(2H)-pyrimidinyl]-benzoate d'isopropyle,
le 2-chloro-4-fluoro-5-[5-bromo-3,6-dihydro-3,4-diméthyl-2,6-dioxo-1(2H)-pyrimidinyl]-benzoate d'isopropyle,
le 2-chloro-4-fluoro-5-[3,6-dihydro-3,4-diméthyl-5-iodo-2,6-dioxo-1(2H)-pyrimidinyl]-benzoate d'isopropyle,
le 2-chloro-4-fluoro-5-[3,6-dihydro-3,4-diméthyl-5-hydroxyméthyl-2,6-dioxo-1(2H)-pyrimidinyl]-benzoate d'isopropyle,
le 2-chloro-4-fluoro-5-[4-éthyl-3,6-dihydro-3-méthyl-2,6-dioxo-1(2H)-pyrimidinyl]-benzoate d'isopropyle,
le 2-chloro-4-fluoro-5-[3,6-dihydro-3-méthyl-4-trifluorométhyl-2,6-dioxo-1(2H)-pyrimidinyl]-benzoate d'isopropyle,
le 2-bromo-4-fluoro-5-(1,2,4,5₁6,7-hexahydro-1-méthyl-2-4-dioxo-3H-cyclopenta[d]pyrimidine-3yl)-benzoate d'isopropyle,
le 2-chloro-4-fluoro-5-[3,6-dihydro-3,4-diméthyl-5-nitro-2,6-dioxo-1(2H)-pyrimidinyl]benzoate d'isopropyle,
le 2-chloro-4-fluoro-5-(1,2,4,5,6,7-hexahydro-1-méthyl-2,4-dioxo-3H-cyclopenta[d]pyrimidine-3-yl)-benzoate de 2-méthoxy-1-méthylène, et
le 2-chloro-4-fluoro-5-(1,2,4,5,6,7-hexahydro-1-méthyl-2,4-dioxo-3H-cyclopenta[d]pyrimidine-3-yl)-benzoate de tert-butyle.

**12.** Un composé selon la revendication 1 ou 2, choisi parmi les suivants :
le 2-chloro-4-fluoro-5-[3,6-dihydro-3,4-diméthyl-5-fluoro-2,6-dioxo-1-(2H)-pyrimidinyl]benzoate d'isopropyle,
le 2-chloro-4-fluoro-5-[3,6-dihydro-3-méthyl-4-propyle-2,6-dioxo-1(2H)-pyrimidinyl]-benzoate d'isopropyle et
le 2-chloro-4-fluoro-5-[3,6-dihydro-5-fluoro-3-méthyl-4-trifluorométhyl-2,6-dioxo-1(2H)-pyrimidinyl]-benzoate d'isopropyle.

**13.** Un composé selon la revendication 1 ou 2, choisi parmi les suivants :
le 2-chloro-5-(1,2,4,5,6,7-hexahydro-2,4-dioxo-3H-cyclopenta[d]pyrimidine-3-yl)-benzoate d'éthyle,
le 2-chloro-4-fluoro-5-(1,2,4,5,6,7-hexahydro-2,4-dioxo-3H-cyclopenta[d]pyrimidine-3-yl)-benzoate d'isopropyle,
le 2-chloro-4-fluoro-5-[1,4,5,6,7,8-hexahydro-2,4-dioxo-3(2H)-quinazolinyl]-benzoate d'isopropyle,
le 2-chloro-4-fluoro-5-[3,6-dihydro-4-méthyl-2,6-dioxo-1(2H)-pyrimidinyl]-benzoate d'isopropyle,
le 2-chloro-4-fluoro-5-[3,6-dihydro-5-méthyl-2,6-dioxo-1(2H)-pyrimidinyl]-benzoate d'isopropyle,
le 2-bromo-4-fluoro-5-(1,2,4,5,6,7-hexahydro-2,4-dioxo-3H-cyclopenta[d]pyrimidine-3-yl)-benzoate d'isopropyle,
le 2-chloro-4-fluoro-5-{1,2,5,7-tétrahydro-2,4-dioxo-thiéno[3,4-d]pyrimidine-3(4H)-yl}-benzoate d'isopropyle,
le 2-chloro-4-fluoro-5-[4-éthyl-3,6-dihydro-5-méthyl-2,6-dioxo-1(2H)-pyrimidinyl]-benzoate d'isopropyle,
le 2-chloro-5-(1,2,4,5,6,7-hexahydro-1-méthyl-2,4-dioxo-3H-cyclopenta[d]pyrimidine-3-yl)-benzoate d'éthyle,
le 2-chloro-5-[1,4,5,6,7,8-hexahydro-1-méthyl-2,4-dioxo-3(2H)-quinazolinyl]-benzoate d'éthyle,
le 2-chloro-4-fluoro-5-[4-éthyl-3,6-dihydro-2,6-dioxo-1(2H)-pyrimidinyl]-benzoate d'éthyle,
le 2-chloro-5-[3,6-dihydro-3,4-diméthyl-2,6-dioxo-1(2H)-pyrimidinyl]-benzoate d'éthyle,
le 2-chloro-5-[3,6-dihydro-3,4,5-triméthyl-2,6-dioxo-1(2H)-pyrimidinyl]-benzoate d'éthyle,
le 2-chloro-4-fluoro-5-[3,6-dihydro-3,4,5-tri-méthyl-2,6-dioxo-1(2H)-pyrimidinyl]-benzoate d'isopropyle,
le 2-chloro-4-fluoro-5-{1,2,5,7-tétrahydro-1-méthyl-2,4-dioxo-thiéno[3,4-d]pyrimidine-3(4H)-yl}-benzoate d'isopropyle,
le 2-chloro-4-fluoro-5-[4-éthyl-3,6-dihydro-3,5-diméthyl-2,6-dioxo-1(2H)-pyrimidinyl]-benzoate d'isopropyle,
le 2-chloro-4-fluoro-5-[5-cyano-3,6-dihydro-3,4-diméthyl-2,6-dioxo-1(2H)-pyrimidinyl]-benzoate d'isopropyle,
le 2,4-dichloro-5-(1,2,4,5,6,7-hexahydro-1-méthyl-2,4-dioxo-3H-cyclopenta[d]pyrimidine-3-yl)-benzoate d'isopropyle,
le 2-bromo-4-chloro-5-(1,2,4,5,6,7-hexahydro-1-méthyl-2,4-dioxo-3H-cyclopenta[d]pyrimidine-3-yl)-benzoate d'isopropyle,
le 2-chloro-4-fluoro-5-(1,2,4,5,6,7-hexahydro-1-acétyl-2,4-dioxo-3H-cyclopenta[d]pyrimidine-3-yl)-benzoate d'isopropyle,
le 2-chloro-4-fluoro-5-(1,2,4,5,6,7-hexahydro-1-méthoxycarbonyl-2,4-dioxo-3H-cyclopenta[d]-pyrimidine-3-yl)-benzoate d'isopropyle,
le 2-chloro-4-fluoro-5-[5-chloro-3,6-dihydro-3,4-diméthyl-2,6-dioxo-1(2H)-pyrimidinyl]-benzoate d'isopropyle,
le 2-chloro-4-fluoro-5-[5-bromo-3,6-dihydro-4-méthyl-2,6-dioxol-1(2H)-pyrimidinyl]-benzoate d'isopropyle,
le 2-chloro-5-(1,2,4,5,6,7-hexahydro-1-méthyl-2,4-dioxo-3H-cyclopenta[d]pyrimidine-3-yl)-benzoate de tert-butyle,
le 2-chloro-5-[1,4,5,6,7,8-hexahydro-1-méthyl-2,4-dioxo-3(2H)-quinazolinyl]-benzoate d'isopropyle,
le 2-chloro-5-[1,4,5,6,7,8-hexahydro-2,4-dioxo-3(2H)-quinazolinyl]-benzoate d'isopropyle,
le 2-chloro-4-fluoro-5-(1,2,4,5,6,7-hexahydro-1-méthyl-2,4-dioxo-3H-cyclopenta[d]pyrimidine-3-yl)-benzoate de 2-propényle,
le 2-chloro-4-fluoro-5-(1,2,4,5,6,7-hexahydro-1-méthyl-2,4-dioxo-3H-cyclopenta[d]pyrimidine-3-yl)-benzoate de 2-propynyle et
le 2-chloro-4-fluoro-5-(1,2,4,5,6,7-hexahydro-1-méthyl-2,4-dioxo-3H-cyclopenta[d]pyrimidine-3-yl)-benzoate de méthyle.

**14.** Un composé selon la revendication 1 ou 2, choisi parmi les suivants :
le 2-chloro-4-fluoro-5-[3,6-dihydro-5-fluoro-4-méthyl-2,6-dioxo-1-(2H)-pyrimidinyl]-benzoate d'isopropyle,
le 2-chloro-4-fluoro-5-[3,6-dihydro-5-fluoro-4-fluorométhyl-2,6-dioxo-1-(2H)-pyrimidinyl]-benzoate d'isopropyle,
le 2-chloro-4-fluoro-5-[3,6-dihydro-5-fluoro-4-fluorométhyl-3-méthyl-2,6-dioxo-1(2H)-pyrimidinyl]-benzoate d'isopropyle,
le 2-chloro-4-fluoro-5-[3,6-dihydro-4-propyl-2,6-dioxo-1(2H)pyrimidinyl]-benzoate d'isopropyle,
le 2-chloro-4-fluoro-5-[5-chloro-3,6-dihydro-4-méthyl-2,6-dioxo-1(2H)-pyrimidinyl]-benzoate d'isopropyle et
le 2-chloro-4-fluoro-5-[3,6-dihydro-3,4-diméthyl-5-méthoxyméthyl-2,6-dioxo-1(2H)-pyrimidinyl]-benzoate d'isopropyle.

**15.** Composés de formule générale

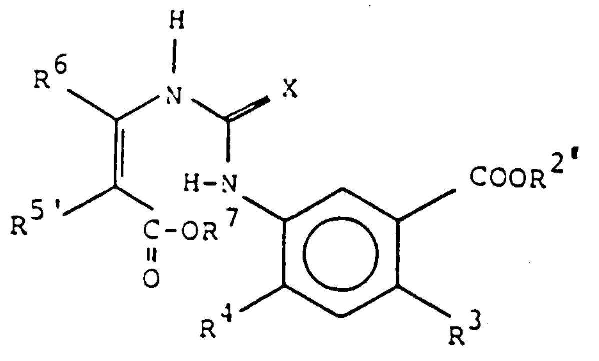

II

dans laquelle

| | |
|---|---|
| $R^{2'}$ | représente un groupe alkyle en C 1-C 6, alcényle en C 2-C 4, alcynyle en C 2-C 4 ou alcoxyalkyle en C 2-C 6, |
| $R^3$ | représente un halogène ou un groupe nitro, |
| $R^4$ | représente l'hydrogène ou un halogène, |
| $R^{5'}$ | représente l'hydrogène, le fluor ou un groupe alkyle en C 1-C 4, |
| $R^6$ | représente l'hydrogène, un groupe alkyle en C 1-C 4 ou fluoralkyle en C 1-C 4, ou bien |
| $R^{5'}$ et $R^6$ | forment ensemble un groupe tri- ou tétraméthylène dans lequel un groupe méthylène peut être remplacé par l'oxygène ou le soufre et qui est éventuellement substitué par |

un groupe alkyle en C 1-C 3,
$R^7$ représente un groupe alkyle en C 1-C 4, et
X représente l'oxygène ou le soufre,
sous réserve que lorsque $R^{5'}$ représente le fluor, $R^6$ représente exclusivement un groupe alkyle en C 1-C 4 ou fluoralkyle en C 1-C 4.

**16.** Le 2-chloro-4-fluoro-5-{3-[2-(éthoxycarbonyl)-1-cyclohexène-1-yl]-uréido}-benzoate d'isopropyle.

**17.** Le 2-chloro-4-fluoro-5-{3-[2-(éthoxycarbonyl)-1-méthylpropényl]-uréido}-benzoate d'isopropyle.

**18.** Produit herbicide caractérisé en ce qu'il contient une quantité efficace d'au moins un composé de formule générale I' selon revendication 2, avec des produits auxiliaires de formulation.

**19.** Produit herbicide selon la revendication 18, caractérisé en ce qu'il contient une quantité efficace d'au moins un des composés mentionnés dans la revendication 11 avec des produits auxiliaires de formulation.

**20.** Produit herbicice selon la revendication 18, caractérisé en ce qu'il contient une quantité efficace d'au moins un des composés mentionnés dans la revendication 12, avec des produits auxiliaires de formulation.

**21.** Procédé de préparation des composés de formule générale I de la revendication 1, et des sels des composés de formule I dans laquelle $R^1$ et/ou $R^2$ représentent l'hydrogène, caractérisé en ce que
a) pour la préparation des composés de formule I dans laquelle $R^1$ représente l'hydrogène et $R^2$ un groupe alkyle en C 1-C 6, alcényle en C 2-C 4, alcynyle en C 2-C 4 ou alcoxyalkyle en C 2-C 6 et $R^5$ a une signification autre que le chlore, le brome, l'iode, un groupe chlorométhyle, bromométhyle, hydroxyméthyle, (alcoxy en C 1-C 5)-méthyle, (alkylthio en C 1-C 5)-méthyle, cyano, nitro ou thiocyanato, et des sels métalliques de ces composés de formule I dans laquelle $R^1$ représente l'hydrogène, on soumet un composé de formule générale

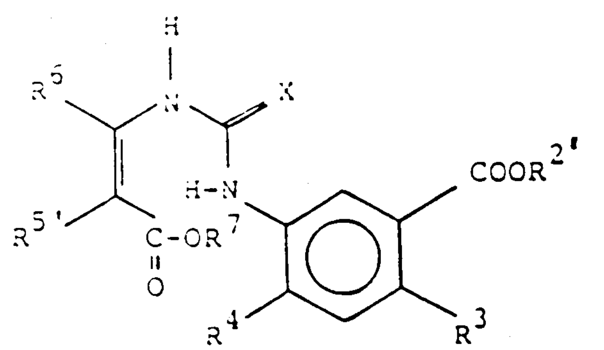

II

dans laquelle
$R^{2'}$ représente un groupe alkyle en C 1-C 6, alcényle en C 2-C 4, alcynyle en C 2-C 4 ou alcoxyalkyle en C 2-C 6,
$R^3$, $R^4$, $R^6$, et X ont les significations indiquées ci-dessus,
$R^{5'}$ représente l'hydrogène, le fluor, un groupe alkyle en C 1-C 4 ou forme avec $R^6$ un groupe tri- ou tétraméthylène éventuellement modifié comme indiqué cidessus, et
$R^7$ représente un groupe alkyle inférieur,
à une cyclisation catalysée par une base, par traitement à l'aide d'une base, et si on le désire, on convertit un tel dérivé d'uracile éventuellement obtenu à l'état de sel métallique en la forme acide ($R^1$ = hydrogène) par traitement à l'aide d'un acide,
b) pour la préparation des composés de formule I dans laquelle $R^1$ représente un groupe alkyle en C 1-C 4, alcényle en C 2-C 4, alcynyle en C 2-C 4, alcoxyalkyle en C 2-C 6, formyle, alcanoyle en C 2-C 6 ou alcoxycarbonyle en C 2-C 6, on traite un dérivé d'uracile de formule générale

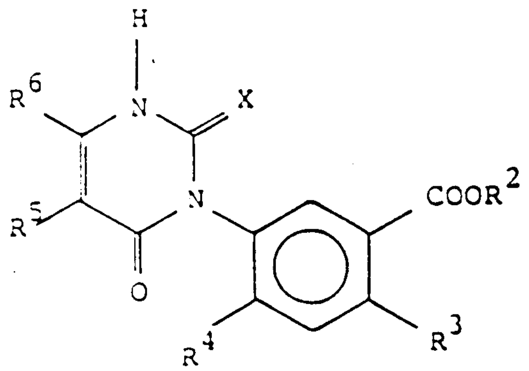

Ia

dans laquelle $R^2$, $R^3$, $R^4$, $R^5$, $R^6$, et X ont les significations indiquées ci-dessus, pour alkylation ou acylation respectivement, par un agent alkylant ou acylant respectivement contenant un groupe alkyle en C 1-C 4, alcényle en C 2-C 4, alcynyle en C 2-C 4, alcoxyalkyle en C 2-C 6, formyle, alcanoyle en C 2-C 6 ou alcoxycarbonyle en C 2-C 6,

c) pour la préparation des composés de formule I dans laquelle $R^1$ représente un groupe alkyle en C 1-C 4, alcényle en C 2-C 4, alcynyle en C 2-C 4 ou alcoxyalkyle en C 2-C 6, $R^5$ a une signification autre que le chlore, le brome, l'iode, un groupe chlorométhyle, bromométhyle, hydroxyméthyle, (alcoxy en C 1-C 5)-méthyle, (alkylthio en C 1-C 5)-méthyle, cyano, nitro ou thiocyanato, et X représente le soufre, on fait réagir un composé de formule générale

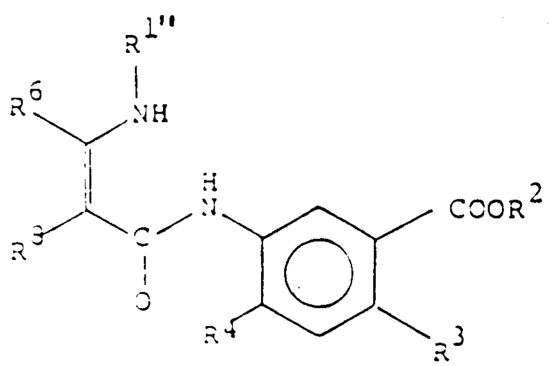

III

dans laquelle

| | |
|---|---|
| $R^{1''}$ | représente un groupe alkyle en C 1-C 4, alcényle en C 2-C 4, alcynyle en C 2-C 4 ou alcoxyalkyle en C 2-C 6, et |
| $R^2$, $R^3$, $R^4$ et $R^6$ | ont les significations indiquées cidessus, et |
| $R^8$ | représente le fluor, un groupe alkyle en C 1-C 4, (alcoxy en C 1-C 4)-carbonyle ou forme avec $R^6$ un groupe tri- ou tétra-méthylène tel que défini cidessus, sous réserve que dans le cas où $R^8$ représente le fluor, $R^6$ représente un groupe alkyle en C 1-C 4 ou fluoralkyle en C 1-C 4, |

avec le N,N'-thiocarbonyl-diimidazolide ou le thiophosgène et, lorsque $R^8$ représente un groupe (alcoxy en C 1-C 4)-carbonyle, on soumet le 5-alcoxycarbonyl-2-thiouracile ainsi obtenu, de formule générale

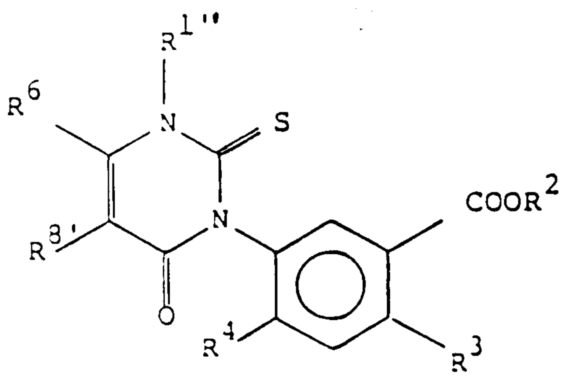

IV

dans laquelle $R^{1''}$, $R^2$, $R^3$, $R^4$ et $R^6$ ont les significations indiquées ci-dessus et $R^{8'}$ représente un groupe (alcoxy en C 1-C 4)-carbonyle, à hydrolyse et décarboxylation par chauffage en présence d'acide chlorhydrique aqueux ou d'acide trifluoracétique,

d) pour la préparation des composés de formule I dans laquelle $R^5$ représente le chlore, le brome ou l'iode, on traite un dérivé d'uracile de formule générale

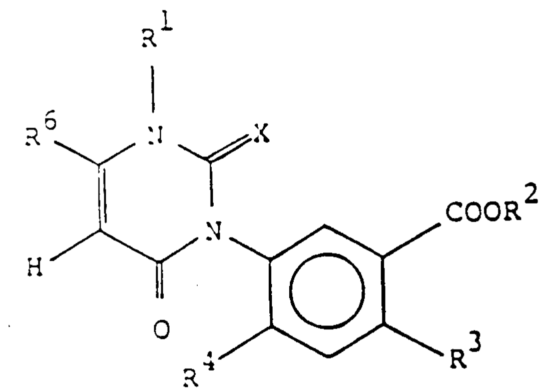

Ib

dans laquelle $R^1$, $R^2$, $R^3$, $R^4$, $R^6$ et X ont les significations indiquées ci-dessus, pour chloruration par le chlore élémentaire ou le chlorure de sulfuryle, pour bromuration par le brome élémentaire ou le bromure de sulfuryle ou pour ioduration par l'iode élémentaire,

e) pour la préparation des composés de formule I dans laquelle $R^5$ représente un groupe chloro- ou bromométhyle, (i) on traite un dérivé d'uracile de formule Ib ci-dessus par le chloro- ou respectivement le bromométhoxyméthane, ou bien (ii) on traite un 5-hydroxyméthyluracile de formule générale

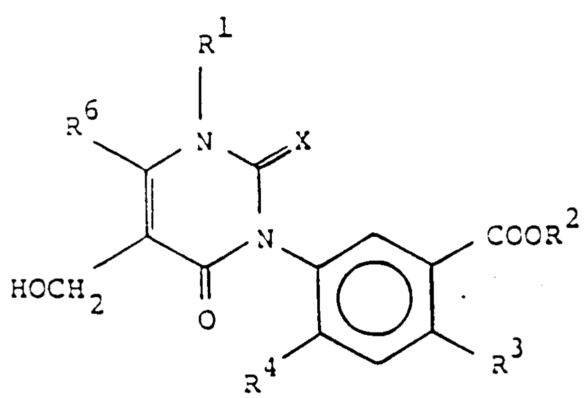

Ic

dans laquelle $R^2$, $R^3$, $R^4$, $R^6$ et X ont les significations indiquées ci-dessus, par le chlorure ou le bromure de thionyle respectivement, ou bien (iii) on traite un 5-méthyluracile de formule générale

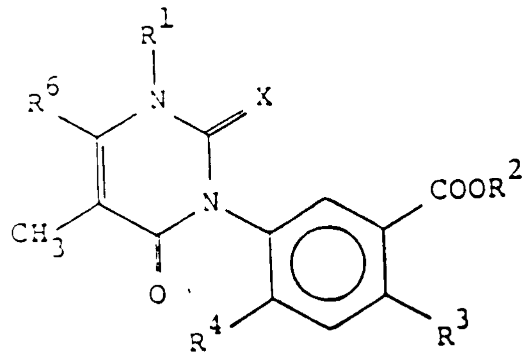

Id

dans laquelle $R^1$, $R^2$, $R^3$ $R^4$ $R^6$ et X ont les significations indiquées ci-dessus, par le N-chloro-succinimide ou le N-bromosuccinimide respectivement,

f) pour la préparation des composés de formule I dans laquelle $R^5$ représente un groupe hydroxy-méthyle, on traite un 5-halogénométhyluracile de formule générale

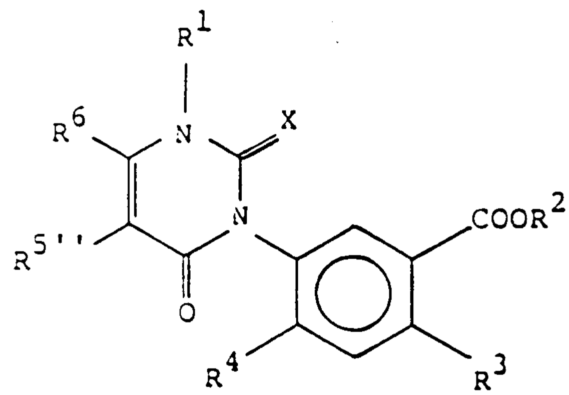

Ie

dans laquelle $R^1$, $R^2$, $R^3$ $R^4$ $R^6$ et X ont les significations indiquées ci-dessus et $R^{5''}$ représente un groupe chloro-, bromo- ou iodo-méthyle,

pour hydrolyse par une solution aqueuse d'une base minerale,

g, pour la préparation des composés de formule I dans laquelle $R^5$ représente un groupe (alcoxy en C 1-C 5)-méthyle ou (alkylthio en C 1-C 5)-méthyle, on traite un 5-halogénométhyluracile de formule le ci-dessus dans laquelle $R^{5''}$ représente un groupe chloro-, ou bromométhyle, par un alcoolate ou thioalcoolate de métal alcalin de formule générale

$R^9M$ V

dans laquelle

- $R^9$ représente un groupe alcoxy en C 1-C 5 ou alkylthio en C 1-C 5 et
- M représente un métal alcalin,

ou par un alcanol en C 1-C 5 ou un alkylmercaptan en C 1-C 5,

h) pour la préparation des composés de formule I dans laquelle $R^5$ représente un groupe (alkylthio en C 1-C 5)-méthyle, on traite un 5-hydroxyméthyluracile de formule Ic ci-dessus par un alkylmercaptan en C 1-C 5,

i) pour la préparation des composés de formule I dans laquelle $R^1$ représente l'hydrogène, $R^5$ un groupe cyano, $R^6$ l'hydrogène ou un groupe alkyle en C 1-C 4 et X l'oxygène, on traite un composé de formule générale

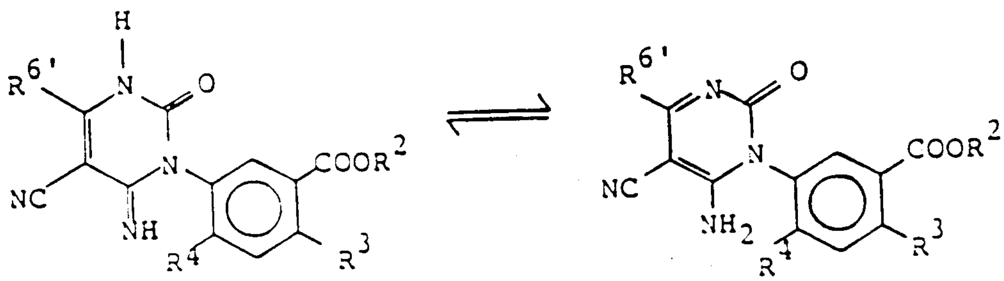

VI

dans laquelle $R^2$, $R^3$ et $R^4$ ont les significations indiquées ci-dessus et $R^{6'}$ représente l'hydrogène ou un groupe alkyle en C 1-C 4,

pour hydrolyse catalysée par un acide, à l'aide d'une solution aqueuse d'un acide minéral,

j) pour la préparation des composés de formule I dans laquelle $R^5$ représente un groupe nitro, on traite un dérivé d'uracile de formule Ib ci-dessus par l'acide nitrique,

k) pour la préparation des composés de formule I dans laquelle $R^5$ représente un groupe thiocyanato, on traite un dérivé d'uracile de formule Ib ci-dessus par le thiocyanogène,

l) pour la préparation des composés de formule I dans laquelle $R^2$ représente l'hydrogène, on hydrolyse un ester benzoïque de formule générale

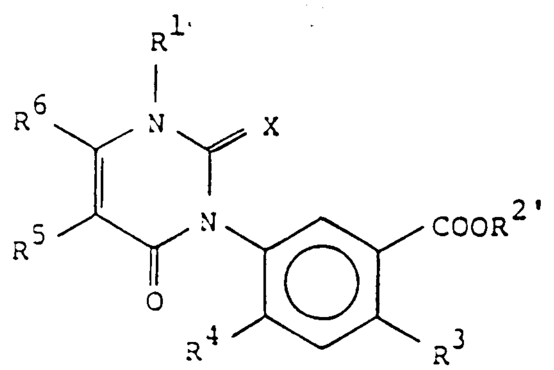

If

dans laquelle $R^1$, $R^{2'}$, $R^3$, $R^4$, $R^5$, $R^6$ et X ont les significations indiquées ci-dessus,

en l'acide benzoïque correspondant par traitement à l'aide d'une solution aqueuse d'un solvant organique et d'une base minérale,

m) pour la préparation des composés de formule I dans laquelle $R^2$ représente un groupe alkyle en C 1-C 6, alcényle en C 2-C 4, alcynyle en C 2-C 4 ou alcoxyalkyle en C 2-C 6,

on estérifie un acide benzoïque de formule générale

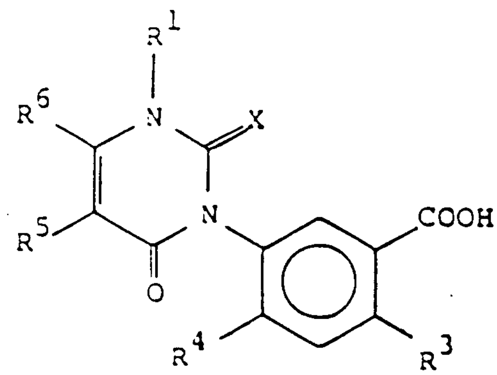

Ig

dans laquelle $R^1$, $R^3$, $R^4$, $R^5$, $R^6$ et X ont les significations indiquées ci-dessus,
ou un dérivé réactif d'un tel acide, c'est-à-dire que, lorsqu'on utilise un sel de l'acide benzoïque Ig en tant que dérivé réactif, on fait réagir ce sel avec un chlorure, un bromure, un iodure, un sulfate, un méthylsulfate ou toluène-sulfonate d'alkyle en C 1-C 6, d'alcényle en C 2-C 4, d'alcynyle en C 2-C 4 ou d'alcoxyalkyle en C 2-C 6 et, dans le cas où on utilise un halogénure de l'acide ou une O-acyl-1,3-dicyclohexylisourée correspondante, un N-acylimidazole correspondant ou un anhydride de l'acide benzoïque Ig en tant que dérivé réactif, on fait réagir celui-ci ave un alcanol en C 1-C 6, un alcénol en C 2-C 4, un alcynol en C 2-C 4 ou un alcoxyalcanol en C 2-C 6, ou bien
n) pour la préparation des composés de formule I dans laquelle $R^2$ représente un groupe alkyle en C 2-C 6, alcényle en C 2-C 4, alcynyle en C 2-C 4 ou alcoxyalkyle en C 2-C 6, on soumet un ester benzoïque de formule If ci-dessus à une réaction de transestérification avec un alcanol, un alcénol ou respectivement un alcynol de formule générale

$R^{2''}OH$ VII

dans laquelle $R^{2''}$ représente un groupe alkyle en C 2-C 6, alcényle en C 2-C 4, alcynyle en C 2-C 4 ou alcoxyalkyle en C 2-C 6,
sous réserve que le réactif de formule VII bouille plus haut que l'alcanol, l'alcénol ou l'alcynol $R^{2'}OH$, et si on le désire, on convertit un composé ainsi obtenu, répondant à la formule I dans laquelle $R^1$ et/ou $R^2$ représentent l'hydrogène, en un sel.

**22.** Procédé selon la revendication 21, caractérisé en ce que l'on met en oeuvre l'une des variantes a), b), d), e), g), h), i), j), k), l), m) et n) et, le cas échéant, la conversion facultative en un sel.

**23.** Procédé pour combattre les végétaux adventices, caractérisé en ce que l'on traite les endroits à protéger contre les végétaux adventices et/ou les végétaux adventices par une quantité efficace d'un composé selon l'une des revendications 2 à 7, 9 à 11 et 13 ou par un produit selon l'une des revendications 18 et 19.

**24.** Procédé pour combattre les végétaux adventices, caractérisé en ce que l'on traite les endroits à protéger contre les végétaux adventices et/ou les végétaux adventices par une quantité efficace d'un composé selon l'une des revendications 8, 12 et 14 ou par un produit selon revendication 20.

**25.** Utilisation d'un composé selon l'une des revendications 2 à 7, 9 à 11 et 13 ou d'un produit selon l'une des revendications 18 et 19 pour la lutte contre les végétaux adventices.

**26.** Utilisation d'un composé selon l'une des revendications 8, 12 et 14 ou d'un produit selon revendication 20 pour la lutte contre les végétaux adventices.

**Revendications pour l'Etat contractant suivant : AT**

**1.** Produit herbicide caractérisé en ce qu'il contient une quantité efficace d'au moins un composé de formule générale

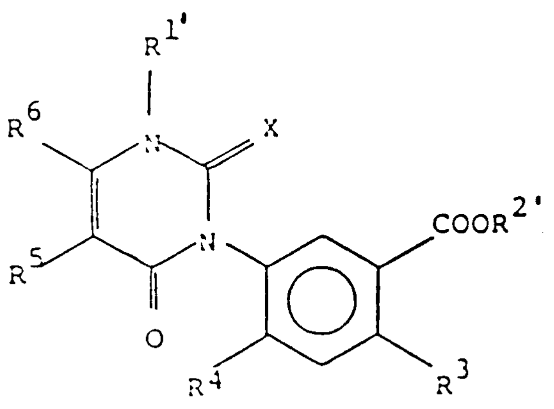

I'

dans laquelle

$R^{1'}$ représente un groupe alkyle en C 1-C 4, alcényle en C 2-C 4, alcynyle en C 2-C 4, alcoxyalkyle en C 2-C 6, formyle, alcanoyle en C 2-C 6 ou alcoxycarbonyle en C 2-C 6,

$R^{2'}$ représente un groupe alkyle en C 1-C 6, alcényle en C 2-C 4, alcynyle en C 2-C 4 ou alcoxyalkyle en C 2-C 6,

$R^3$ représente un halogène ou un groupe nitro,

$R^4$ représente l'hydrogène ou un halogène,

$R^5$ représente l'hydrogène, un halogène, un groupe alkyle en C 1-C 4, chlorométhyle, bromométhyle, hydroxyméthyle, (alcoxy en C 1-C 5)-méthyle, (alkylthio en C 1-C 5)-méthyle, cyano, nitro ou thiocyanato,

$R^6$ représente l'hydrogène, un groupe alkyle en C 1-C 4 ou fluoralkyle en C 1-C 4, ou bien

$R^5$ et $R^6$ forment ensemble un groupe tri- ou tétraméthylène dans lequel un groupe méthylène peut être remplacé par l'oxygène ou le soufre et qui est éventuellement substitué par un groupe alkyle en C 1-C 3,

et

X représente l'oxygène ou le soufre,

sous réserve que (i) lorsque $R^5$ représente le fluor, $R^6$ représente exclusivement un groupe alkyle en C 1-C 4 ou fluoralkyle en C 1-C 4 et (ii) lorsque $R^5$ représente un groupe cyano, $R^6$ représente exclusivement l'hydrogène ou un groupe alkyle en C 1-C 4 et X exclusivement l'oxygène,

avec des produits auxiliaires de formulation.

**2.** Produit herbicide selon revendication 1, pour lequel $R^{1'}$ de la formule I' représente un groupe méthyle.

**3.** Produit herbicide selon revendication 1 ou 2, pour lequel $R^{2'}$ de la formule I' représente un groupe alkyle en C 1-C 6 ou alcoxyalkyle en C 2-C 6.

**4.** Produit herbicide selon l'une des revendications 1 à 3, pour lequel $R^3$ de la formule I' représente le chlore ou le brome.

**5.** Produit herbicide selon l'une des revendications 1 à 4, pour lequel $R^4$ de la formule I' représente le fluor.

**6.** Produit herbicide selon l'une des revendications 1 à 5, pour lequel $R^5$ de la formule I' représente l'hydrogène, le chlore, le brome, un groupe méthyle ou éthyle.

**7.** Produit herbicide selon l'une des revendications 1 à 6, pour lequel $R^5$ de la formule I' représente le fluor.

**8.** produit herbicide selon l'une des revendications 1 à 6, pour lequel $R^6$ de la formule I' représente un groupe méthyle, éthyle ou trifluorométhyle.

**9.** Produit herbicide selon l'une des revendications 1 à 5, pour lequel $R^5$ et $R^6$ de la formule I' forment ensemble un groupe tri- ou tétra-méthylène.

**10.** Produit herbicide selon revendication 1, caractérisé en ce qu'il contient une quantité efficace d'au moins un composé pris dans le groupe suivant :

le 2-chloro-4-fluoro-5-(1,2,4,5,6,7-hexahydro-1-méthyl-2,4-dioxo-3H-cyclopenta[d]pyrimidine-3-yl)-benzoate d'isopropyle,

le 2-chloro-4-fluoro-5-[1,4,5,6,7,8-hexahydro-1-méthyl-2,4-dioxo-3(2H)-quinazolinyl]-benzoate d'isopropyle,

le 2-chloro-4-fluoro-5-[3,6-dihydro-3,4-diméthyl-2,6-dioxo-1(2H)-pyrimidinyl]-benzoate d'isopropyle,

le 2-chloro-4-fluoro-5-[5-bromo-3,6-dihydro-3,4-diméthyl-2,6-dioxo-1(2H)-pyrimidinyl]-benzoate d'isopropyle,

le 2-chloro-4-fluoro-5-[3,6-dihydro-3,4-diméthyl-5-iodo-2,6-dioxo-1(2H)-pyrimidinyl]-benzoate d'isopropyle,

le 2-chloro-4-fluoro-5-[3,6-dihydro-3,4-diméthyl-5-hydroxyméthyl-2,6-dioxo-1(2H)-pyrimidinyl]-benzoate d'isopropyle,

le 2-chloro-4-fluoro-5-[4-éthyl-3,6-dihydro-3-méthyl-2,6-dioxo-1(2H)-pyrimidinyl]-benzoate d'isopropyle,
le 2-chloro-4-fluoro-5-[3,6-dihydro-3-méthyl-4-trifluorométhyl-2,6-dioxo-1(2H)-pyrimidinyl]-benzoate d'isopropyle,
le 2-bromo-4-fluoro-5-(1,2,4,5,6,7-hexahydro-1-méthyl-2,4-dioxo-3H-cyclopenta[d]pyrimidine-3-yl)-benzoate d'isopropyle,
le 2-chloro-4-fluoro-5-[3,6-dihydro-3,4-diméthyl-5-nitro-2,6-dioxo-1(2H)-pyrimidinyl]-benzoate d'isopropyle,
le 2-chloro-4-fluoro-5-(1,2,4,5,6,7-hexahydro-1-méthyl-2,4-dioxo-3H-cyclopenta[d]pyrimidine-3-yl)-benzoate de 2-méthoxy-1-méthylène et
le 2-chloro-4-fluoro-5-(1,2,4,5,6,7-hexahydro-1-méthyl-2,4-dioxo-3H-cyclopenta[d]pyrimidine-3-yl)-benzoate de tert-butyle,
avec des produits auxiliaires de formulation.

**11.** Produit herbicide selon revendication 1, caractérisé en ce qu'il contient une quantité efficace d'au moins un composé pris dans le groupe suivant :
le 2-chloro-4-fluoro-5-[3,6-dihydro-3,4-diméthyl-5-fluoro-2,6-dioxo-1(2H)-pyrimidinyl]-benzoate d'isopropyle,
le 2-chloro-4-fluoro-5-[3,6-dihydro-3-méthyl-4-propyl-2,6-dioxo-1(2H)-pyrimidinyl]-benzoate d'isopropyle et
le 2-chloro-4-fluoro-5-[3,6-dihydro-5-fluoro-3-méthyl-4-trifluorométhyl-2,6-dioxo-1(2H)-pyrimidinyl]-benzoate d'isopropyle,
avec des produits auxiliaires de formulation.

**12.** Produit herbicide selon la revendication 1, caractérisé en ce qu'il contient une quantité efficace d'au moins un composé pris dans le groupe suivant :
le 2-chloro-5-(1,2,4,5,6,7-hexahydro-1-méthyl-2,4-dioxo-3H-cyclopenta[d]pyrimidine-3-yl)-benzoate d'éthyle,
le 2-chloro-5-[1,4,5,6,7,8-hexahydro-1-méthyl-2,4-dioxo-3(2H)-quinazolinyl]-benzoate d'éthyle,
le 2-chloro-5-[3,6-dihydro-3,4-diméthyl-2,6-dioxo-1(2H)-pyrimidinyl]-benzoate d'éthyle,
le 2-chloro-5-[3,6-dihydro-3,4,5-triméthyl-2,6-dioxo-1(2H)-pyrimidinyl]-benzoate d'éthyle,
le 2-chloro-4-fluoro-5-[3,6-dihydro-3,4,5-triméthyl-2,6-dioxo-1(2H)-pyrimidinyl]-benzoate d'isopropyle,
le 2-chloro-4-fluoro-5-{1,2,5,7-tétrahydro-1-méthyl-2,4-dioxo-thiéno[3,4-d]pyrimidine-3(4H)-yl}-benzoate d'isopropyle,
le 2-chloro-4-fluoro-5-[4-éthyl-3,6-dihydro-3,5-diméthyl-2,6-dioxo-1(2H)-pyrimidinyl]-benzoate d'isopropyle,
le 2-chloro-4-fluoro-5-[5-cyano-3,6-dihydro-3,4-diméthyl-2,6-dioxo-1(2H)-pyrimidinyl]-benzoate d'isopropyle,
le 2,4-dichloro-5-(1,2,4,5,6,7-hexahydro-1-méthyl-2,4-dioxo-3H-cyclopenta[d]pyrimidine-3-yl)-benzoate d'isopropyle,
le 2-bromo-4-chloro-5-(1,2,4,5,6,7-hexahydro-1-méthyl-2,4-dioxo-3H-cyclopenta[d]pyrimidine-3-yl)-benzoate d'isopropyle,
le 2-chloro-4-fluoro-5-(1,2,4,5,6,7-hexahydro-1-acétyl-2,4-dioxo-3H-cyclopenta[d]pyrimidine-3-yl)-benzoate d'isopropyle,
le 2-chloro-4-fluoro-5-(1,2,4,5,6,7-hexahydro-1-méthoxycarbonyl-2,4-dioxo-3H-cyclopenta[d]-pyrimidine-3-yl)-benzoate d'isopropyle,
le 2-chloro-4-fluoro-5-[5-chloro-3,6-dihydro-3,4-diméthyl-2,6-dioxo-1(2H)-pyrimidinyl]-benzoate d'isopropyle,
le 2-chloro-5-(1,2,4,5,6,7-hexahydro-1-méthyl-2,4-dioxo-3H-cyclopenta[d]pyrimidine-3-yl)-benzoate de tert-butyle,
le 2-chloro-5-[1,4,5,6,7,8-hexahydro-1-méthyl-2,4-dioxo-3(2H)-quinazolinyl]-benzoate d'isopropyle,
le 2-chloro-5-[1,4,5,6,7,8-hexahydro-2,4-dioxo-3(2H)-quinazolinyl]-benzoate d'isopropyle,
le 2-chloro-4-fluoro-5-(1,2,4,5,6,7-hexahydro-1-méthyl-2,4-dioxo-3H-cyclopenta[d]pyrimidine-3-yl)-benzoate de 2-propényle,
le 2-chloro-4-fluoro-5-(1,2,4,5,6,7-hexahydro-1-méthyl-2,4-dioxo-3H-cyclopenta[d]pyrimidine-3-yl)-benzoate de 2-propynyle et
le 2-chloro-4-fluoro-5-(1,2,4,5,6,7-hexahydro-1-méthyl-2,4-dioxo-3H-cyclopenta[d]pyrimidine-3-yl)-benzoate de méthyle,
avec des produits auxiliaires de formulation.

**13.** Produit herbicide selon la revendication 1, caractérisé en ce qu'il contient une quantité efficace de 2-chloro-4-fluoro-5-[3,6-dihydro-3,4-diméthyl-5-méthoxyméthyl-2,6-dioxo-1(2H)-pyrimidinyl]-benzoate d'isopropyle avec des produits auxiliaires de formulation.

**14.** Procédé de préparation des composés de formule générale

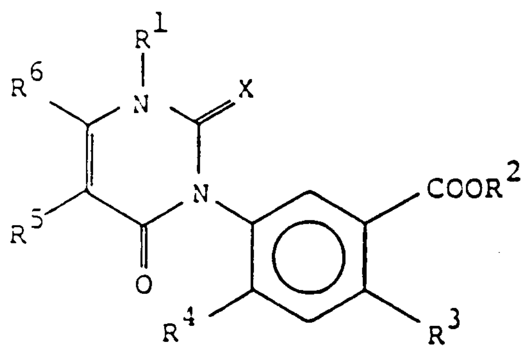

I

dans laquelle

| | |
|---|---|
| $R^1$ | représente l'hydrogène, un groupe alkyle en C 1-C 4, alcényle en C 2-C 4, alcynyle en C 2-C 4, alcoxyalkyle en C 2-C 6, formyle, alcanoyle en C 2-C 6 ou alcoxycarbonyle en C 2-C 6 et |
| $R^2$, $R^3$, $R^4$, $R^5$, $R^6$ et X | ont les significations indiquées dans la revendication 1, |

et des sels des composés de formule I dans laquelle $R^1$ et/ou $R^2$ représentent l'hydrogène,
caractérisé en ce que

a) pour la préparation des composés de formule I dans laquelle $R^1$ représente l'hydrogène et $R^2$ un groupe alkyle en C 1-C 6, alcényle en C 2-C 4, alcynyle en C 2-C 4 ou alcoxyalkyle en C 2-C 6 et $R^5$ a une signification autre que le chlore, le brome, l'iode, un groupe chlorométhyle, bromométhyle, hydroxyméthyle, (alcoxy en C 1-C 5)-méthyle, (alkylthio en C 1-C 5)-méthyle, cyano, nitro ou thiocyanato, et des sels métalliques de ces composés de formule I dans laquelle $R^1$ représente l'hydrogène, on soumet un composé de formule générale

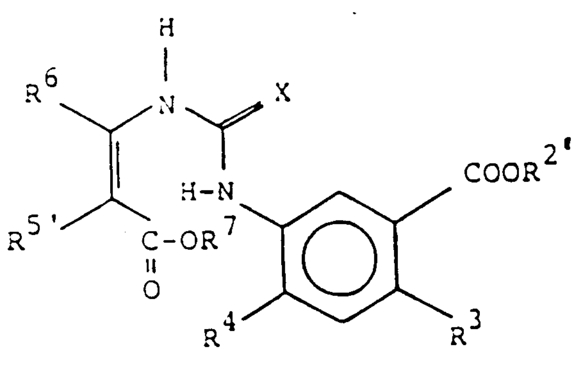

II

dans laquelle

| | |
|---|---|
| $R^{2'}$ | représente un groupe alkyle en C 1-C 6, alcényle en C 2-C 4, alcynyle en C 2-C 4 ou alcoxyalkyle en C 2-C 6, |
| $R^3$, $R^4$, $R^6$ et X | ont les significations indiquées dans la revendication 1, |
| $R^{5'}$ | représente l'hydrogène, le fluor, un groupe alkyle en C 1-C 4 ou forme avec $R^6$ un groupe tri- ou tétraméthylène éventuellement modifié comme indiqué ci-dessus, et |
| $R^7$ | représente un groupe alkyle inférieur, |

à une cyclisation catalysée par une base, par traitement à l'aide d'une base, et si on le désire, on

convertit un tel dérivé d'uracile éventuellement obtenu à l'état de sel métallique en la forme acide ($R^1$ = hydrogène) par traitement à l'aide d'un acide,
b) pour la préparation des composés de formule I dans laquelle $R^1$ représente un groupe alkyle en C 1-C 4, alcényle en C 2-C 4, alcynyle en C 2-C 4, alcoxyalkyle en C 2-C 6, formyle, alcanoyle en C 2-C 6 ou alcoxycarbonyle en C 2-C 6, on traite un dérivé d'uracile de formule générale

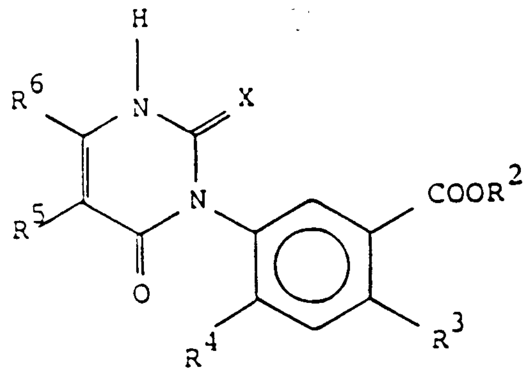

Ia

dans laquelle $R^2$, $R^3$, $R^4$, $R^5$, $R^6$ et X ont les significations indiquées dans la revendication 1, pour alkylation ou acylation respectivement, par un agent alkylant ou acylant respectivement contenant un groupe alkyle en C 1-C 4, alcényle en C 2-C 4, alcynyle en C 2-C 4, alcoxyalkyle en C 2-C 6, formyle, alcanoyle en C 2-C 6 ou alcoxycarbonyle en C 2-C 6,
c) pour la préparation des composés de formule I dans laquelle $R^1$ représente un groupe alkyle en C 1-C 4, alcényle en C 2-C 4, alcynyle en C 2-C 4 ou alcoxyalkyle en C 2-C 6, $R^5$ a une signification autre que le chlore, le brome, l'iode, un groupe chlorométhyle, bromométhyle, hydroxyméthyle, (alcoxy en C 1-C 5)-méthyle, (alkylthio en C 1-C 5)-méthyle, cyano, nitro ou cyanato, et X représente le soufre, on fait réagir un composé de formule générale

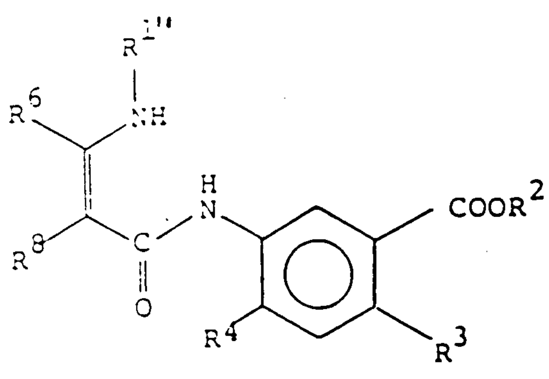

III

dans laquelle

| | |
|---|---|
| $R^{1''}$ | représente un groupe alkyle en C 1-C 4, alcényle en C 2-C 4, alcynyle en C 2-C 4 ou alcoxyalkyle en C 2-C 6, |
| $R^2$, $R^3$, $R^4$ et $R^6$ | ont les significations indiquées dans la revendication 1, et |
| $R^8$ | représente le fluor, un groupe alkyle en C 1-C 4, (alcoxy en C 1-C 4)-carbonyle ou forme avec $R^6$ un groupe tri- ou tétra-méthylène tel que défini dans la revendication 1, sous réserve que dans le cas où $R^8$ représente le fluor, $R^6$ représente un groupe alkyle en C 1-C 4 ou fluoralkyle en C 1-C 4, |

avec le N,N'-thiocarbonyl-diimidazolide ou le thiophosgène et, lorsque $R^8$ représente un groupe (alcoxy en C 1-C 4)-carbonyle, on soumet le 5-alcoxycarbonyl-2-thiouracile ainsi obtenu, de formule générale

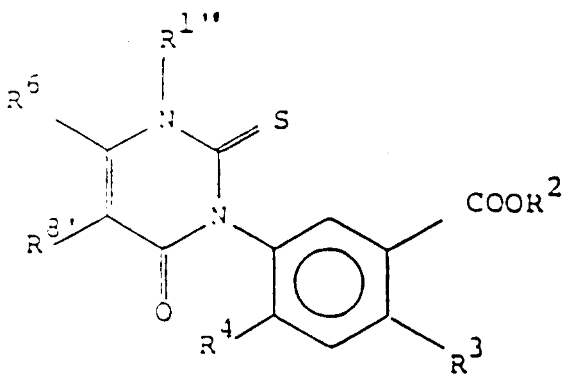

IV

dans laquelle $R^{1''}$, $R^2$, $R^3$, $R^4$ et $R^6$ ont les significations indiquées dans la revendication 1 et $R^{8'}$ représente un groupe (alcoxy en C 1-C 4)-carbonyle, à hydrolyse et décarboxylation par chauffage en présence d'acide chlorhydrique aqueux ou d'acide trifluoracétique,

d) pour la préparation des composés de formule I dans laquelle $R^5$ représente le chlore, le brome ou l'iode, on traite un dérivé d'uracile de formule générale

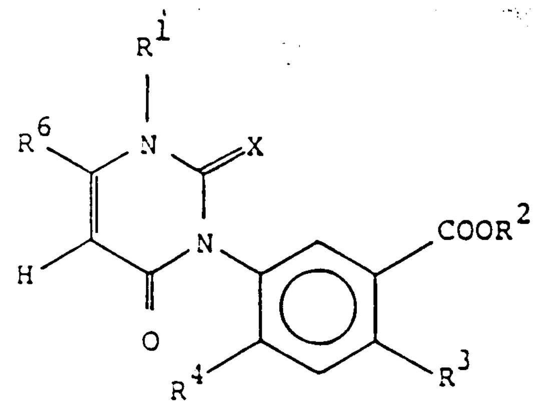

Ib

dans laquelle $R^1$, $R^2$, $R^3$, $R^4$, $R^6$ et X ont les significations indiquées dans la revendication 1, pour chloruration par le chlore élémentaire ou le chlorure de sulfuryle, pour bromuration par le brome élémentaire ou le bromure de sulfuryle ou pour ioduration par l'iode élémentaire,

e) pour la préparation des composés de formule I dans laquelle $R^5$ représente un groupe chloro- ou bromométhyle, (i) on traite un dérivé d'uracile de formule Ib ci-dessus par le chloro- ou respectivement le bromométhoxyméthane, ou bien (ii) on traite un 5-hydroxyméthyluracile de formule générale

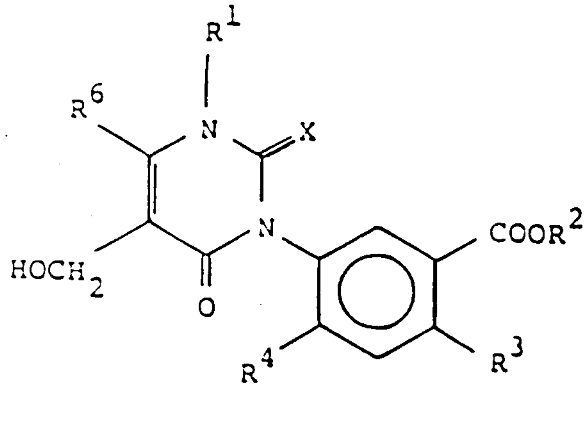

Ic

dans laquelle $R^2$, $R^3$, $R^4$, $R^6$ et X ont les significations indiquées dans la revendication 1, par le chlorure ou le bromure de thionyle respectivement, ou bien (iii) on traite un 5-méthyluracile de formule générale

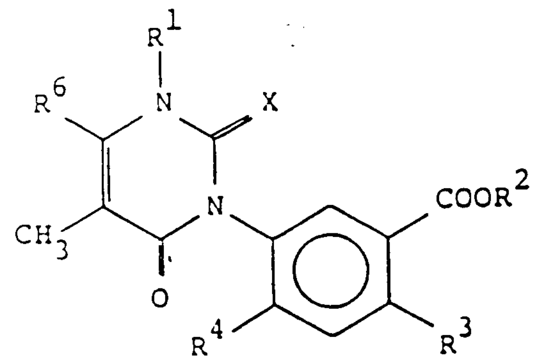

Id

dans laquelle $R^1$, $R^2$, $R^3$, $R^4$, $R^6$ et X ont les significations indiquées dans la revendication 1, par le N-chloro-succinimide ou le N-bromosuccinimide respectivement,

f) pour la préparation des composés de formule I dans laquelle $R^5$ représente un groupe hydroxyméthyle, on traite un 5-halogénométhyluracile de formule générale

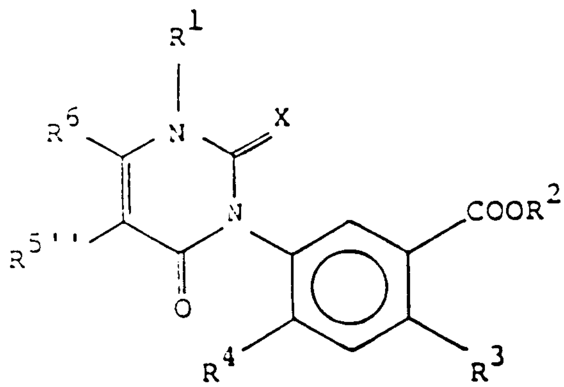

Ie

dans laquelle $R^1$, $R^2$, $R^3$, $R^4$, $R^6$ et X ont les significations indiquées dans la revendication 1 et $R^{5''}$ représente un groupe chloro-, bromo- ou iodo-méthyle, pour hydrolyse par une solution aqueuse d'une base minérale,

g) pour la préparation des composés de formule I dans laquelle $R^5$ représente un groupe (alcoxy en C 1-C 5)-méthyle ou (alkylthio en C 1-C 5)-méthyle, on traite un 5-halogénométhyluracile de formule Ie ci-dessus dans laquelle $R^{5''}$ représente un groupe chloro- ou bromo-méthyle, par un alcoolate ou thioalcoolate de métal alcalin de formule générale

$R^9M$ V

dans laquelle

- $R^9$ représente un groupe alcoxy en C 1-C 5 ou alkylthio en C 1-C 5 et
- M représente un métal alcalin,

ou par un alcanol en C 1-C 5 ou un alkylmercaptan en C 1-C 5,

h) pour la préparation des composés de formule I dans laquelle $R^5$ représente un groupe (alkylthio en C 1-C 5)-méthyle, on traite un 5-hydroxyméthyluracile de formule Ic ci-dessus par un alkylmercaptan en C 1-C 5,

i) pour la préparation des composés de formule I dans laquelle $R^1$ représente l'hydrogène, $R^5$ un groupe cyano, $R^6$ l'hydrogène ou un groupe alkyle en C 1-C 4 et X l'oxygène, on traite un composé de formule générale

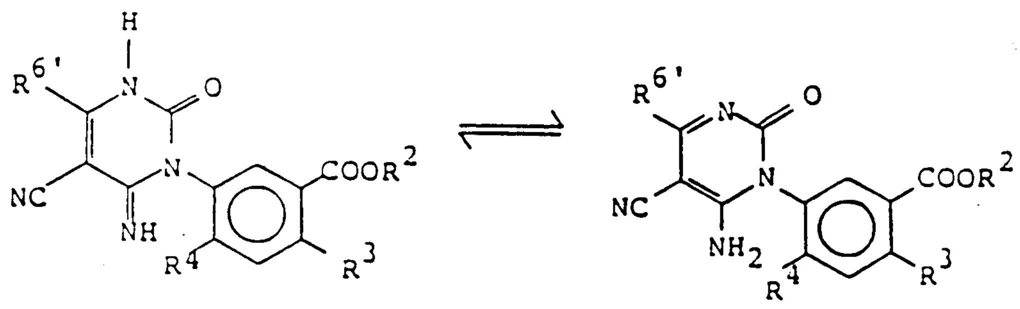

VI

dans laquelle $R^2$, $R^3$ et $R^4$ ont les significations indiquées dans la revendication 1 et $R^{6'}$ représente l'hydrogène ou un groupe alkyle en C 1-C 4, pour hydrolyse catalysée par un acide, à l'aide d'une solution aqueuse d'un acide minéral,

j) pour la préparation des composés de formule I dans laquelle $R^5$ représente un groupe nitro, on traite un dérivé d'uracile de formule Ib ci-dessus par l'acide nitrique,

k) pour la préparation des composés de formule I dans laquelle $R^5$ représente un groupe thiocyanato, on traite un dérivé d'uracile de formule Ib ci-dessus par le thiocyanogène,

l) pour la préparation des composés de formule I dans laquelle $R^2$ représente l'hydrogène, on hydrolyse un ester benzoïque de formule générale

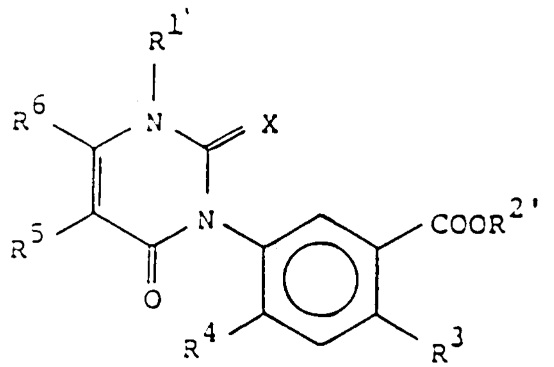

If

dans laquelle $R^1$, $R^{2'}$, $R^3$, $R^4$, $R^5$, $R^6$ et X ont les significations indiquées dans la revendication 1, en l'acide benzoïque correspondant par traitement à l'aide d'une solution aqueuse d'un solvant organique et d'une base minérale,

m) pour la préparation des composés de formule I dans laquelle $R^2$ représente un groupe alkyle en C 1-C 6, alcényle en C 2-C 4, alcynyle en C 2-C 4 ou alcoxyalkyle en C 2-C 6, on estérifie un acide benzoïque de formule générale

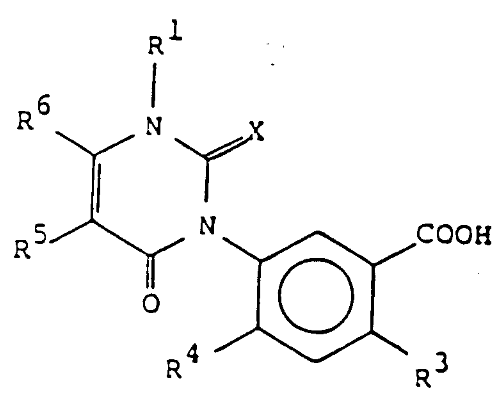

Ig

dans laquelle $R^1$, $R^3$, $R^4$, $R^5$, $R^6$ et X ont les significations indiquées dans la revendication 1, ou un dérivé réactif d'un tel acide, c'est-à-dire que, lorsqu'on utilise un sel de l'acide benzoïque Ig en tant que dérivé réactif, on fait réagir ce sel avec un chlorure, un bromure, un iodure, un sulfate, un méthylsulfate ou toluène-sulfonate d'alkyle en C 1-C 6, d'alcényle en C 2-C 4, d'alcynyle en C 2-C 4 ou d'alcoxyalkyle en C 2-C 6 et, dans le cas où on utilise un halogénure de l'acide ou une O-acyl-1,3-dicyclohexylisourée correspondante, un N-acylimidazole correspondant ou un anhydride de l'acide benzoïque Ig en tant que dérivé réactif, on fait réagir celui-ci avec un alcanol en C 1-C 6, un alcénol en C 2-C 4, un alcynol en C 2-C 4 ou un alcoxyalcanol en C 2-C 6, ou bien

n) pour la préparation des composés de formule I dans laquelle $R^2$ représente un groupe alkyle en C 2-C 6, alcényle en C 2-C 4, alcynyle en C 2-C 4 ou alcoxyalkyle en C 2-C 6, on soumet un ester benzoïque de formule If ci-dessus à une réaction de transestérification avec un alcanol, un alcénol ou respectivement un alcynol de formule générale

$R^{2''}OH$ VII

dans laquelle $R^{2''}$ représente un groupe alkyle en C 2-C 6, alcényle en C 2-C 4, alcynyle en C 2-C 4 ou alcoxyalkyle en C 2-C 6,

sous réserve que le réactif de formule VII bouille plus haut que l'alcanol, l'alcénol ou l'alcynol $R^{2'}OH$, et si on le désire, on convertit un composé ainsi obtenu, répondant à la formule I dans laquelle $R^1$ et/ou $R^2$ représentent l'hydrogène en un sel.

**15.** Procédé selon revendication 14, caractérisé en ce que l'on met en oeuvre une des variantes a), b), d), e), g), h), i), j), k), l), m), n) et le cas échéant la conversion facultative en un sel.

**16.** Procédé de préparation d'un produit herbicide, caractérisé en ce que l'on mélange au moins un des composes mentionnés dans la revendication 1 avec des produits auxiliaires de formulation.

**17.** Procédé pour combattre les végétaux adventices, caractérisé en ce que l'on traite les endroits à protéger contre les végétaux adventices et/ou les végétaux adventices par la quantité efficace d'un produit selon l'une des revendications 1 à 6, 8 à 10 et 12.

**18.** Procédé pour combattre les végétaux adventices, caractérisé en ce que l'on traite les endroits à protéger contre les végétaux adventices et/ou les végétaux adventices par une quantité efficace d'un produit selon l'une des revendications 7, 11 et 13.

**19.** Utilisation d'un produit selon l'une des revendications 1 à 6, 8 à 10 et 12 pour combattre les végétaux adventices.

**20.** Utilisation d'un produit selon l'une des revendications 7, 11 et 13, pour combattre les végétaux adventices.